# EUROPEAN PATENT SPECIFICATION

(11) **EP 2 877 579 B1**
(45) Date of publication and mention of the grant of the patent: **18.12.2019**
(21) Application number: 13823783.9
(22) Date of filing: 26.07.2013
(51) Int. Cl.: C12N 15/11, C07H 21/02, C12N 15/113, A61K 31/7088

(54) **MODULATION OF RENIN-ANGIOTENSIN SYSTEM (RAS) RELATED DISEASES BY ANGIOTENSINOGEN**
MODULATION VON MIT DEM RENINANGIOTENSINSYSTEM (RAS) ASSOZIIERTEN ERKRANKUNGEN DURCH ANGIOTENSINOGEN
MODULATION DE MALADIES ASSOCIÉES AU SYSTÈME RÉNINE ANGIOTENSINE (RAS) PAR L'ANGIOTENSINOGÈNE

(30) Priority: 27.07.2012 US 201261676858 P
(43) Date of publication of application: 03.06.2015
(73) Proprietor: Ionis Pharmaceuticals, Inc., Carlsbad, CA 92010 (US)
(72) Inventor: CROOKE, Rosanne M., Carlsbad, CA 92010 (US); GRAHAM, Mark J., Carlsbad, CA 92010 (US); MULLICK, Adam, Carlsbad, CA 92010 (US)
(74) Representative: Carpmaels & Ransford LLP
(86) International application number: PCT/US2013/052399
(87) International publication number: WO 2014/018930

(56) References cited:
- WO-A1-00/71751
- WO-A1-97/33623
- WO-A1-2011/041545
- WO-A1-2012/007327
- US-A1- 2004 077 611
- US-A1- 2008 261 231
- VAN DE WAL ET AL: "Determinants of increased angiotensin II levels in severe chronic heart failure patients despite ACE inhibition", INTERNATIONAL JOURNAL OF CARDIOLOGY, ELSEVIER SCIENCE PUBLISHERS, AMSTERDAM, NL, vol. 106, no. 3, 26 January 2006 (2006-01-26), pages 367-372, XP005199475, ISSN: 0167-5273, DOI: 10.1016/J.IJCARD.2005.02.016

### SEQUENCE LISTING

<110> Isis Pharmaceuticals, Inc. Adam Mullick Rosanne M. Crooke Mark J. Graham
<120> MODULATION OF RENIN-ANGIOTENSIN SYSTEM
   (RAS) RELATED DISEASES BY ANGIOTENSINOGEN
<130> BIOL0205WO
<150> 61/676,858
   <151> 2012-07-27
<160> 23
<170> FastSEQ for Windows Version 4.0
<210> 1
   <211> 2587
   <212> DNA
   <213> Homo sapiens
<220>
   <221> CDS
   <222> (509)...(1966)
<400> 1
   atcccatgag cgggcagcag ggtcagaagt ggcccccgtg ttgcctaagc aagactctcc 60
   cctgccctct gccctctgca cctccggcct gcatgtccct gtggcctctt gggggtacat 120
   ctcccggggc tgggtcagaa ggcctgggtg gttggcctca ggctgtcaca cacctaggga 180
   gatgctcccg tttctgggaa ccttggcccc gactcctgca aacttcggta aatgtgtaac 240
   tcgaccctgc accggctcac tctgttcagc agtgaaactc tgcatcgatc actaagactt 300
   cctggaagag gtcccagcgt gagtgtcgct tctggcatct gtccttctgg ccagcctgtg 360
   gtctggccaa gtgatgtaac cctcctctcc agcctgtgca caggcagcct gggaacagct 420
   ccatccccac ccctcagcta taaatagggc atcgtgaccc ggccggggga agaagctgcc 480
   gttgttctgg gtactacagc agaagggt atg cgg aag cga gca ccc cag tct    532
                                  Met Arg Lys Arg Ala Pro Gln Ser
                                   1               5
   gag atg gct cct gcc ggt gtg agc ctg agg gcc acc atc ctc tgc ctc   580
   Glu Met Ala Pro Ala Gly Val Ser Leu Arg Ala Thr Ile Leu Cys Leu
       10                  15                  20
   ctg gcc tgg gct ggc ctg gct gca ggt gac cgg gtg tac ata cac ccc   628
   Leu Ala Trp Ala Gly Leu Ala Ala Gly Asp Arg Val Tyr Ile His Pro
    25                  30                  35                  40
   ttc cac ctc gtc atc cac aat gag agt acc tgt gag cag ctg gca aag   676
   Phe His Leu Val Ile His Asn Glu Ser Thr Cys Glu Gln Leu Ala Lys
                   45                  50                  55
   gcc aat gcc ggg aag ccc aaa gac ccc acc ttc ata cct gct cca att   724
   Ala Asn Ala Gly Lys Pro Lys Asp Pro Thr Phe Ile Pro Ala Pro Ile
               60                  65                  70
   cag gcc aag aca tcc cct gtg gat gaa aag gcc cta cag gac cag ctg   772
   Gln Ala Lys Thr Ser Pro Val Asp Glu Lys Ala Leu Gln Asp Gln Leu
           75                  80                  85
   gtg cta gtc gct gca aaa ctt gac acc gaa gac aag ttg agg gcc gca    820
   Val Leu Val Ala Ala Lys Leu Asp Thr Glu Asp Lys Leu Arg Ala Ala
       90                  95                  100
   atg gtc ggg atg ctg gcc aac ttc ttg ggc ttc cgt ata tat ggc atg    868
   Met Val Gly Met Leu Ala Asn Phe Leu Gly Phe Arg Ile Tyr Gly Met
   105                 110                 115                 120
   cac agt gag cta tgg ggc gtg gtc cat ggg gcc acc gtc ctc tcc cca    916
   His Ser Glu Leu Trp Gly Val Val His Gly Ala Thr Val Leu Ser Pro
                   125                 130                 135
   acg gct gtc ttt ggc acc ctg gcc tct ctc tat ctg gga gcc ttg gac    964
   Thr Ala Val Phe Gly Thr Leu Ala Ser Leu Tyr Leu Gly Ala Leu Asp
               140                 145                 150
   cac aca gct gac agg cta cag gca atc ctg ggt gtt cct tgg aag gac    1012
   His Thr Ala Asp Arg Leu Gln Ala Ile Leu Gly Val Pro Trp Lys Asp
           155                 160                 165
   aag aac tgc acc tcc cgg ctg gat gcg cac aag gtc ctg tct gcc ctg    1060
   Lys Asn Cys Thr Ser Arg Leu Asp Ala His Lys Val Leu Ser Ala Leu
       170                 175                 180
   cag gct gta cag ggc ctg cta gtg gcc cag ggc agg gct gat agc cag    1108
   Gln Ala Val Gln Gly Leu Leu Val Ala Gln Gly Arg Ala Asp Ser Gln
   185                 190                 195                 200
   gcc cag ctg ctg ctg tcc acg gtg gtg ggc gtg ttc aca gcc cca ggc    1156
   Ala Gln Leu Leu Leu Ser Thr Val Val Gly Val Phe Thr Ala Pro Gly
                   205                 210                 215
   ctg cac ctg aag cag ccg ttt gtg cag ggc ctg gct ctc tat acc cct    1204
   Leu His Leu Lys Gln Pro Phe Val Gln Gly Leu Ala Leu Tyr Thr Pro
               220                 225                 230
   gtg gtc ctc cca cgc tct ctg gac ttc aca gaa ctg gat gtt gct gct    1252
   Val Val Leu Pro Arg Ser Leu Asp Phe Thr Glu Leu Asp Val Ala Ala
           235                 240                 245
   gag aag att gac agg ttc atg cag gct gtg aca gga tgg aag act ggc    1300
   Glu Lys Ile Asp Arg Phe Met Gln Ala Val Thr Gly Trp Lys Thr Gly
       250                 255                 260
   tgc tcc ctg atg gga gcc agt gtg gac agc acc ctg gct ttc aac acc    1348
   Cys Ser Leu Met Gly Ala Ser Val Asp Ser Thr Leu Ala Phe Asn Thr
   265                 270                 275                 280
   tac gtc cac ttc caa ggg aag atg aag ggc ttc tcc ctg ctg gcc gag    1396
   Tyr Val His Phe Gln Gly Lys Met Lys Gly Phe Ser Leu Leu Ala Glu
                   285                 290                 295
   ccc cag gag ttc tgg gtg gac aac agc acc tca gtg tct gtt ccc atg    1444
   Pro Gln Glu Phe Trp Val Asp Asn Ser Thr Ser Val Ser Val Pro Met
               300                 305                 310
   ctc tct ggc atg ggc acc ttc cag cac tgg agt gac atc cag gac aac    1492
   Leu Ser Gly Met Gly Thr Phe Gln His Trp Ser Asp Ile Gln Asp Asn
           315                 320                 325
   ttc tcg gtg act caa gtg ccc ttc act gag agc gcc tgc ctg ctg ctg    1540
   Phe Ser Val Thr Gln Val Pro Phe Thr Glu Ser Ala Cys Leu Leu Leu
       330                 335                 340
   atc cag cct cac tat gcc tct gac ctg gac aag gtg gag ggt ctc act    1588
   Ile Gln Pro His Tyr Ala Ser Asp Leu Asp Lys Val Glu Gly Leu Thr
   345                 350                 355                 360
   ttc cag caa aac tcc ctc aac tgg atg aag aaa cta tct ccc cgg acc    1636
   Phe Gln Gln Asn Ser Leu Asn Trp Met Lys Lys Leu Ser Pro Arg Thr
                   365                 370                 375
   atc cac ctg acc atg ccc caa ctg gtg ctg caa gga tct tat gac ctg    1684
   Ile His Leu Thr Met Pro Gln Leu Val Leu Gln Gly Ser Tyr Asp Leu
               380                 385                 390
   cag gac ctg ctc gcc cag gct gag ctg ccc gcc att ctg cac acc gag    1732
   Gln Asp Leu Leu Ala Gln Ala Glu Leu Pro Ala Ile Leu His Thr Glu
           395                 400                 405
   ctg aac ctg caa aaa ttg agc aat gac cgc atc agg gtg ggg gag gtg    1780
   Leu Asn Leu Gln Lys Leu Ser Asn Asp Arg Ile Arg Val Gly Glu Val
       410                 415                 420
   ctg aac agc att ttt ttt gag ctt gaa gcg gat gag aga gag ccc aca    1828
   Leu Asn Ser Ile Phe Phe Glu Leu Glu Ala Asp Glu Arg Glu Pro Thr
   425                 430                 435                 440
   gag tct acc caa cag ctt aac aag cct gag gtc ttg gag gtg acc ctg    1876
   Glu Ser Thr Gln Gln Leu Asn Lys Pro Glu Val Leu Glu Val Thr Leu
                   445                 450                 455
   aac cgc cca ttc ctg ttt gct gtg tat gat caa agc gcc act gcc ctg    1924
   Asn Arg Pro Phe Leu Phe Ala Val Tyr Asp Gln Ser Ala Thr Ala Leu
               460                 465                 470
   cac ttc ctg ggc cgc gtg gcc aac ccg ctg agc aca gca tga            1966
   His Phe Leu Gly Arg Val Ala Asn Pro Leu Ser Thr Ala
           475                 480                 485
   ggccagggcc ccagaacaca gtgcctggca aggcctctgc ccctggcctt tgaggcaaag 2026
   gccagcagca gataacaacc ccggacaaat cagcgatgtg tcacccccag tctcccacct 2086
   tttcttctaa tgagtcgact ttgagctgga aagcagccgt ttctccttgg tctaagtgtg 2146
   ctgcatggag tgagcagtag aagcctgcag cggcacaaat gcacctccca gtttgctggg 2206
   tttattttag agaatggggg tggggaggca agaaccagtg tttagcgcgg gactactgtt 2266
   ccaaaaagaa ttccaaccga ccagcttgtt tgtgaaacaa aaaagtgttc ccttttcaag 2326
   ttgagaacaa aaattgggtt ttaaaattaa agtatacatt tttgcattgc cttcggtttg 2386
   tatttagtgt cttgaatgta agaacatgac ctccgtgtag tgtctgtaat accttagttt 2446
   tttccacaga tgcttgtgat ttttgaacaa tacgtgaaag atgcaagcac ctgaatttct 2506
   gtttgaatgc ggaaccatag ctggttattt ctcccttgtg ttagtaataa acgtcttgcc 2566
   acaataagcc tccaaaaaaa a                                            2587
<210> 2
   <211> 1670
   <212> DNA
   <213> Homo sapiens
<400> 2
   agggtatgcg gaagcgagca ccccagtctg agatggctcc tgccggtgtg agcctgaggg 60
   ccaccatcct ctgcctcctg gcctgggctg gcctggctgc aggtgaccgg gtgtacatac 120
   accccttcca cctcgtcatc cacaatgaga gtacctgtga gcagctggca aaggccaatg 180
   ccgggaagcc caaagacccc accttcatac ctgctccaat tcaggccaag acatcccctg 240
   tggatgaaaa ggccctacag gaccagctgg tgctagtcgc tgcaaaactt gacaccgaag 300
   acaagttgag ggccgcaatg gtcgggatgc tggccaactt cttgggcttc cgtatatatg 360
   gcatgcacag tgagctatgg ggcgtggtcc atggggccac cgtcctctcc ccaacggctg 420
   tctttggcac cctggcctct ctctatctgg gagccttgga ccacacagct gacaggctac 480
   aggcaatcct gggtgttcct tggaaggaca agaactgcac ctcccggctg gatgcgcaca 540
   aggtcctgtc tgccctgcag gctgtacagg gcctgctagt ggcccagggc agggctgata 600
   gccaggccca gctgctgctg tccacggtgg tgggcgtgtt cacagcccca ggcctgcacc 660
   tgaagcagcc gtttgtgcag ggcctggctc tctatacccc tgtggtcctc ccacgctctc 720
   tggacttcac agaactggat gttgctgctg agaagattga caggttcatg caggctgtga 780
   caggatggaa gactggctgc tccctgatgg gagccagtgt ggacagcacc ctggctttca 840
   acacctacgt ccacttccaa gggaagatga agggcttctc cctgctggcc gagccccagg 900
   agttctgggt ggacaacagc acctcagtgt ctgttcccat gctctctggc atgggcacct 960
   tccagcactg gagtgacatc caggacaact tctcggtgac tcaagtgccc ttcactgaga 1020
   gcgcctgcct gctgctgatc cagcctcact atgcctctga cctggacaag gtggagggtc 1080
   tcactttcca gcaaaactcc ctcaactgga tgaagaaact atctccccgg tgctgaacag 1140
   catttttttt gagcttgaag cggatgagag agagcccaca gagtctaccc aacagcttaa 1200
   caagcctgag gtcttggagg tgaccctgaa ccgcccattc ctgtttgctg tgtatgatca 1260
   aagcgccact gccctgcact tcctgggccg cgtggccaac ccgctgagca cagcatgagg 1320
   ccagggcccc agaacacagt gcctggcaag gcctctgccc ctggcctttg aggcaaaggc 1380
   cagcagcaga taacaacccc ggacaaatca gcgatgtgtc acccccagtc tcccaccttt 1440
   tcttctaatg agtcgacttt gagctggaaa gcagccgttt ctccttggtc taagtgtgct 1500
   gcatggagtg agcagtagaa gcctgcagcg gcacaaatgc acctcccagt ttgctgggtt 1560
   tattttagag aatgggggtg gggaggcaag aaccagtgtt tagcgcggga ctactgttcc 1620
   aaaaagaatt ccaaccgacc agcttgtttg tgaaacaaaa aagtgttccc           1670
<210> 3
   <211> 1399
   <212> DNA
   <213> Homo sapiens
<400> 3
   agaagctgcc gttgttctgg gtactacagc agaagggtat gcggaagcga gcaccccagt 60
   ctgagatggc tcctgccggt gtgagcctga ggaccaccat cctctgcctc ctggcctggg 120
   ctggcctggc tgcaggtgac cgggtgtaca tacacccctt ccacctcgtc atccacaatg 180
   agagtacctg tgagcagctg gccctacagg accagctggt gctagtcgct gcaaaacttg 240
   acaccgaaga caagttgagg gccgcaatgg tcgggatgct ggccaacttc ttgggcttcc 300
   gtatatatgg catgcacagt gagctatggg gcgtggtcca tggggccacc gtcctctccc 360
   caacggctgt ctttggcacc ctggcctctc tctatctggg agccttggac cacacagctg 420
   acaggctaca ggcaatcctg ggtgttcctt ggaaggacaa gaactgcacc tcccggctgg 480
   atgcgcacaa ggtcctgtct gccctgcagg ctgtacaggg cctgctagtg gcccagggca 540
   gggctgatag ccaggcccag ctgctgctgt ccacggtggt gggcgtgttc acagccccag 600
   gcctgcacct gaagcagccg tttgtgcagg gcctggctct ctatacccct gtggtcctcc 660
   cacgctctct ggacttcaca gaactggatg ttgctgctga gaagattgac aggttcatgc 720
   aggctgtgac aggatggaag actggctgct ccctgatggg agccagtgtg gacagcaccc 780
   tggctttcaa cacctacgtc cacttccaag ggaagatgaa gggcttctcc ctgctggccg 840
   agccccagga gttctgggtg gacaacagca cctcagtgtc tgttcccatg ctctctggca 900
   tgggcacctt ccagcactgg agtgacatcc aggacaactt ctcggtgact caagtgccct 960
   tcactgagag cgcctgcctg ctgctgatcc agcctcacta tgcctctgac ctggacaagg 1020
   tggagggtct cactttccag caaaactccc tcaactggat gaagaaactg tctccccgga 1080
   ccatccacct gaccatgccc caactggtgc tgcaaggatc ttatgacctg caggacctgc 1140
   tcgcccaggc tgagctgccc gccattctgc acaccgagct gaacctgcaa aaattgagca 1200
   atgaccgcat cagggtgggg gaggtgctga acagcatttt tttgagcttg aagcggatga 1260
   gagagagccc acagagtcta cccaacagct taacaagcct gaggtcttgg aggtgaccct 1320
   gaaccgccca ttcctgtttg ctgtgtatga tcaaagcgcc actgccctgc acttcctggg 1380
   ccgcgtggcc aacccgctg                                            1399
<210> 4
   <211> 1584
   <212> DNA
   <213> Homo sapiens
<220>
   <221> CDS
   <222> (39) ... (1439)
<400> 4
   agaagctgcc gttgttctgg gtactacagc agaagggt atg cgg aag cga gca ccc 56
   cag tct gag atg gct cct gcc ggt gtg agc ctg agg gcc acc atc ctc   104
   tgc ctc gtc atc cac aat gag agt acc tgt gag cag ctg gca aag gcc   152
   aat gcc ggg aag ccc aaa gac ccc acc ttc ata cct gct cca att cag   200
   gcc aag aca tcc cct gtg gat gaa aag gcc cta cag gac cag ctg gtg   248
   cta gtc gct gca aaa ctt gac acc gaa gac aag ttg agg gcc gca atg   296
   gtc ggg atg ctg gcc aac ttc ttg ggc ttc cgt ata tat ggc atg cac   344
   agt gag cta tgg ggc gtg gtc cat ggg gcc acc gtc ctc tcc cca acg   392
   gct gtc ttt ggc acc ctg gcc tct ctc tat ctg gga gcc ttg gac cac   440
   aca gct gac agg cta cag gca atc ctg ggt gtt cct tgg aag gac aag   488
   aac tgc acc tcc cgg ctg gat gcg cac aag gtc ctg tct gcc ctg cag   536
   gct gta cag ggc ctg cta gtg gcc cag ggc agg gct gat agc cag gcc   584
   cag ctg ctg ctg tcc acg gtg gtg ggc gtg ttc aca gcc cca ggc ctg   632
   cac ctg aag cag ccg ttt gtg cag ggc ctg gct ctc tat acc cct gtg   680
   gtc ctc cca cgc tct ctg gac ttc aca gaa ctg gat gtt gct gct gag   728
   aag att gac agg ttc atg cag gct gtg aca gga tgg aag act ggc tgc   776
   tcc ctg acg gga gcc agt gtg gac agc acc ctg gct ttc aac acc tac    824
   gtc cac ttc caa ggg agg atg aag ggc ttc tcc ctg ctg gcc gag ccc    872
   cag gag ttc tgg gtg gac aac agc acc tca gtg tct gtt ccc atg ctc    920
   tct ggc atg ggc acc ttc cag cac tgg agt gac atc cag gac aac ttc    968
   tcg gtg act caa gtg ccc ttc act gag agc gcc tgc ctg ctg ctg atc    1016
   cag cct cac tat gcc tct gac ctg gac aag gtg gag ggt ctc act ttc    1064
   cag caa aac tcc ctc aac tgg atg aag aaa ctg tct ccc cgg acc atc    1112
   cac ctg acc atg ccc caa ctg gtg ctg caa gga tct tat gac ctg cag    1160
   gac ctg ctc gcc cag gct gag ctg ccc gcc att ctg cac acc gag ctg    1208
   aac ctg caa aaa ttg agc aat gac cgc atc agg gtg ggg gag gtg ctg    1256
   aac agc att ttt ttt gag ctt gaa gcg gat gag aga gag ccc aca gag    1304
   tct acc caa cag ctt aac aag cct gag gtc ttg gag gtg acc ctg aac    1352
   cgc cca ttc ctg ttt gct gtg tat gat caa agc gcc act gcc ctg cac    1400
   ttc ctg ggc cgc gtg gcc aac ccg ctg agc aca gca tga ggccagggcc     1449
   ccagaacaca gtgcctggca aggcctctgc ccctggcctt tgaggcaaag gccagcagca 1509
   gataacaacc ccggacaaat cagcgatgtg tcacccccag tctcccacct tttcttctaa 1569
   tgagtcgact ttgag                                                  1584
<210> 5
   <211> 1691
   <212> DNA
   <213> Homo sapiens
<220>
   <221> CDS
   <222> (40) ... (1251)
<400> 5
   aagaagctgc cgttgttctg ggtactacag cagaagggt atg cgg aag cga gca     54
   ccc cag tct gag atg gct cct gcc ggt gtg agc ctg agg gcc acc atc   102
   ctc tgc ctc ctg gcc tgg gct ggc ctg gct gca ggt gac cgg gtg tac   150
   ata cac ccc ttc cac ctc gtc atc cac aat gag agt acc tgt gag cag   198
   ctg gca aag gcc aat gcc ggg aag ccc aaa gac ccc acc ttc ata cct   246
   gct cca att cag gcc aag aca tcc cct gtg gat gaa aag gcc cta cag   294
   gac cag ctg gtg cta gtc gct gca aaa ctt gac acc gaa gac aag ttg   342
   agg gcc gca atg gtc ggg atg ctg gcc aac ttc ttg ggc ttc cgt ata   390
   tat ggc atg cac agt gag cta tgg ggc gtg gtc cat ggg gcc acc gtc   438
   ctc tcc cca acg gct gtc ttt ggc acc ctg gcc tct ctc tat ctg gga   486
   gcc ttg gac cac aca gct gac agg cta cag gca atc ctg gat gtt gct   534
   gct gag aag att gac agg ttc atg cag gct gtg aca gga tgg aag act   582
   ggc tgc tcc ctg atg gga gcc agt gtg gac agc acc ctg gct ttc aac   630
   acc tac gtc cac ttc caa ggg aag atg aag ggc ttc tcc ctg ctg gcc   678
   gag ccc cag gag ttc tgg gtg gac aac agc acc tca gtg tct gtt ccc    726
   atg ctc tct ggc atg ggc acc ttc cag cac tgg agt gac atc cag gac    774
   aac ttc tcg gtg act caa gtg ccc ttc act gag agc gcc tgc ctg ctg    822
   ctg atc cag cct cac tat gcc tct gac ctg gac aag gtg gag ggt ctc    870
   act ttc cag caa aac tcc ctc aac tgg atg aag aaa ctg tct ccc cgg    918
   acc atc cac ctg acc atg ccc caa ctg gtg ctg caa gga tct tat gac    966
   ctg cag gac ctg ctc gcc cag gct gag ctg ccc gcc att ctg cac acc    1014
   gag ctg aac ctg caa aaa ttg agc aat gac cgc atc agg gtg ggg gag    1062
   gtg ctg aac agc att ttt ttt gag ctt gaa gcg gat gag aga gag ccc    1110
   aca gag tct acc caa cgg ctt aac aag cct gag gtc ttg gag gtg acc    1158
   ctg aac cgc cca ttc ctg ttt gct gtg tat gat caa agc gcc act gcc    1206
   ctg cac ttc ctg ggc cgc gtg gcc aac ccg ctg agc aca gca tga        1251
   ggccagggcc ccagaacaca gtgcctggca aggcctctgc ccctggcctt tgaggcaaag 1311
   gccagcagca gataacaacc ccggacaaat cagcgatgtg tcacccccag tctcccacct 1371
   tttcttctaa tgagtcgact ttgagctgga aagcagccgt ttctccttgg tctaagtgtg 1431
   ctgcatggag tgagcagtag aagcctgcag cggcgcaaat gcacctccca gtttgctggg 1491
   tttattttag agaatggggg tggggaggca agaaccagtg tttagcgcgg gactactgtt 1551
   ccaaaaagaa ttccaaccga ccagcttgtt tgtgaaacaa aaaagtgttc ccttttcaag 1611
   ttgagaacaa aaattgggtt ttaaaattaa agtatacatt tttgcattgc cttcggtttg 1671
   tatttagtgt cttgaatgta                                             1691
<210> 6
   <211> 16101
   <212> DNA
   <213> Homo sapiens
<400> 6
<210> 7
   <211> 1856
   <212> DNA
   <213> Homo sapiens
<220>
   <221> CDS
   <222> (48) ... (1496)
<400> 7
   gatagctgtg cttgtctagg ttggcgctga aggatacaca gaagcaa atg cac aga    56
                                                       Met His Arg
                                                        1
   tcg gag atg act ccc acg ggg gca ggc ctg aag gcc acc atc ttc tgc    104
   Ser Glu Met Thr Pro Thr Gly Ala Gly Leu Lys Ala Thr Ile Phe Cys
       5                   10                  15
   atc ttg acc tgg gtc agc ctg acg gct ggg gac cgc gta tac atc cac    152
   Ile Leu Thr Trp Val Ser Leu Thr Ala Gly Asp Arg Val Tyr Ile His
    20                  25                  30                  35
   ccc ttc cat ctc ctt tac cac aac aag agc acc tgc gcc cag ctg gag    200
   Pro Phe His Leu Leu Tyr His Asn Lys Ser Thr Cys Ala Gln Leu Glu
                    40                  45                  50
   aac ccc agt gtg gag aca ctc cca gag tca acg ttc gag cct gtg ccc    248
   Asn Pro Ser Val Glu Thr Leu Pro Glu Ser Thr Phe Glu Pro Val Pro
                55                  60                  65
   att cag gcc aag acc tcc cct gtg aat gag aag acc ctg cat gat cag    296
   Ile Gln Ala Lys Thr Ser Pro Val Asn Glu Lys Thr Leu His Asp Gln
            70                  75                  80
   ctc gtg ctg gcc gcc gag aag cta gag gat gag gac cgg aag cgg gct    344
   Leu Val Leu Ala Ala Glu Lys Leu Glu Asp Glu Asp Arg Lys Arg Ala
       85                  90                  95
   gcc cag gtc gca atg atc gcc aac ttc gtg ggc ttc cgc atg tac aag    392
   Ala Gln Val Ala Met Ile Ala Asn Phe Val Gly Phe Arg Met Tyr Lys
   100                 105                 110                 115
   atg ctg aat gag gca gga agt ggg gcc agt ggg gcc atc ctc tca cca    440
   Met Leu Asn Glu Ala Gly Ser Gly Ala Ser Gly Ala Ile Leu Ser Pro
                   120                 125                 130
   cca gct ctc ttt ggc acc ctg gtc tct ttc tac ctt gga tcc tta gat    488
   Pro Ala Leu Phe Gly Thr Leu Val Ser Phe Tyr Leu Gly Ser Leu Asp
               135                 140                 145
   ccc acg gcc agc cag ctg cag acg ctg ctg gat gtc cct gtg aag gag    536
   Pro Thr Ala Ser Gln Leu Gln Thr Leu Leu Asp Val Pro Val Lys Glu
           150                 155                 160
   gga gac tgc acc tcc cga cta gat gga cac aag gtc ctc gct gcc ctg    584
   Gly Asp Cys Thr Ser Arg Leu Asp Gly His Lys Val Leu Ala Ala Leu
       165                 170                 175
   cgg gcc att cag ggc ttg ctg gtc acc cag ggt ggg agc agc agc cag    632
   Arg Ala Ile Gln Gly Leu Leu Val Thr Gln Gly Gly Ser Ser Ser Gln
   180                 185                 190                 195
   aca ccc ctg cta cag tcc att gtg gtg ggg ctc ttc act gct cca ggc    680
   Thr Pro Leu Leu Gln Ser Ile Val Val Gly Leu Phe Thr Ala Pro Gly
                   200                 205                 210
   ttt cgt cta aag cac tca ttt gtt cag agc ctg gct ctc ttt acc cct    728
   Phe Arg Leu Lys His Ser Phe Val Gln Ser Leu Ala Leu Phe Thr Pro
               215                 220                 225
   gcc ctc ttc cca cgc tct ctg gat tta tcc act gac cca gtt ctt gcc    776
   Ala Leu Phe Pro Arg Ser Leu Asp Leu Ser Thr Asp Pro Val Leu Ala
           230                 235                 240
   act gag aaa atc aac agg ttc ata aag gct gtg aca ggg tgg aag atg    824
   Thr Glu Lys Ile Asn Arg Phe Ile Lys Ala Val Thr Gly Trp Lys Met
       245                 250                 255
   aac ttg cca ctg gag ggg gtc agt aca gac agc acc cta ctt ttc aac    872
   Asn Leu Pro Leu Glu Gly Val Ser Thr Asp Ser Thr Leu Leu Phe Asn
   260                 265                 270                 275
   acc tac gtt cac ttc caa gga acg atg aga ggt ttc tct cag ctg cct    920
   Thr Tyr Val His Phe Gln Gly Thr Met Arg Gly Phe Ser Gln Leu Pro
                   280                 285                 290
   gga gtc cat gaa ttc tgg gtg gac aac agc atc tcg gtg tct gtg ccc    968
   Gly Val His Glu Phe Trp Val Asp Asn Ser Ile Ser Val Ser Val Pro
               295                 300                 305
   atg atc tcc ggc act ggc aac ttc cag cac tgg agt gac acc cag aac    1016
   Met Ile Ser Gly Thr Gly Asn Phe Gln His Trp Ser Asp Thr Gln Asn
           310                 315                 320
   aac ttc tcc gtg acg tgc gtg ccc cta ggt gag aga gcc acc ctg ctg    1064
   Asn Phe Ser Val Thr Cys Val Pro Leu Gly Glu Arg Ala Thr Leu Leu
       325                 330                 335
   ctc atc cag ccc cac tgc acc tca gat ctc gac agg gtg gag gcc ctc    1112
   Leu Ile Gln Pro His Cys Thr Ser Asp Leu Asp Arg Val Glu Ala Leu
   340                 345                 350                 355
   atc ttc cgg aac gac ctc ctg act tgg ata gag aac ccg cct cct cgg    1160
   Ile Phe Arg Asn Asp Leu Leu Thr Trp Ile Glu Asn Pro Pro Pro Arg
                   360                 365                 370
   gcc atc cgc ctg act ctg ccc cag ctg gaa atc cga gga tcc tac aat    1208
   Ala Ile Arg Leu Thr Leu Pro Gln Leu Glu Ile Arg Gly Ser Tyr Asn
                   375                 380                 385
   ctg cag gac ctg ctg gct gag gac aag ctg ccc acc ctt ttg ggt gcg   1256
   Leu Gln Asp Leu Leu Ala Glu Asp Lys Leu Pro Thr Leu Leu Gly Ala
           390                 395                 400
   gag gca aat ctg aac aac att ggt gac acc aac ccc cga gtg gga gag   1304
   Glu Ala Asn Leu Asn Asn Ile Gly Asp Thr Asn Pro Arg Val Gly Glu
       405                 410                 415
   gtt ctc aat agc atc ctc ctc gaa ctc aaa gca gga gag gag gaa cag   1352
   Val Leu Asn Ser Ile Leu Leu Glu Leu Lys Ala Gly Glu Glu Glu Gln
   420                 425                 430                 435
   ccg acc acg tct gtc cag cag cct ggc tca ccg gag gca ctg gat gtg   1400
   Pro Thr Thr Ser Val Gln Gln Pro Gly Ser Pro Glu Ala Leu Asp Val
                   440                 445                 450
   acc ctg agc agc ccc ttc ctg ttc gcc atc tac gag cag gac tca ggc   1448
   Thr Leu Ser Ser Pro Phe Leu Phe Ala Ile Tyr Glu Gln Asp Ser Gly
               455                 460                 465
   acg ctg cac ttt ctg ggc aga gtg aat aac ccc cag agt gtg gtg tga   1496
   Thr Leu His Phe Leu Gly Arg Val Asn Asn Pro Gln Ser Val Val
           470                 475                 480
   ggccttgtgc ctagccatgg agacaaggcc ggtgtcggag aaccgttctg ggcaaaactc 1556
   agtgctgtca cccctggctc cccatcacgc cttgtagcgc ggcagaggcc gtctccttgg 1616
   agactgcgct gaccgagaat aaatgatgag cagcagagcc tcctgggatg tgggtttgtt 1676
   tggatactgg ggtgacagcc agaagctggc actctgcaca ggactgccac tctggaagaa 1736
   atttggacca aaaaactgtt tgtgacacca aaaagcaccc cccctttttt ttatttgagg 1796
   acagaaattg ggttttaaca ttaaaatgca cattatcccc ttaaaaaaaa aaaaaaaaaa 1856
<210> 8
   <211> 358
   <212> DNA
   <213> Mus musculus
<400> 8
   agccagacac ccctgctaca gtccattgtg gtggggctct tcactgctcc aggctttcgt 60
   ctaaagcact catttgttca gagcctggct ctctttaccc ctgccctctt cccacgctct 120
   ctggatttat ccactgaccc agttcttgcc actgagaaaa tcaacaggtt cataaaggct 180
   gtgacagggt ggaagatgaa cttgccactg gagggggtca gtacagacag caccctactt 240
   ttcaacacct acgttcactt ccaaggaacg atgagaggtt ctctcagctg cctggagtcc 300
   atgaattctg ggtggacaac agcatctcgg agaaccgttc tgggcaaaac tcagtgct   358
<210> 9
   <211> 457
   <212> DNA
   <213> Mus musculus
<400> 9
   cttgtctagg ttggcgctga aggaacacag aagcaaatgc acagatcgga gatgactccc 60
   acgggggcag gcctgaaggc caccatcttc tgcatcttga cctgggtcag cctgacggct 120
   ggggaccgcg tatacatcca cccagttctt gccactgaga aaatcaacag gttcataaag 180
   gctgtgacag ggtggaagat gaacttgcca ctggaggggg tcagtacaga cagcacccta 240
   cttttcaaca cctacgttca cttccaagga acgatgagag gtttctctca gctgcctgga 300
   gtccatgaat tctgggtggg caacagcatc tcggtgtctg tgcccatgat ctccggcact 360
   ggcaacttcc agcactggag tgacacccag aacaacttct ccgtgacgtg cgtgccccta 420
   ggtgagagag ccaccctgct gctcatccag ccccact                          457
<210> 10
   <211> 830
   <212> DNA
   <213> Mus musculus
<400> 10
   cggacgcgtg gggtctaggt tggcgctgaa ggatacacag aagcaaatgc acagatcgga 60
   gatgactccc acgggggcag gcctgaaggc caccatcttc tgcatcttga cctgggtcag 120
   cctgacggct ggggaccgcg tatacatcca ccccttccat ctcctttacc acaacaagag 180
   cacctgcgcc cagctggaga accccagtgt ggagacactc ccagagtcaa cgttcgagcc 240
   tgtgcccatt caggccaaga cctcccctgt gaatgagaag accctgcaga cgctgctgga 300
   tgtccctgtg aaggagggag actgcacctc ccgactagat ggacacaagg tcctcgctgc 360
   cctgcgggcc attcagggct tgctggtcac ccagggtggg agcagcagcc agacacccct 420
   gctacagtcc attgtggtgg ggctcttcac tgctccaggc tttcgtctaa agcactcatt 480
   tgttcagagc ctggctctct ttacccctgc cctcttccca cgctctctgg atttatccac 540
   tgacccagtt cttgccactg agaaaatcaa caggttcata aacggctgtg acagggtgga 600
   agatgaactt gccactggag ggggtcagta cagacagcac cctacttttc aacacctacg 660
   ttcacttcca aggaacgatg acgaggtttc tctcagctgc ctggagtcca tgaattctgg 720
   gtggacaaca gcatctcggt gtctgtgcgc atgatctccg gcactggcaa cttccagcac 780
   tggagtgaca ccagaacaac ttctcgtgaa gtgcgtgccc taggtgagac           830
<210> 11
   <211> 633
   <212> DNA
   <213> Mus musculus
<400> 11
   tcagtactgt gcgtgccctc aggtagcaga gccaccttgc tgtcattcag cctcatctgc 60
   acatcagatc ttcacagggt gcaggccctc atcttccgaa acgacctcct gacttggata 120
   gagaacccgc ctcctcgggc catccgcctg actctgcccc agctggaaat ccgaggatcc 180
   tacaatctgc aggacctgct ggctgaggac aagctgccca cccttttggg tgcggaggca 240
   aatctgaaca acattggtga caccaacccc cgagtgggag aggttctcaa tagcatcctc 300
   ctcgaactca aagcaggaga ggaggaacag ccgaccacgt ctgtccagca gcctggctca 360
   ccggaggcac tggatgtgac cctgagcagc cccttcctgt tcgccatcta cgagcaggac 420
   tcaggcacgc tgcactttct gggcagagtg aataaccccc agagtgtggt gtgaggcctt 480
   gtgcctagcc atggagacaa ggccggtgtc ggagaaccgt tctgggcaaa actcagtgct 540
   gtcacccctg gctccccatc acgccttgta gcgcggcaga ggccgtctcc ttggagactg 600
   cgctgaccga gaataaatga tgagcagcag aaa                             633
<210> 12
   <211> 16201
   <212> DNA
   <213> Mus musculus
<400> 12
   ccgtcactct acagccctgc ctcctacagc tggtctccca ctgagatctc tggtagttat 60
   tttatgtctg tgcccagaga aggttgctct tcagagccca tctcaaacac catcaagtcc 120
   tgcatggtgc atctgtcttc taagccagcg actgctctgc ccactctctc tttccacctc 180
   tactctccca tgccactttt aggtgaaggc aggttgtgcc tgtgtccaca tggccaagag 240
   attgcttgaa gttgtggatg cgatgcaagc tagtcgccac ccatggtagc ctgcaggtgg 300
   gactgtctgc caggcctact cctgctgtct tctcagatcc cacccttccc tggtgtccac 360
   cagtctcatc ccttaaactt tcacaaccat tctccaccct caagggaggc gctccaaaac 420
   ctccaacctc caaaactgct gctttccgtc catgagccca gaacattttg tttcaatatg 480
   gctctaccac agtcagaatt tcccagccta atcaggtgtg ctagaagttc cggaaggcgc 540
   ttcctactgg tgatgtgtgt cctcagccca ctggaagccc ctgtacagaa caggcaacta 600
   acgtaactac aaatgtccaa ggtcgtccac cctgagagca gctggtgacg tgaggttcag 660
   ctcctgcaag tgagccccct tcctggcatg cccagagagg cttacgagtg catcacgagg 720
   gggctttcat cccaaggtct gcatggctgg cttcaggttg tcacaaccca ctcaatcctg 780
   tgactgtggt cctggctcca gggaactggg gtaaatgtgt aacccaaggc cagcctattt 840
   ttgcatgagg ctcatctgcc agtagggctt cctggaaggg gcccagagga acatcaccct 900
   ggccctgatc catcttggtc aagcctggat tctcatggtt ccctgatctg ggtcctcccc 960
   cagcctctgt acagagtagc ccaggaatag atccatctcc accccttgag tataaataag 1020
   cctgcttggc tcaccagggg atagctgtgc ttgtctaggt tggcgctgaa ggtaagcaga 1080
   gcactcttgt cttggttctt tcatgggatc tggccaggca ggagctgagc tgtgggtggg 1140
   aagaggaggc cgggactggg cagagacact gggaagggct ggatggcatt ttttctggag 1200
   tgagaagtct gtggtagcag gcgcttcagg agatgtgagc actgactgct tctcctattc 1260
   cgaaaggcta gatgtccagg taaactgtcc cctgtgggtc accaaactgg gctcactggg 1320
   aactgatggt ctttcattag gggatggcca ggtccttttg cctcctgtga gtccagatgg 1380
   gtcctcaaaa gcagagagac tctcacaggg tcagcactag ggtggccaag tcccctggac 1440
   ctcctccagc ctgaagggag aacaaggctt cattgttcag actgcaagaa gcaggaaggg 1500
   acaagtcacg tgacatctct atccaggctc aatgctgtgg tctgtgtctt gtggctcctc 1560
   accatgtcct gtagggcctc aacttacccc tctgtgaaca ggcttcatca cagttctgag 1620
   gttctctagg gctggggata agttaggagc atccagtaac atggaagtac tttgaaaagt 1680
   tagctccttc tttattctta gtgtggtgtg tgtgtgtgtg ccgtgttgtg tatgtgcata 1740
   ttatttattt atttacttct ggtttctgag acagggtttc tctgttgtag tctttgctgt 1800
   cctagaactc actctgtaaa gcgtgcaggc ctcagattca gagatatgtc tgcccctttt 1860
   tttttttttt tttttggaag acagggtctt actgtgtagc tctggttgtc ctagaactca 1920
   ctaaattctg tctgcctcaa cctcttgatt gtatcaccat acccagccca aggtatatat 1980
   gtatttatgt gaatgtgaca tatgtgtgta tgacgtgtgt atttatgtac atatgtatgt 2040
   atgtatgtat gtatgtatgt atgtatgtat gtatgtatgt gtatatatga agtgtagggg 2100
   aggagcatgg agacatagga gccatgaaca cagatagaga ctagaggagg ataccaggtg 2160
   tcctgccagc tactgtcctc cttagtccct tgagacaggg tcccttcctg aagctggggc 2220
   tagactggca gccaggaagc cctggagatc atcctgtctc ctcgctccac ctctctggag 2280
   ttaggggagt atgtggttgt gactggtctt tcacaagggg tcaggggacg agaacccgga 2340
   ttttcatatt tatgcaacaa cgtgttcttg cgtgctgaac ctgccctagg ctcctcagtt 2400
   ccttatttct tttaagaggc aatatgtgtc atgtctagat gccactcatg gattcaagtg 2460
   ttagggctga gtcccagctg tgtgacctgg tgcaggtcac aagacttgtc tgttcctgta 2520
   caaatggcac tgatgattct acagctatcc ctggatgacc ttaggaaggt gggctaacct 2580
   gtggcactca ggtacctgtg ggaggcagac cagtggggca catagtaaat acatcgatac 2640
   aggatatagg gtgcttctgg tggctatgag gtacagagga ggtgagagag aggtgtgggg 2700
   agcctgaggt agactttgag tggtgaagat gaactattga cagaagacag cactggaatg 2760
   caccctgata atgacaggac aggcaacata gccctgagaa gccacaagac agggacacag 2820
   agaggcagct ggtggggagg gaatagaagt gtgtgtgtgg gggtgagatc tcctatgtcc 2880
   tcaagatttg ctgcaatcac acttagggtg aacttgactg acagcagctt acagctaaga 2940
   acaaacagtc acttgtgcct aaggacagta gtgacgcgtt tcctgggaca gcagccatgg 3000
   cactaccttc tggacagagg ccgttcatag gaacagcagt ctaggaaaca ttgtgttcac 3060
   ccccacagct catccagtgg cctgggcacc tgaggcaggt cctgacccta ctgtgttgga 3120
   ttataaagca aggaaaggcc agtgagtaca ggcagagctc tctactctgc tttcccaaac 3180
   ctctaaactt tctttaagaa tgatgggtga agccagatgt gtgtgtcaaa aggatgaagc 3240
   actagacagt cagaagccaa aggttctcag accagtggga tgaccagagc agagcaaaga 3300
   gcaccttgtc tgtctgtgga ggatattcta tgtgcagcct cggcaagcac gggaatatgg 3360
   gagtccaggg tgaccttgga gctttgcaac tccatagtga cattctgaga tcattaactt 3420
   atgaagagaa aggtttgtct tggctcatgg tactggaatt gcaggtcaaa ttcaggtaga 3480
   ctgttgctcg ggacttctgg ggtgttggct gacaatggca atgacatctg gtaaaacgag 3540
   ctgcttccaa tgtagctagg aagcaaaagc aacgaagaag gaaagacaca ggtcctgcga 3600
   cccctcttaa tggcatgccc ccagtggcct tgggccccca ctaggaccta caccttaaat 3660
   gtctgcagca ccttcaacag aacaatacgg acattaagat cctcacctag ggaccttgca 3720
   ggtggtttgg tggagggttg tacatggaaa ggtgaagcag cagaaaggaa gggtcaaggt 3780
   gacctcagtc agggttgagg tgccaaggct cacagtggtg gccacagtga ggtaaggggt 3840
   tggaagccac atgccaggct ggttcctata gaagcaagcg cacatggtct gcttggtcag 3900
   aactgaagga tggatagaac tcaccttccc acactaggac cagaggaata agaggtaggc 3960
   tttgcagagt cagctcagga gccttcctct gggatccaag gctaagtcta tgtactgtgg 4020
   gtggtatctg aggctgggaa aggctatcca gctcaatgga ccaccccatc ccctggtcca 4080
   tgactggcca gagccccaag cccttcctgt acctcatagc catctgggtg aatcaattcc 4140
   ttgccttacc atagaccact gaaaccaacc gtgggtgagc aggctgggca gatgaaccaa 4200
   ttatagagtc ttctttagct gctgtgtggg atccagcctg cctggagcat atctgaagga 4260
   cctgggcttg ggcaaggcct ttggctctca attatggctg caggctagga tctcatggac 4320
   actaaaatca accctagggc acacccacgt gggtacaaag gctccacaaa acccagtggg 4380
   ccatccacat gtcctccagg aataaactct tagcctttgt cacttgtatg cctggttcta 4440
   ccaggactgt gccacagcct agtatgatag ataacaagag aacctccaga aacccaaagc 4500
   aaggcatggt cagagccaag ttctcctggg gcctctctct gcctttgtct tgcaaattcc 4560
   ctcctctccc tgtgactcta atagatttct cttcctgtac cctgatatcc tcgtgtgtgt 4620
   gtgtgtgtgt gtgtgtgtgt gtgtgtgtgt gtgtgtgtgc gcatatgtgt gttctcatgt 4680
   gtatagtgac tggatgacaa cctttcatgt cgtgtcaaaa tggtgttcat ctcctttgag 4740
   acaggctaga gagaacccta gggagtcaag ccccaggaat ctacctgtct ccagtgcctg 4800
   agctctgagg ttacatgcat atgctattgc acctagtatt tttttaagat ttatttattt 4860
   atttatttac ttatttattt attatataag tgcactgtag ctatcttcag acacaccaga 4920
   agagggcatc agatctcatt acagatggtt gtgaaccacc atgtggttgc tgggatttga 4980
   actcaggacc tctggaagag cagtcagtgc tcttaaccgc tgagccatct ctccagcccc 5040
   cacacctagt gtttttgcac gggttctgag gatccaggtc aggccctcaa gcttgcaata 5100
   gaaacacttc gccaactgat ctgtagccct gtcccttgct gctctctgtg tataagaatg 5160
   ccagccaatt tgcatcggag cccactgggg agggctcgtt ttaccttgtc tttaaaggtg 5220
   ctattgccaa atacagtcac attctgatga atgtggtgga gcaggcctgg aattccagct 5280
   atggcagagg ctgaagcagg agggttacaa gctcatataa tatagtgatt tcaaggttaa 5340
   gcagggcgat ttactggact ttgtcttaca ataaaaagtc aaaagagaac tagagatgta 5400
   actcgttgat agaatgcttt ccaagcatgc acgagagcct gggtcaatcc ctagcccact 5460
   tagaaactac aagtggtggg gtgcacctgt aatgcgggag gtggaggcag gaggattaga 5520
   actccaagcc atcctactct atacatggtg agttcaagac cagctggggc tgcatgaacc 5580
   ttgtctggag tgggctgtaa tggtacagca cctacctagc aactcaagaa gcctcggact 5640
   ccatcgtcag acaggcacac cgcagggccc tggaggtggg aatgttagca aggagatctg 5700
   ggagcagttc agtggatgag tgtgctttcc acccggaaca tcctgggttc ccacggatct 5760
   ctgactgctc tccttctcct gactggcccc cgctgtaggt gccgtctcct gggctatcta 5820
   cagcagccgc gcttttggct ttgagtgagt tcttaagtac atagcaaaca aaagcagaaa 5880
   cagaccgcaa gggatggcta gaagcgccag acacatggca aacatcagat caggctctga 5940
   agacacccag cagagaccga ctgtacaaac acacgtgcac accctggaat gggatgaaga 6000
   aaagtcagcc tgactcccaa agccagtagc tcagagagtg tgaacagcac atggctattt 6060
   agccacagga tgtacattat agccactggg aataccaggg tagctaaagg tttggcttca 6120
   gggctctaaa taatctcttg tcactctcga aaaatgtttt tccaggatac acagaagcaa 6180
   atgcacagat cggagatgac tcccacgggg gcaggcctga aggccaccat cttctgcatc 6240
   ttgacctggg tcagcctgac ggctggggac cgcgtataca tccacccctt ccatctcctt 6300
   taccacaaca agagcacctg cgcccagctg gagaacccca gtgtggagac actcccagag 6360
   tcaacgttcg agcctgtgcc cattcaggcc aagacctccc ctgtgaatga gaagaccctg 6420
   catgatcagc tcgtgctggc cgccgagaag ctagaggatg aggaccggaa gcgggctgcc 6480
   caggtcgcaa tgatcgccaa cttcgtgggc ttccgcatgt acaagatgct gaatgaggca 6540
   ggaagtgggg ccagtggggc catcctctca ccaccagctc tctttggcac cctggtctct 6600
   ttctaccttg gatccttaga tcccacggcc agccagctgc agacgctgct ggatgtccct 6660
   gtgaaggagg gagactgcac ctcccgacta gatggacaca aggtcctcgc tgccctgcgg 6720
   gccattcagg gcttgctggt cacccagggt gggagcagca gccagacacc cctgctacag 6780
   tccattgtgg tggggctctt cactgctcca ggctttcgtc taaagcactc atttgttcag 6840
   agcctggctc tctttacccc tgccctcttc ccacgctctc tggatttatc cactgaccca 6900
   gttcttgcca ctgagaaaat caacaggttc ataaaggctg tgacagggtg gaagatgaac 6960
   ttgccactgg agggggtcag tacagacagc accctacttt tcaacaccta cgttcacttc 7020
   caaggtgagg caaacacttg ggtcactggt cctggttgta ccagaggcag cactgggttt 7080
   ccatgacatc cccatgggtc atgtgtgaga tggacagagg tgactggttt ctgcctctgt 7140
   gtgcctggtg aagccctgga ggctaaaaaa aaagcaaact tggctcaaga tggcacagcc 7200
   tctgcctccc tacccaactg cctttctggg ctgtgtcata ctcctgagac tacggtggtt 7260
   gacccccaca ggaacaccca gctcacccgc tcacaggggg agctgcacac atatccctgt 7320
   ccaggaagat gctcatccat tcgatgatct cttactactt gtaagaccgg gcaggggtct 7380
   ttgaggggct tgagatcctg atcctccagg tttcttatta aacatcagca agtatggaga 7440
   cccccaaggg caaatgcctg aggcaacact gcagggaaca ttgctacctg tgaggtggaa 7500
   aagggagtga gctggaacag gctggaggag gaggccaagg gcaaacatcc ttgcaaagcc 7560
   ttggtaggtc agggtgggag tgagggtgga ggggctcaga ggtggcacac cctgtgagga 7620
   tgctctttct ccagtaggct gagcagggca gttttatttc tagaaggtca gactttggcc 7680
   cctgacagca ctgcaggggt gtgtgtgtga ggggtccata taccacccaa aggtgtgcag 7740
   tggtccaggg acactgttct gggaacagat gctagggtcc cagatcttca aattgaggtt 7800
   actagaggag gggtgcattg gtggggccag caggaagaca tcccggcatg gggtagaggg 7860
   atcagggatg actggccact gtgtcaatat ctttgcagtt tataaattca catggggaaa 7920
   gaccttggcc tgccttgaga ctgggaaagg gggaggccag aatgtcgagg gaacgagaca 7980
   gctctgagac tggtgaaggg tggctacacc tgtgtaagtt ggcgaggagc ctggaactga 8040
   gccacagtcc tcatgagaaa ccctgggaag acgatcactg agaaaggagc acttctaaag 8100
   ggcagcagtg ccggtcatgg cttgctgtag aggactgcag aaccagccgg gagggaatag 8160
   agagaggtga agggaaggga tggaggagga atgggaggag ggaggtaagg agggaagagg 8220
   agaagggtgg gagaaggaat gggaaggagg aggaaaaagg gggaggaatg gtaggatgga 8280
   ggaggaggaa ggggaggagg aagagaagga gaaaaggggg acgaatggga aggaggaaaa 8340
   aaagaggagg aatgggagga agaaaggagg aggaatggga ggaggaatgg gaaggaggag 8400
   gaaaaggggg agtgggagga gggaggagga ggaaggggtg gtggaagatg aggaggaatg 8460
   gaggagaaat ggtaaggagg agaaaaagga gaggaagggg aggaatggga ggagggagga 8520
   ggaggaggga gaggaggaag aatggggagg aataggaagg agaaaaaaag aggaggaatg 8580
   ggaggaggaa agggaggagg aaggggagga aggggagaag gaatgagagg agaaaagagg 8640
   aggaagaatg ggggaaggga tgggaaggag aaaaaagagg aggaatggga ggaggaaagg 8700
   gaggaggaag gggaggagaa aagaggagga aggggaggag gaggaaaaag gtcaaggaaa 8760
   agaggaaggg gaaatggagg aagagagaga gagagagaga gggaagaaaa ggaaggagat 8820
   gaggaggggg aagaggggca ggttccacga cactagggga catgactggg agaagtttac 8880
   gcaaaggaga aggagaaaaa tgggttgtgc cagctctcag agacctggat gctcacccgt 8940
   gtcctgtcct cactgctgcc tggccttttc tgcctatctc catctgccag aggcctgccc 9000
   tcttcctcac acccatgagg ttgcagatgt gcctcagaaa ctccaggtcc ccttagcaac 9060
   agccttgggg acagtttttg tatttgtatc tagtatttgt atctagtgag gcctcgaggc 9120
   cttgggcttg tggtctctcc agtgttcttt gtccagtact tgatgtcccc ggagcttgaa 9180
   tggccctggc aggttccatg acactagggg acatgactgg gagaagttta cgcagcccac 9240
   agtagggctg gtgagcagag aggagtctgg ggagcacggg gagaacgggg aacagacagc 9300
   ttcgggtcgc tctcgaagcc gtgccccatt tctcatttct cttttatgct agagacaaaa 9360
   attcagtgct ttccccctaa actgaagatt cctgaggccc ctgaagtgat tgtcagcgtc 9420
   tctctcatgt gagcttcgtg tgtgtgacag ggtctcactg agtaccttgg actggcttgg 9480
   actctgaaat acttcttacc tcagtgttct gagtgctggg atcacaggtg tgagccactc 9540
   tagtgacctt tagggaaggg cttttttttt tttttttttt tttttttttt gagacagggt 9600
   ttttctctgt gtagccctgg ctgtcctgga actcactttg tagaccaggc tggccttgaa 9660
   ctcagaaatc cacctgcctc tgcctcccaa gtgctgggat taaagcaggt gccccaccac 9720
   tgcctggctt agggaagggc tcttatcact caccagagag ggctctgagg tcaagaccat 9780
   tctacttcca cgcagtcgga acccagcaac cccttcttgc tggagttaca ctggggaccc 9840
   ctgacttcat acagcaggac caatcagagc aggtgctacg tgagccaagc aagcctgccc 9900
   ctcaatctca gccctatctc cctgaactcc taaatgtccc tttcgtggta aatgggaggg 9960
   cttgggggca tgtgtgagcc ggaattcagt gctgggtagg gcagtcataa ggagaatgca 10020
   acaatgtctg agtgcagtgc tggctgagcc ggtggcatca ggctggggtg gctcagcttg 10080
   gaacgtgttt ccttcctgtc ctgtccctag ttccctcctg ccctcagcag ctgggatggt 10140
   ggtggtgggg attcatccct gccccaaggc cctgtgttca gatgcatcgg tcactgtaga 10200
   gttacacctg tctccagggc aagggatgct ggacagtgga gtcttgcttg ggccaggagc 10260
   ctgggcagct gcattcaaat atgctgtgtg tgaggcaggg gccaaagtct ctagcaacct 10320
   gttgtgagta ggcagagaga aatggctctg gtgatgccac cgagttggga caggtcatgg 10380
   caggtttgtc taatcctgtc gagtgtccaa tcagagcagg ccccatgtga accaagggct 10440
   ctgggcacag ttggggtgtc tgtgtcggcc cactgatggc ctcaaaggac tccatggtga 10500
   cacagagaga gcaggactcc ggaagccgaa ccaggggctc agtgttgctg ggaatcgaat 10560
   gctgctctaa aaatatcttt tttttttttt tttttttggt atatgtgtgt ggtgtacgcg 10620
   tatgcatact gtacagagga acaggttgag tgtcttctat tttttcctta tttatttatt 10680
   tatttattta cttatttatt tatttataga cagcatttct taccaagcct ggggcttacc 10740
   atttcttcta ggctggccag tgagctccaa gggtccttct atcccctcat ccccagtgct 10800
   ggcattatgg acctgcataa ccatgtctca ctggcacaag gggctggggc tctaggctca 10860
   ggtcatcagg tgcatagagc aagtagttat ccatggagcc atctctccag accataatgt 10920
   ctaaggtttg aaaaagtgag tttgcaaacc agaacgggtc tcggttgagc ttgcttggtc 10980
   agagaggaac agaccgagtt ctccaggtgt cagatgtgag acctcggact ccacaccatc 11040
   atgggctgtg tctcctgctg ctcttgggga tcgctgtagg ggaaattacc caagtggctg 11100
   caatttacaa aagtatcaga acacctgagg gtgaagtctc cagggcagtg caggcgaggg 11160
   aggaagctga ggcaggagcc agggtggagg agtcccgccc ctgcttcccc acccctgttg 11220
   agcttctctt tggtctctgc tgctttcagg aacgatgaga ggtttctctc agctgcctgg 11280
   agtccatgaa ttctgggtgg acaacagcat ctcggtgtct gtgcccatga tctccggcac 11340
   tggcaacttc cagcactgga gtgacaccca gaacaacttc tccgtgacgt gcgtgcccct 11400
   aggtgagaga gccaccctgc tgctcatcca gccccactgc acctcagatc tcgacagggt 11460
   ggaggccctc atcttccgga acgacctcct gacttggata gagaacccgc ctcctcggta 11520
   ggaagtgtgc ggggaacctt ggggagtaga gctgcatgaa tggcatctgc tgcatgctca 11580
   cacatcagtc tgtccaggtc tgtccatgta aacagcatgt cctgttacca ccccctccct 11640
   ccctcctgcc atcatgagca gtgtggttgt gggtatggct gttatctgtg aggctatgga 11700
   tattcccttc ctccacaggg aggcagtgag ccaactgggg ctggtttagg gtttctattg 11760
   ctgcaacgaa gcaccatgac caaaaagcaa gttggggagg aacgggttta tttggtttac 11820
   acttccagat catagtccat cactgaagga agacaggaca ggaactcaaa caggacagga 11880
   acctgggggc aggagctgat gcagaggcca tggagggatg cagcttactg gcttgcttcc 11940
   cctggcttgc ccagcccgct ttattataga accaggaccg ccaccccagg gatggcacca 12000
   cccacaatgg gcagggccct tctccatcaa tcactaatta tgaaaatgcc ctacaggctt 12060
   gcctaatctg gaggcattct cttagctgag actcttagtt tgtgtcaagt tgacataaaa 12120
   ctatccagca cagataaggg ccattatctt attagcgttt tggttttaga gagggtctag 12180
   cactcgtggt aatccccctg cctccacttc tcaagtgcca gggtgatagg gatgaggtgc 12240
   tctgcctggc taatggcctg ttagtcaaag gcatctttgg cttttccagg gaaggcactc 12300
   cttatgcagc caggctgaga gcacagggca cacagctgcc caccaccagc aagctctcca 12360
   cagtcgatgt tactggtcct cctcctgttt caggaggttt ttgttgtgtt tgtttctgag 12420
   aagttccttg gggtgacaat gtcctgtatt gttgtgtgac ccttcttggg gagatgcaag 12480
   caaatatata ttcactccag atagggcacc aacttcagac ccaagaatca atcccttccg 12540
   agtagaattc ggtcattcag agcattcatt gcagttactt cgttgggcag catgggcgac 12600
   cactcacgga agctgcatcc ctggagctcc ctgcacaacc tggaggcagc tctaccagtc 12660
   ccctttcccc agcagtcatt tcctgattat accatgctgg gaggatcctg tgcagcttcc 12720
   agagcagctt tctccagctg tgtcttatgc acccccttcc cgcagggact gtttcaactc 12780
   aggacactga ctggctgagc gcttagtctc tttttcagtg ggtgggagct tcccagcccc 12840
   ctaaaggctt tatctccaca gggccatccg cctgactctg ccccagctgg aaatccgagg 12900
   atcctacaat ctgcaggacc tgctggctga ggacaagctg cccacccttt tgggtgcgga 12960
   ggcaaatctg aacaacattg gtgacaccaa cccccgagtg ggagaggtga gtgctgctct 13020
   ggctgtgtag cagggaaggg ggggtggatg gtcacacgga ggaagctgtg cccaagacca 13080
   gcagggcagg aaggaaagag cagcaggcac tgccaggact ctggctccca gtgacatggg 13140
   gaacagcggt ctcccttggc tctcttggca tctctagttc caggcccatc tctgttcctg 13200
   cttagtccag ccttgcatcc tccataaagc tgtggtagat ggggaatgcc ccatgccctg 13260
   gtctgcagtt tctttgggga gcatctcaac catagacaga gagctatgta aaacagagga 13320
   tgtgcctgga ttctgttggc attgccaagg atgctgagaa acaggggagt cactgtagaa 13380
   gacatgaccc cactgtgctg tttagccaaa tatgggagag taccaagatg gcaggtgaac 13440
   aaggggacag gggcatgttg agcagcagcc tctatgggag ctgggtgttg gtgcccagcc 13500
   cccacttaga ggacaggtgt gtccacactt ccttctggca ggctggacag tgtccccctt 13560
   tggggcggtt gagccagtcc ctggcctgta acccctcctt aaggagagat ctctctgctt 13620
   tgcaggttct caatagcatc ctcctcgaac tcaaagcagg agaggaggaa cagccgacca 13680
   cgtctgtcca gcagcctggc tcaccggagg cactggatgt gaccctgagc agccccttcc 13740
   tgttcgccat ctacgagcag gactcaggca cgctgcactt tctgggcaga gtgaataacc 13800
   cccagagtgt ggtgtgaggc cttgtgccta gccatggaga caaggccggt gtcggagaac 13860
   cgttctgggc aaaactcagt gctgtcaccc ctggctcccc atcacgcctt gtagcgcggc 13920
   agaggccgtc tccttggaga ctgcgctgac cgagaataaa tgatgagcag cagagcctcc 13980
   tgggatgtgg gtttgtttgg atactggggt gacagccaga agctggcact ctgcacagga 14040
   ctgccactct ggaagaaatt tggaccaaaa aactgtttgt gacaccaaaa agcacccccc 14100
   ctttttttta tttgaggaca gaaattgggt tttaacatta aaatgcacat tatcccctta 14160
   ttttgggtct gtaattagct cactgagtat tagaccatga cgtccagagt cctacaatgt 14220
   cttacgactt cttatccctg gcaccatgat ttgaaaacat attcctcact gtgctgagaa 14280
   ttgtctttca gtgataagct aaacacagta ttatgggcag agcagacatg cttagtttag 14340
   ctgacaggct gagcaacatt cctgaagctc ttgacaagga ggccaatgtg tgccggccag 14400
   gtggtagagg agagggctgt caccaagagg ccttgagaaa ggacctgggc ggaggggcct 14460
   tcctttactg ctaccacctc tctgcctgtc cccttgagat caaatctgca gcttccagat 14520
   ctaggagtgt gctgaggaag gagcaagaca ggccgaggta gtggtgtgtg cgtgtgcgtg 14580
   tgtgtgtgcg tgcgtgtgtg tgtgcgtgtg tgtgcatgtg tgtgtgtgca tgtgcgtgtg 14640
   agcatgtgcg tgtgagcatg tgcatgtgtg cgcgcgcatg tgtgcacgtg tgtgtgtgtg 14700
   tgtgcgagcg cgtgtgcatg tgtgtgcatg tgtgtgtgtg tgtgtgtgca tgtgcatgtg 14760
   cgtggcaggc tgcctcctcc ctgtacagac tgggaagcag gtgagagcat tgtagcatgc 14820
   tagcctcagg cgaaggccag agagtacagt aaagcccaca gggcacagac agtggggaat 14880
   cctgtgttag aacatggagt cacctctctg gcctaagctg gagtttgact ctgtgtgagc 14940
   atagcctaac ctcacagacg gcttctctgg agcttggagc tgagaccata gggtcctgta 15000
   agtacaggtg gggcataaag tgtgttcctg tgaccggggt ccactctgag gcccggcttc 15060
   tctcatccta gtcctgttgc ctcctgaaaa ctcctctctg gcttcctgta cctcaaaggc 15120
   acttaccctg tgcgtgtggc aatctagcta gtccaacccc tgcttgtgcc aggcaacata 15180
   gtgagggacc tgtgtaccat ggttaactag aggccaactc ctcactctcc ctggcctatg 15240
   ggcttacagg gatgctaaga aaggccctgg tccagaggga ggtggcgtga acttctcatg 15300
   gctgtgacaa aatagcagac aaagcaatgt aagggtttat tctgggtcat gcctgagtgg 15360
   tggggagagt gtaggagggg ggcacaagac agctggccac ttacagtgaa gacacaccca 15420
   tggagatcac acatacacac acacacacac acacatacac acgctatacc acagccacac 15480
   atggggaagg ggaggggccg aggctggtgt tcaactcatt ttcttagccc atgggatgct 15540
   gctgtcaatt cttacgaggt atctcccaca tcatttaacc ccacctagaa actgcctcac 15600
   aggcatatcc aggggcttgt ttcttggatg accctaaatt ctatcaggct gataagatga 15660
   accatcctag gagtccatgg gtgtcttagt cagggtttct attcctgcac aaatatcatg 15720
   accaagaagc aagttgggga ggaaagggtt tattcagctt atacttccac actgctgttc 15780
   atcaccaaag gaagtcagga ctggaactca agcaggtcag gaagcaggag ctgatgcaga 15840
   ggccatggaa tgatgttgct cactggcttg cttcccctgg ctcgctcagc ctgctctcct 15900
   atagaaccca agactgccag ctcagagatg gtcccaccca cacgggacct ttcccccttg 15960
   atcactaatt gagaaaatgc cttacagttg tatctcatgg aggcatttcc tcaactgaag 16020
   ctcctttctc tgtgataact ccagctgtgt caagttgaca caaaactagc cagtacaatt 16080
   gaccccttgt caacttgaca cacaaacaca tcactagtaa gcctcaaccc ttactttctt 16140
   attcatcccc aagatctaaa taactttaaa agtcccacag tctttacata ttcttaaaat 16200
   a 16201
<210> 13
   <211> 1958
   <212> DNA
   <213> Rattus norvegicus
<220>
   <221> CDS
   <222> (201) ... (1634)
<400> 13
   ccttgctcca tcttggctaa gcctggattc ccatggtccc ccgacctggg tcctccccca 60
   gcctctgtac agagtagcct gggaatagat ccatcttcac cccctcgagt ataaataagg 120
   ctgcttggtt caccagggga tagctgtgct tgtctgggct ggagctaaag gacacacaga 180
   agcaagtcca cagatccgtg atg act ccc acg ggg gca ggc ctg aag gcc acc 233
                         Met Thr Pro Thr Gly Ala Gly Leu Lys Ala Thr
                         1               5                   10
   atc ttc tgc atc ctg acc tgg gtc agc ctg aca gct ggg gac cgc gta   281
   Ile Phe Cys Ile Leu Thr Trp Val Ser Leu Thr Ala Gly Asp Arg Val
               15                  20                  25
   tac atc cac ccc ttt cat ctc ctc tac tac agc aag agc acc tgc gcc   329
   Tyr Ile His Pro Phe His Leu Leu Tyr Tyr Ser Lys Ser Thr Cys Ala
           30                  35                  40
   cag ctg gag aac ccc agt gtg gag acg ctc cca gag cca acc ttt gag   377
   Gln Leu Glu Asn Pro Ser Val Glu Thr Leu Pro Glu Pro Thr Phe Glu
       45                  50                  55
   cct gtg ccc att cag gcc aag acc tcc ccc gtg gat gag aag acc ctg   425
   Pro Val Pro Ile Gln Ala Lys Thr Ser Pro Val Asp Glu Lys Thr Leu
   60                  65                  70                  75
   cga gat aag ctc gtg ctg gcc act gag aag cta gag gct gag gat cgg   473
   Arg Asp Lys Leu Val Leu Ala Thr Glu Lys Leu Glu Ala Glu Asp Arg
                   80                  85                  90
   cag cga gct gcc cag gtc gcg atg att gcc aac ttc atg ggt ttc cgc   521
   Gln Arg Ala Ala Gln Val Ala Met Ile Ala Asn Phe Met Gly Phe Arg
               95                  100                 105
   atg tac aag atg ctg agt gag gca aga ggt gta gcc agt ggg gcc gtc   569
   Met Tyr Lys Met Leu Ser Glu Ala Arg Gly Val Ala Ser Gly Ala Val
           110                 115                 120
   ctc tct cca ccg gcc ctc ttt ggc acc ctg gtc tct ttc tac ctt gga   617
   Leu Ser Pro Pro Ala Leu Phe Gly Thr Leu Val Ser Phe Tyr Leu Gly
       125                 130                 135
   tcg ttg gat ccc acg gcc agc cag ttg cag gtg ctg ctg ggc gtc cct   665
   Ser Leu Asp Pro Thr Ala Ser Gln Leu Gln Val Leu Leu Gly Val Pro
   140                 145                 150                 155
   gtg aag gag gga gac tgc acc tcc cgg ctg gac gga cat aag gtc ctc   713
   Val Lys Glu Gly Asp Cys Thr Ser Arg Leu Asp Gly His Lys Val Leu
                   160                 165                 170
   act gcc ctg cag gct gtt cag ggc ttg ctg gtc acc cag ggt gga agc   761
   Thr Ala Leu Gln Ala Val Gln Gly Leu Leu Val Thr Gln Gly Gly Ser
               175                 180                 185
   agc agc cag aca ccc ctg cta cag tcc acc gtg gtg ggc ctc ttc act     809
   Ser Ser Gln Thr Pro Leu Leu Gln Ser Thr Val Val Gly Leu Phe Thr
           190                 195                 200
   gcc cca ggc ttg cgc cta aaa cag cca ttt gtt gag agc ttg ggt ccc     857
   Ala Pro Gly Leu Arg Leu Lys Gln Pro Phe Val Glu Ser Leu Gly Pro
       205                 210                 215
   ttc acc ccc gcc atc ttc cct cgc tct ctg gac tta tcc act gac cca     905
   Phe Thr Pro Ala Ile Phe Pro Arg Ser Leu Asp Leu Ser Thr Asp Pro
   220                 225                 230                 235
   gtt ctt gct gcc cag aaa atc aac agg ttt gtg cag gct gtg aca ggg     953
   Val Leu Ala Ala Gln Lys Ile Asn Arg Phe Val Gln Ala Val Thr Gly
                   240                 245                 250
   tgg aag atg aac ttg cca cta gag ggg gtc agc acg gac agc acc cta     1001
   Trp Lys Met Asn Leu Pro Leu Glu Gly Val Ser Thr Asp Ser Thr Leu
               255                 260                 265
   ttt ttc aac acc tac gtt cac ttc caa ggg aag atg aga ggc ttc tcc     1049
   Phe Phe Asn Thr Tyr Val His Phe Gln Gly Lys Met Arg Gly Phe Ser
           270                 275                 280
   cag ctg act ggg ctc cat gag ttc tgg gtg gac aac agc acc tca gtg     1097
   Gln Leu Thr Gly Leu His Glu Phe Trp Val Asp Asn Ser Thr Ser Val
       285                 290                 295
   tct gtg ccc atg ctc tcg ggc act ggc aac ttc cag cac tgg agt gac     1145
   Ser Val Pro Met Leu Ser Gly Thr Gly Asn Phe Gln His Trp Ser Asp
   300                 305                 310                 315
   gcc cag aac aac ttc tcc gtg aca cgc gtg ccc ctg ggt gag agt gtc     1193
   Ala Gln Asn Asn Phe Ser Val Thr Arg Val Pro Leu Gly Glu Ser Val
                   320                 325                 330
   acc ctg ctg ctg atc cag ccc cag tgc gcc tca gat ctc gac agg gtg     1241
   Thr Leu Leu Leu Ile Gln Pro Gln Cys Ala Ser Asp Leu Asp Arg Val
               335                 340                 345
   gag gtc ctc gtc ttc cag cac gac ttc ctg act tgg ata aag aac ccg     1289
   Glu Val Leu Val Phe Gln His Asp Phe Leu Thr Trp Ile Lys Asn Pro
           350                 355                 360
   cct cct cgg gcc atc cgt ctg acc ctg ccg cag ctg gaa att cgg gga     1337
   Pro Pro Arg Ala Ile Arg Leu Thr Leu Pro Gln Leu Glu Ile Arg Gly
       365                 370                 375
   tcc tac aac ctg cag gac ctg ctg gct cag gcc aag ctg tct acc ctt     1385
   Ser Tyr Asn Leu Gln Asp Leu Leu Ala Gln Ala Lys Leu Ser Thr Leu
   380                 385                 390                 395
   ttg ggt gct gag gca aat ctg ggc aag atg ggt gac acc aac ccc cga     1433
   Leu Gly Ala Glu Ala Asn Leu Gly Lys Met Gly Asp Thr Asn Pro Arg
                   400                 405                 410
   gtg gga gag gtt ctc aac agc atc ctc ctt gaa ctc caa gca ggc gag     1481
   Val Gly Glu Val Leu Asn Ser Ile Leu Leu Glu Leu Gln Ala Gly Glu
               415                 420                 425
   gag gag cag ccc aca gag tct gcc cag cag cct ggc tca ccc gag gtg     1529
   Glu Glu Gln Pro Thr Glu Ser Ala Gln Gln Pro Gly Ser Pro Glu Val
           430                 435                 440
   ctg gac gtg acc ctg agc agt ccg ttc ctg ttc gcc atc tac gag cgg   1577
   Leu Asp Val Thr Leu Ser Ser Pro Phe Leu Phe Ala Ile Tyr Glu Arg
       445                 450                 455
   gac tca ggt gcg ctg cac ttt ctg ggc aga gtg gat aac ccc caa aat   1625
   Asp Ser Gly Ala Leu His Phe Leu Gly Arg Val Asp Asn Pro Gln Asn
   460                 465                 470                 475
   gtg gtg tga tgcctcctgt gtagccatgg agacaaggcc agcgtcagag           1674
   Val Val
   agctatcctg ggcaaaaatc agtgccttca cccctggctt cccgtcactc cttccagcaa 1734
   ggcagaggcc gtctccttgg agatggcgct aactgagaat aaatgatgag cagcagcctc 1794
   ctggggtgtg ggtttgtttg gacactgggg tgagagccag gagctggcac tctgtatagg 1854
   aggactgcca tcctggaaaa aaaaaatgga ccaaacaact gtttgtgaaa taaaaaaaaa 1914
   aaaattccct ttttatttga aaaaaaaaaa aaaaaaaaaa aaaa                  1958
<210> 14
   <211> 783
   <212> DNA
   <213> Rattus norvegicus
<400> 14
   tggaagggac ccagcggaac atcgccctgg ccttgctcca tcttggctaa gcctggattc 60
   ccatggtccc ccgacctggg tcctccccca gcctctgtac agagtagcct gggaatagat 120
   ccatcttcac cccctcgagt ataaataagg ctgcttggtt caccagggga tagctgtgct 180
   tgtctgggct ggagctaaag gacacacaga agcaagtcca cagatccgtg atgactccca 240
   cgggggcagg cctgaaggcc accatcttct gcatcctgac ctgggtcagc ctgacagctg 300
   gggaccgcgt atacctccac ccctttcatc tcctctacta cagttagagt acctgagccc 360
   tgctggagaa tctcagtgtg gagactctcc cactagtcaa cctctgatac tgtgttagtt 420
   caagacactg gtctcttaag cgcaacttgg tgtcctgctt aattggctta gcctgcgccc 480
   ttttgcattt tcatttagat gttgcttttt tactgatttc ttcacaccca cgcctatgtc 540
   atcttccttc ttctctacat acttgatttc ataaataaat aaatttatat ctcctcttgt 600
   ttacctaatt ttttttctat ccatgcactt gaattacttc tctcattttt tcattttgtt 660
   cctcttctca tcctattaag attcaattcc acctcactta ttagcacttt cctaccactt 720
   ttcttttcct ccatttctag ttaatacatc ccacctgcac atacttaata atttctacat 780
   ttc                                                               783
<210> 15
   <211> 553
   <212> DNA
   <213> Rattus norvegicus
<220>
   <221> misc_feature
   <222> 227, 540
   <223> n = A,T,C or G
<400> 15
   cacgagggga agcagcagcc agacacccct gctacagtcc accgtggtgg gcctcttcac 60
   tgccccaggc ttgcgcctaa aacagccatt tgttgagagc ttgggtccct tcacccccgc 120
   catcttccct cgctctctgg acttatccac tgacccagtt cttgctgccc agaaaatcaa 180
   caggtttgtg caggctgtga cagggtggaa gatgaacttg ccactanagg gggtcagcac 240
   ggacagcacc ctatttttca acacctacgt tcacttccaa gggaagatga gaggcttctc 300
   ccagctgact gggctccatg agttctgggt ggacaacagc acctcagtgt ctgtgcccat 360
   gctctcgggc actggcaact tccagcactg gagtgacgcc cagaacaact tctccgtgac 420
   acgcgtgccc ctgggtgaga gtgtcaccct gctgctgatc cagccccagt gcgcctcaga 480
   tctcaacagc atcctccttg aactccaagc aggcgaggag gagcagccca cagagtctgn 540
   ccagcagcct ggc                                                    553
<210> 16
   <211> 15751
   <212> DNA
   <213> Rattus norvegicus
<400> 16
   gaagccagcc atggaggtcc caggatgcag atggaggaag ctctgcctcc ctctcacact 60
   ctttcctaga gagtgaagga gcaagggacc atgggggctt caggccagtc agctaacccc 120
   ctcagagcag acggaggcta cacagccagc ctggaccaag agccctgttg tctgacttgc 180
   cactccaggt ggagtaaata agtctgtgga gtgatgacca gccagcagag actgacgcag 240
   aggaggctcg tgctcagaca gccttagtag caagggctac ctgctctcag tacagtgcgg 300
   ttacacctca gtctggtccc aggcttcctc acgctggaca attggctaga acagctccag 360
   cagtcacgtt gcctctgctg actggtttct catgaggccc acagaaatag ccagggtcag 420
   aatggctgac agtacaggac ctcgtcagat ggcaagtaca tcttcattcc tggctgtgtt 480
   cctcagcttg ggaattctcc aaaccccagg gcaaaggctc tttactgagg ctcccctgca 540
   tacagggctg gcgacatccc tgccatcact ggccagctcc atagccgccc ttctctcttc 600
   gcagagggct gaggctgaaa gttcctttgg atcgcatggc cagtctctct ggtcactacc 660
   catctcctga gatgaccgtg aggccttcat ctcccagact tctcacaggg gacaacaaga 720
   tgttcagtga gcttaagaat ccagccatct agtgcttgcc tagcaagggc aaggccctgg 780
   gttcggtccc cagctccgaa aaaaagaaaa gaaaaaaaaa aaaaagaatc cagccatctg 840
   tggtcagaaa cagagaggag agacctatct atcactatac ttgggaactc tactcttgtg 900
   tccccagcac agggcctcct gctgaacccc aaagctacta aggcatccac acactcctct 960
   aggaaatcta tgcaccactg ccctttcctg agctcttact ccttgtacat cccttggtcc 1020
   tctggcatcc ttagtcctga gtgcagaccg tcatggctgc agcctggagt gcagaccgtc 1080
   atggctgtag ccttgatgcc tccaacaact ggcttcccac tgagatagat cttctggtaa 1140
   ttattttaca tctgtgcgca gagaaggtca ctctctagag cccagctcag acaccatcaa 1200
   atcctgcatg gtccatctag tttcttctga gccagggact gctctgccaa tcctcccttt 1260
   ccgccttcac tttctagtgc cactttaggg taaaggcagg ttgtgcctgt gtcaactcca 1320
   tggccaagag attacttgac gtactggatg caaggcaagt tagtcgccca cccatggtgg 1380
   actacagggg caaccgtcta ccagacctac tcctgctgtc ttctcagctc ccacccttcc 1440
   ttgggatcca cccgtctcat tctctaaacc tccatgacta ttccacactc aagggcggtg 1500
   ctctgaaacc tccagcctcc aaaacagccg ccttccatcc acaagcccag aacattttgt 1560
   ttcaatatgg ctttaccaca gttgggattt cccaacctga ccagatgtgc tagaagtttc 1620
   ccaaaggtgc gggaagggac tggaagcccc tgtgcagaac agacaactaa cgtaattaca 1680
   accattcaag gtcgtccatc ctgagagccg ctgatgactt atgagaggtt catctcctgc 1740
   aggtaggtcc cttcctggca tgtcctgaga ggcttataag cagatcacga ggggcttacg 1800
   tcccaaggtc tgcatggtgg cttcagttgt cacaacccag ccagtcctgt gactgtgatc 1860
   ccagctccgg ggaactgggg taaatatgta actcaacgcc agcccttttt tttttttttt 1920
   tttttttttt tttgcctgaa gctcatctgc cactagggct tcctggaagg gacccagcgg 1980
   aacatcgccc tggccttgct ccatcttggc taagcctgga ttcccatggt cccccgacct 2040
   gggtcctccc ccagcctctg tacagagtag cctgggaata gatccatctt caccccctcg 2100
   agtataaata aggctgcttg gttcaccagg ggatagctgt gcttgtctgg gctggagcta 2160
   aaggtaagca gagccctctt gggtcctttc tggtccttcg tgggatctga ctgggcagga 2220
   gctgagcttc agggaagaag aggaggcagg ggttgggtag agacactgga aggggttgga 2280
   tggcatttgt cctggagtga ggagctagaa gtctgtggtg gcaggcgctt cagatgtgac 2340
   cactgacttg tgtctcctct cccgaaaggc tagatgtcca ggtaagctgt cccctgcggg 2400
   tcaccactct gggctcaccg gcaactgctt gtgttttgtt aggggatggc caggccttct 2460
   gcttcccgtg agcccagatg ggtcctcaaa ggcagaggga ctttcacagg ctcagcacta 2520
   gagtggctga gtccgctgaa cctcttccag ctcgaaggga gaacaaggct ccattgtttg 2580
   gactgcaagc agcaaggagg gacaggtccc atggtcatct caagtagcat ctctgtccag 2640
   gctcagtgct ggggcctggg tcttgtggct tctcaccacg tcctgtagcg tctcaactta 2700
   cccctctgca aacaggctcc ttgtggttcg aaggttctct agggctgggg ataagttagg 2760
   agcacccagt agcaaggaag aactttgata atgtcaagct aagcaaaaaa ggtagctcct 2820
   tattttcagt gtgtgtgtgt atgtgtatgt gtgtgcgtgt gtgtgtgtgt acatgtgtgt 2880
   gtgcatgtgt gtgtgcatgt atgtatgcac gtgtgtgtgc atgtgtgtgt gcatgtgtgt 2940
   gcatgtgtgt gcgtacacgt gtgtgctgtg tagtgtatgt gcatcttatt tattcattta 3000
   tttttgtttt tcgagacagg gtttctctgt tgtagccttt gctgtcctag aactcactcc 3060
   gtagagcatg ctggcctcag attcggagac ctgtctgcct cggcctcctg agtgctggga 3120
   ctaaatgatt ttttttttta aattttaaga cagggtctta ctgtgtagct ctggttgccc 3180
   tagaactcac aaaattctgt ctgcctctgc ctctgattct atcaccatgc cctgcccaag 3240
   gtatgtgtgt atttatatga atgtgacata tgtgtatgat gtgtgtgtgt gtgtgtgtgt 3300
   gaagtgtgtg tggtatatgg ggagtgtgga gactcaagag ccatgaacac agatagaggc 3360
   cagaggagga catcagctgt cctgccaggt actgtcctcc ttagtctctg gagacagggt 3420
   cccttactga agttggggct aggctggcag acaggaagcc cgggagatca tcctgtctcc 3480
   tccgtccacg tctctggagt tgggggatta tgtggtcatg actggtttgc tgagcctgcc 3540
   ccgggctcct cagttccttc tttctttcaa gaggaagtgt gcgtcgtgtc tagataccac 3600
   tcatggagtc aggtgtcagg tctgagtccc agctgtgtga cctggtgctg gccacaagac 3660
   ttgtctgttc ctctacaaaa cagcactgac gactccaccc ctggctgtcc caggatgacc 3720
   ttagggaggt gggctaacct gtggctgctc ggtagagaga aggcgaggga ggggtgtggg 3780
   agcccagggt agactttgag aggtgaagag gatgcactta gggtgaactt gactggcagc 3840
   agcttatggc tgagagcaaa cagtcacttg tgcctgagga cagtggtgac gcgcttcctg 3900
   ggacagtggc tgtggcacag cttccccctc cccccctgga cagaggccat tcacggggac 3960
   agcaatatgg gaagcattgt tttcacctcg atggctcacc ctgcccatgg gggaccgagg 4020
   caggctcgac cctactgtgc cttggtttat aatagcacag tgaacaggat gccgtcattc 4080
   ctgggaggaa tgggagggcc agtgggtaca ggcagagttt tctactctgc ttttcagaac 4140
   cacagaactt tgggaacatt gagtggatca gatgcatgtg ccaggagggt aaagtgcagg 4200
   acagtcagaa gccaaaggtg accagtggga tgaccagagc agagcaaaga gtaccttgcc 4260
   tgtctggagg caatattcta tgtgtggccc aggccaagtg ccacgacccg ggtgtggggg 4320
   gccagggtga cctcggagct ttgcaacacc agagtgacat cctgagatca ttaacttacc 4380
   aagagaaaag ttttgtcttg gctcgtggga ctggagtcac aggtcaaatt cagacagact 4440
   tgttgctcgg gaccttctgg gggtgttggc tatcaacagc aacagtggca tttggccaaa 4500
   caaattgctt cccatgtagc aaggaagcaa aaccagtgaa ggaaagacgc aggtcctgag 4560
   atcactcttt tttttttttt tttttttttt tgggtctttt tttcggagct ggggaccgaa 4620
   cccagggcct tgcgctttct aggcaagcgc tctaccactg agatgaatcc ccaatacccc 4680
   tgcgatcact cttaaaggcc tggccctact ggactcaggg cccccactag gacttgcatc 4740
   ctaaatgttt acagcacctc ccacggacca atgctgggca ttaagattgt cacacagggg 4800
   ggttggggat ttagctcagt ggtagggcgc ttgcctagca aacgcaaggc cctgggttca 4860
   gtccccagct ccagaaaaaa gaaaaaaaaa gattgtcaca taggaacctt gcgagggttt 4920
   gccgagcaca gcggaaggtg ggagaagcag aaaggaaggg tcaaggtgac ctccagtcag 4980
   ggttgaggca ccaaggccca gcggtggtcg ccatagtgag gtaaggggta ggaagccaca 5040
   catcaggctg gttccttcta ttgaagcaag agcacgtggt tcacacggtc agaactgaag 5100
   ggcagataga gctcgcctcc ccacaccagg atcagaggag taagacgtgg gtttttcaga 5160
   gtcagctcag gacccttctg ctaagatcgc aggctaactc tatgaactgt gtgtggtagc 5220
   taaggctgga gctgaggttc aaggagaaag gctatccagt tcaatggacc acctcatcct 5280
   tggtccatga ctggccagag ccccaagccc ttcctgtgct ccacagccat ctgggcagtt 5340
   ggcaaatcaa cccccggctg aagatagact gttgaagtga gcaggctggg cagacaggcc 5400
   agtcatggag tcttctgtag ctgctgtgtg ggatccagtc tgcctggagc acgtaacggg 5460
   caggaagggt ctggccttgg gcaaggcctt tggttcccaa ttctggctgc aggctaggct 5520
   ctaatggaca ctaaagtcaa tcaatcctag ggcacaccca tgtgggtaca acggctccac 5580
   agagtgggca gtcctcatgt ccttctctag ggatgagctt atagcctttg tcacttgtat 5640
   gcctggttcc accaggacta tgctacggtc tagtatgata acaaaaggac cttcagaaac 5700
   ccaaagcaag gcatggtcag agccaagttc ttctgaggcc tctctctgcc tttggcttgc 5760
   agattccctc ctctccctgt gactctaata gatttctctg ttcctgtatc ctgatatccc 5820
   tttgtgtgtg tgtgtgtgtg tgtgtgtgtg tgtgtgtgtg tgtgtgtgca tatgtgtgtt 5880
   tgtgtgtgtg catatgtgtg tatttgagtg tgtgtgtctg agtgtgtgca tatgtgtgta 5940
   tgtgagtgtg tctgtgtgtg tgtatatgtg tgtgtgtgca tatgtgtgta gagcctggat 6000
   gaccaccttt tgtgtcatgt caaaattgtg tccatctcct ttgagacagg gtctctattg 6060
   gcttagagct cacacattag gctagattaa tttgccagag aaccctgggg agtcaaaccc 6120
   caggaatcta cctgtcttcg gtgcctgagc acggaggtta cgtgcatctg ctgccatacc 6180
   taaaggttta ttatttattt tatatatatg agtacactgt cgctgtcttc agacacacca 6240
   gaagaagaca tcagatccca ttacagatgg ttgtgagcta ccatgtggtt gctgggattt 6300
   gaactcagga cctctgaaag cactgtcagt gctcttaact actgagccat ctctccagcc 6360
   ccacacctac tagttttaca tgggtcctgg ggatccagcc caggtcctca ggcttgcgat 6420
   agaagtactt cagcacctga tctgtatccc tgtccctcct tgccctctgt gtagaaaaat 6480
   gccagtcaat ttacatccga gcccaccagg agggcctcat cttacttggt cgtgtcttta 6540
   aaggctctgt ctccaaatac agtcacattc tgatgaccgt ggtggagcag gcctggaatc 6600
   ctggctatgg cgggggatga agcagaaggg tcacaggctc aaagctgcct agaagagaga 6660
   gtggtttcaa agttagccag ggcgacttat tggaccttgt cttaaaaaaa aaaattttta 6720
   gtcaaaagag tgctagaggt gtggctcgtt gggagaatgc tttctgatca tgcgtgagag 6780
   cccaggttga tctccagccc acttataaac tacaaggggt ggggtgcagg agctggaggc 6840
   aggaggatca gaacttccaa gtcatcctaa tctatctagg ggttcaagac caactgggtc 6900
   tgcatgaaat cttgtctggg gtgggatgta acggtaaagc acctacttag caactcaaga 6960
   ggccctgggt tccatcccca aacagccaca ctccagggtc ccggaggtgg gagcaactcg 7020
   gcgggtgaca gtgcgttcca cacggatcat cctgggttcc catggatctc tgactgcact 7080
   cctcctcctc ctaactgacc cgagctgtag gtgcctcctc ctgggtcgtc tacagtagcc 7140
   ttgcttcgag ccttgagtga gttcttaagt acatagcaaa caaaagcaga tagcaaggga 7200
   cgggtagaag caccagaaat acagcaaaca tcggatcagg ctctcaagac gtccagcaga 7260
   gacgactgaa caagtgcacc cacacaccct ggcatgggga tggggagtca gccctatttc 7320
   caaaagccag tagctcagag agcgggagca gcacatggca gtttagccac agagtgcaca 7380
   ttacacacag tcatggggaa taccagggca actaaaggtt tggcttcagg gctccaaata 7440
   accacttttc actctcacaa aaaacccatt attccaggac acacagaagc aagtccacag 7500
   atccgtgatg actcccacgg gggcaggcct gaaggccacc atcttctgca tcctgacctg 7560
   ggtcagcctg acagctgggg accgcgtata catccacccc tttcatctcc tctactacag 7620
   caagagcacc tgcgcccagc tggagaaccc cagtgtggag acgctcccag agccaacctt 7680
   tgagcctgtg cccattcagg ccaagacctc ccccgtggat gagaagaccc tgcgagataa 7740
   gctcgtgctg gccactgaga agctagaggc tgaggatcgg cagcgagctg cccaggtcgc 7800
   gatgattgcc aacttcatgg gtttccgcat gtacaagatg ctgagtgagg caagaggtgt 7860
   agccagtggg gccgtcctct ctccaccggc cctctttggc accctggtct ctttctacct 7920
   tggatcgttg gatcccacgg ccagccagtt gcaggtgctg ctgggcgtcc ctgtgaagga 7980
   gggagactgc acctcccggc tggacggaca taaggtcctc actgccctgc aggctgttca 8040
   gggcttgctg gtcacccagg gtggaagcag cagccagaca cccctgctac agtccaccgt 8100
   ggtgggcctc ttcactgccc caggcttgcg cctaaaacag ccatttgttg agagcttggg 8160
   tcccttcacc cccgccatct tccctcgctc tctggactta tccactgacc cagttcttgc 8220
   tgcccagaaa atcaacaggt ttgtgcaggc tgtgacaggg tggaagatga acttgccact 8280
   agagggggtc agcacggaca gcaccctatt tttcaacacc tacgttcact tccaaggtga 8340
   ggcaaacgct tgggtcattg gtcctggctg ggtttctatg gcatccccat ggttcatgcg 8400
   tgagagggac agagatgaca ggtttctgcc tctgtgttac cagtgactcc ctggaggcca 8460
   aaaaagataa cctgactcaa gatggcatgg ccctgcccaa ctgcccttct gggctgggtg 8520
   atgctcttga ggctagagtg gctgacccac gcaggaatac ccagcttact ccctccctca 8580
   cacggggagc tgccgtatat ccctgtctga gaagttgccc atctgttcta tggcctctta 8640
   ctacttgtaa accaggcaga gggctctgag gggcaggaga tcctgaccct cctggtttct 8700
   caagagacgt cagcaagtat ggagacaccc cccccccccg agcaaatgcc tggggcagta 8760
   ctgcagggaa cagcactaac tgaagaggaa gggaatgagt tgtaacaggc tggagaaggc 8820
   cacaggcaga catccttgca aagccttggg agaccaggtt aggagtgtgc aggggctggg 8880
   ctcagagagg gcagaccttg ggaggatgct ctttttccag tgggctgagg gggacagttt 8940
   tattcctagg acattggacc ttggcccaca acagcatggc aggtgtgtgt gtgtgtgtgt 9000
   gtgtgtgtgt gtgtgtgtgt gtgtgtgtat gtgcagggtg tgcagtggtc cagggacacc 9060
   atcctgagaa catgtgccag ggccccaggt cttcagattg atggttacta ggggaggggt 9120
   gaccaatggg gccagcagca gggagacatc acagtctggg gtagatgtag aaggatcagg 9180
   gatgactggg ccactctgac aatatctttg cagcctataa attcatgtgg ggaaagacct 9240
   tggcctgctt tgagactggg aaaggtgtgg ggggtagaat gtggacggaa agaggtaacc 9300
   ctgaggctgg tggagggtgg ctacacccat gtgagtcgga aaggaacccg gaactgagcc 9360
   acagtccctg tgggaaaccc tgggaggact atcactgaga aaggagcact tctacagggc 9420
   atcagtgtca gtgatggctt gctgtagaga atgcctgttt tcacacccca gactctggag 9480
   ccagcaggga acagaggagg gcgaagggaa ggagtggagg aggggggggg agagggaggt 9540
   gaggagggag ggggagagga gtgagaacag gaacgggaag gaggaggaat gggaggaaga 9600
   aaagcagagg aaggggaagg agaaggaaaa gaggaggagt gggaagaggg gaaggaggat 9660
   tgggggaggg agggagagga ggaatgggag gaggaaaaga ggagaatggc aggaggaagg 9720
   gggaataagg ggaggaggaa gaggagaaag aggaaaggga gggggaagtg gaggaagagg 9780
   aaaaagaggg tgggaaaatg aaggaagagg aagagaggga gagggaagaa aagaaaaaag 9840
   gagacgagga aggaaaagag gagaggttgg gggtggggca aaaaatgagt tgtgccagca 9900
   ctcagaggcc tggatgctca cttgtactct gtcctgatct cactgctgtt tggcctttcc 9960
   tgcccatctc catccgccag aggcttgtcc tcttcctcac acccatgagg ctgcagactg 10020
   cctcacaaac tccaggtccc cttagcaata gcattgggga cagttaagca tttgtatcta 10080
   gcgaagcctc aaggccttgg gctcgtggtc tctacagtgt tctttgccca gtacttgatg 10140
   tctccagagc ttgaatagcc ctggtgggtt ccatgacact aagggacatg accaggggaa 10200
   gtttactcag tccataatag gactgctgaa cagggaagga tccggggagc atagggagaa 10260
   caggaaacag acagcttcac atctctctca aagccttgtc ctgtttcaca tttctcattt 10320
   ctttttcttc catttattta ttatgtatag agttctgcct gcaggtacac ttgcacacca 10380
   gaagggggca tcagttccca ttacagatgg ttgtgaacca ccatgtggtt gctgggattt 10440
   gaactcagga cctctggaag agcagtcagt gctcctaacc gctgagtcat ttctccagcc 10500
   caatgcctgg ctcttacgag ggactggggc tgtaggtgca ggtcatcagg cctatagagc 10560
   caagttacca tggagccatc tctccagacc ataataatgt ctgaggttct aaagagtgag 10620
   tttgcaaacc agaacaggtc tgggttgagc ttgtctgttc aggtaggagc agactgagtc 10680
   cctcgggtgt cccatgtgag acctcagact ccacaccaac atgggctgtg ttttttgctg 10740
   ctcttggggt ctctgcaggg aactcaagtg gctacaatta gaagtagcat cagagagcac 10800
   ctgaaggtga catctccagg tcagtacagc atcacagagg gagtagaggt gaggaagctg 10860
   aggcaggagc cagggtggaa gagtcccagc cctgcctccc catcctaccc ccgttgagtt 10920
   tcctttggtc tctgttgctt tcagggaaga tgagaggctt ctcccagctg actgggctcc 10980
   atgagttctg ggtggacaac agcacctcag tgtctgtgcc catgctctcg ggcactggca 11040
   acttccagca ctggagtgac gcccagaaca acttctccgt gacacgcgtg cccctgggtg 11100
   agagtgtcac cctgctgctg atccagcccc agtgcgcctc agatctcgac agggtggagg 11160
   tcctcgtctt ccagcacgac ttcctgactt ggataaagaa cccgcctcct cggtaggaag 11220
   catgtgggac ccttgggaag tagatttgca tccaatgcac gctcacacgt cagcctgtcc 11280
   aggtctgtcc tgtcaccacc cctcccacat cctgccatcg ggagcagtgt ggttgcgggt 11340
   atggctgtta tctgtgaggt tacggatact accttcctcc acggggaggc aaggacccaa 11400
   ctggggctgt ttttagggtt aatatttgct gcaaggaagc atcgtgacca aaatgcaagt 11460
   tggggaggaa agggtttatc tggtttacac ttctagatca tagcccatca ctgaaggaag 11520
   acaagacggg aactcaaacg gcaggaacct gggagcagga gctgatgcag aggccatgga 11580
   ggggcgctgt tcactgactt gttcctcctg gcttggccag cctgctttat tatagaaaca 11640
   ggaccaccat tccagggtgg aaccacccac tatgggctgg cccctccccc catcaatcat 11700
   taattaagaa aatgccctac aggcctgcct acagcctgat cttaaaggag gcgctctctt 11760
   agttgagact ctagcttgtg tcaagttgac ataaaactat ccagcagggg tgggcactat 11820
   ctcattagca ttttggtttt tagagagcat ctagagtttg tggtaattcc cccacctctg 11880
   cttctcaagc gcctggatga tagggttgag gtgctctgtc tgtcaggcta atagccagtt 11940
   actcaaaagc atctttggct tttccaggga aggtgttcct cacgcagccg ggctgagaac 12000
   gaagggcaga cagctgccca ccagccccga gctctccaca gtagatgttc caggttctcc 12060
   tcctgtttca ggaggttttg ttgtgtttgt ttccaagaag ttccttgggg tgacaacgtc 12120
   ctgtgttgtg tggcccgtct gggcatatac gcaagcaaat atgtattcac cccagatagg 12180
   gcagggactt cggagcgaag aatcaattcc ctccgagtag gattcggtca ttcagaacat 12240
   tcattgtggt tacctccgtg aggcagcatg ggcgaccacc cgtggaagct gcatccttgg 12300
   agctccctga acaacctgag gcatctctac cagtcctcct accccatcca ctatttcctg 12360
   tttatacaat tcggggagga acccgtggct ctcccagctt ccagatgtgt cttatgcaac 12420
   tgtttcaact cgggacacag ctgcacaaca ctggctggct gagcagttac tctcctttct 12480
   cagtgggtgg gagctcccca gcaccctaaa ggcattatct ccacagggcc atccgtctga 12540
   ccctgccgca gctggaaatt cggggatcct acaacctgca ggacctgctg gctcaggcca 12600
   agctgtctac ccttttgggt gctgaggcaa atctgggcaa gatgggtgac accaaccccc 12660
   gagtgggaga ggtgagtgct gccctggctg tgtctcaggg gagggcgggc aggcggatgg 12720
   ccacatggag gaagctatgc gcagggtcag cagggcagga aggaaagagc aggcccagta 12780
   ggcactgcca gggctctctg gctcccagtg gcatgggtca cagcagtctc cctcagctct 12840
   cttggcatct ctgttcctgc ttagtccagc tttgcatcct ccacgaagct gaggtagatt 12900
   aggacgggga tgcctcgatg ccctgatctg caacttcttt ggagagcatc tctgcagaag 12960
   acagagaact atgtaaaaca gaggaggtgt ctggatactg ttgacgttgc aaaggatgct 13020
   gggaaacaga ggggagtcac tagagaaagc atggccccgc cgtgctgatt agccaaatat 13080
   tggagagtcc cctcaagatg gcaggtggag acggggcacg ctgaacaggg gcacgacacc 13140
   caggtgggag ctgggtgtcg gtgcgcagcc cccacgtaga ggacagtgtg tacacattgc 13200
   cttctggcag gctggacagc gtcccccctc ggggcagttg agcccagtcc ctgtcctgta 13260
   atccctcctg aaggagagat gtctctgctt tgcaggttct caacagcatc ctccttgaac 13320
   tccaagcagg cgaggaggag cagcccacag agtctgccca gcagcctggc tcacccgagg 13380
   tgctggacgt gaccctgagc agtccgttcc tgttcgccat ctacgagcgg gactcaggtg 13440
   cgctgcactt tctgggcaga gtggataacc cccaaaatgt ggtgtgatgc ctcctgtgta 13500
   gccatggaga caaggccagc gtcagagagc tatcctgggc aaaaatcagt gccttcaccc 13560
   ctggcttccc gtcactcctt ccagcaaggc agaggccgtc tccttggaga tggcgctaac 13620
   tgagaataaa tgatgagcag cagcctcctg gggtgtgggt ttgtttggac actggggtga 13680
   gagccaggag ctggcactct gtataggagg actgccatcc tggaaaaaaa aaatggacca 13740
   aacaactgtt tgtgaaataa aaaaaaaaaa attccctttt tatttgagaa cacaaagtgg 13800
   gttttaacat taaaatgcac actgtcccct tgttttgggt ttgcaattag ctgagtgtga 13860
   gaccacgacc tccgagtccc ctatgccctg agttcctatc cctggcacca ctcattcctc 13920
   acttagctga gagttcctct cagtggtaaa ctcagacatg gtgttaccac tggcagaaca 13980
   gacaagctag tttccctgac agactgagca atgtgactgc agctcttgac agggaggcca 14040
   acgtatgctg gctgggtggt ggacgagagg gctgtcacta agtggcactg agagacgacc 14100
   tgggcggagg gggcttcctt tatatgtgtc caaagaccac acagggttgc tactgcttct 14160
   ctacctgtcc gcttgagatt aaacctacag cttccagatc tgggactgtg ctgaggaagg 14220
   agcgagacag ggtgaggtag tggtgtgtct gtgcgtgtct gtgtgtgtac gtgtctgtgt 14280
   gtacgtctct ctctctctct gtgtacgtgt gtgtgtgtgt gtgtgtgtgt gtgtgtgtgt 14340
   gtgtgtgtgt gtgtgtgagg gtgccttcct ccctgtacaa actgggaagc aggtgaacaa 14400
   tgtagtgtgt taaccacagg aaaagaccag agtgcctata gaatccacag ggcacagaca 14460
   gtgggaaatc ctgtattaaa acatggagtt gcctctctgg cctaagctgg agttttgact 14520
   ctgcatgagc ttagcctaac cttgaaggca gggctgctct gaagcttgga ttgagactag 14580
   agggtcctgt aaggacaggt agagcctaga acatgttcct gtgaccgagg tccacccggg 14640
   gttgggggtg gagcttaaga cggcctgcca ctcccagtga acacacacac atggacacca 14700
   caccacaccc acatgggggt cgggggagag gctgggactg gtgtacagct cactttctta 14760
   gcccacggga tgctgccgtc aagtcttaag aaggttcccc acatctcttc gtcccacata 14820
   gaaaccctca tacatgccca gggctttgtt tcttggatga ctgaattcca tcgggctgac 14880
   aagacgagct gtcataggag tctgtgggag acagtagcaa atggacatcc ggccaacatt 14940
   agggttagtc cttaccaaag gctactgtct cctacagcag ccaggcttgc caagggttgg 15000
   ttgttcccca ctcctcgcta tacacttgag tctggcctgc accgtcttgc taaccagcct 15060
   ggtgtcagtg ccctggtggg acacagcaca gctgagctct gtctgtgact gcgctggaag 15120
   ccagcacaag tcctgctgct cacagagagc cgccgtcctc ggcatgagca gctcgtccac 15180
   cccagtgctg ctggcttggc ccctcagcca tgggtcctcc caatggagaa cagcttgaac 15240
   cacaggtggt agtaaggacc cgcaggccct taggggaggt gacaacacac attgattttg 15300
   aacttctgcc tctacgacag aatcatttct tcccaatcag ctgccatatt tgcggtcact 15360
   gaaagccgag tgagaaatga atgcatgtcc tttgactaca taggagggag atggtgggct 15420
   tcaggtccct gaatgacaga ctggcctcct cctcacaggc ttctcatact ggaaagcagt 15480
   aaactgcgtg gagacgggtt tttagtgtga atgttgaagc tgggagaatg tgtaacttca 15540
   actttaaacc tgtctgaata aaactggctc tcagcgggga aaccaaggaa aggtaacaag 15600
   ggagctgttt tggggagcca gattcaagtc tgagaccgtt agcttttgcc tgaacaacct 15660
   gctctcaagg acagagacat cttagggaga ctggaattca atggtttgga gtcagagcca 15720
   acagggtaag agaggcccac tcttgacctc t                                15751
<210> 17
   <211> 20
   <212> DNA
   <213> Artificial Sequence
<220>
   <223> Synthetic oligonucleotide
<400> 17
   cactgatttt tgcccaggat 20
<210> 18
   <211> 21
   <212> DNA
   <213> Artificial Sequence
<220>
   <223> Primer
<400> 18
   agcacgactt cctgacttgg a 21
<210> 19
   <211> 21
   <212> DNA
   <213> Artificial Sequence
<220>
   <223> Primer
<400> 19
   ttgtaggatc cccgaatttc c 21
<210> 20
   <211> 20
   <212> DNA
   <213> Artificial Sequence
<220>
   <223> Probe
<400> 20
   aacccgcctc ctcgggccat 20
<210> 21
   <211> 21
   <212> DNA
   <213> Artificial Sequence
<220>
   <223> Primer
<400> 21
   gctttggacg aatcttgctc a 21
<210> 22
   <211> 16
   <212> DNA
   <213> Artificial Sequence
<220>
   <223> Primer
<400> 22
   tccccgctcc tccagg 16
<210> 23
   <211> 22
   <212> DNA
   <213> Artificial Sequence
<220>
   <223> Probe
<400> 23
   aaaatgccct cggtccggga aa 22

## Description

### FIELD OF THE INVENTION

The present invention relates to compounds for use in treating a RAS pathway related disease by administering an angiotensinogen modulator to an animal. The present invention also relates to compounds for use in treating hypertension and organ damage by administering an angiotensinogen inhibitor to an animal.

### BACKGROUND OF THE INVENTION

Angiotensinogen (AGT), also known as SERPINA8 or ANHU, is a member of the serpin family and is a component of the renin-angiotensin system (RAS) pathway (also known as the renin-angiotensin-aldosterone system (RAAS)). It is primarily produced in the liver and is released into the circulation where renin converts it into angiotensin I. Angiotensin I is subsequently converted into angiotensin II by angiotension converting enzyme (ACE). Angiotensin II is a peptide hormone which causes vasoconstriction which, in turn, can increase blood pressure. Angiotensin II also stimulates secretion of the hormone aldosterone from the adrenal cortex. Aldosterone causes the kidneys to increase reabsorption of sodium and water leading to an increase of the fluid volume in a body which, in turn, can increase blood pressure. Over stimulation or activity of the RAS pathway can lead to high blood pressure. Chronic high blood pressure is known as hypertension. The high blood pressure in a hypertensive subject requires the heart to work harder to circulate blood through the blood vessels.

The World Health Organization (WHO) has identified hypertension as a leading cause of cardiovascular morbidity. Hypertension is a major risk factor for various disease, disorders and conditions such as shortened life expectancy, chronic kidney disease, stroke, myocardial infarction, heart failure, aneurysms of the blood vessels (e.g. aortic aneurysm), peripheral artery disease, heart damage (e.g., heart enlargement or hypertrophy) and other cardiovascular related diseases, disorders and/or conditions.

The prevelance of resistant hypertension (RHTN), hypertension resistant to drug treatment, has steadily increased in number likely due to an ageing population and an ever increasing incidence of obesity. The current projection of approximately 10 million RHTN adults in the United States is expected to continue to rise.

Anti-hypertensive drugs, renal denervation, baroreceptor activation therapy, diet changes and lifestyle changes may reduce hypertension and reduce the diseases, disorders and/or conditions associated with hypertension (Paulis et al., Nat Rev Cardiol, 2012, 9:276-285). However, there are limitations to the therapies currently approved for treating hypertension as a significant subset of all hypertensive patients do not achieve adequate blood pressure control. For example, drugs such as ACE inhibitors and angiotensin receptor blockers (ARBs) that target parts of the RAS pathway are limited in their ability to inhibit the RAS pathway (Nobakht et al., Nat Rev Nephrol, 2011, 7:356-359).

Accordingly, there is a need to find alternative treatments to inhibit the RAS pathway and treat hypertension, especially resistant hypertension. Antisense technology is emerging as an effective means for reducing the expression of certain gene products. Certain early antisense oligonucleotides targeting AGT have provided limited benefit (WO 1997/33623). Compositions disclosed herein are uniquely useful for therapeutic applications for the modulation of AGT.

RMA van de Wal et al. (Int J Cardiol, 2006, 3:367-372) describes determinants of increased angiotensin II levels in severe chronic heart failure patients despite ACE inhibition.

### SUMMARY OF THE INVENTION

Provided herein is a compound comprising a modified antisense oligonucleotide consisting of 12 to 30 linked nucleosides, wherein the modified antisense oligonucleotide is targeted to an angiotensinogen (AGT) nucleic acid, for use in treating an animal having a RAS pathway related disease, disorder and/or condition, wherein the RAS pathway related disease, disorder or condition is resistant hypertension.

Disclosed herein are methods, compounds, and compositions for modulating levels of AGT mRNA and/or protein in an animal. Disclosed herein are methods, compounds, and compositions for modulating levels of AGT mRNA and/or protein in an animal in order to modulate a RAS pathway related disease, disorder and/or condition in the animal. Also disclosed herein are methods, compounds, and compositions for identifying an animal having or at risk for a RAS related disease, disorder and/or condition and administering a therapeutically effective amount of a compound targeting AGT to the animal for: ameliorating the RAS related disease, disorder and/or condition in the animal; treating the animal at risk for the RAS related disease, disorder and/or condition; inhibiting AGT expression in the animal suffering from the RAS related disease, disorder and/or condition; reducing the risk of the RAS related disease, disorder and/or condition in the animal; treating RAS dependent organ damage in an animal suffering from a RAS pathway related disease. In certain embodiments, the AGT modulator or compound targeting AGT is an AGT specific inhibitor.

In certain embodiments, AGT specific inhibitors decrease levels of AGT mRNA and/or protein. In certain embodiments, AGT specific inhibitors are nucleic acids, proteins, or small molecules. In certain embodiments, the nucleic acid is an antisense oligonucleotide. In certain embodiments, the antisense oligonucleotide is a modified antisense oligonucleotide.

In certain embodiments, an animal having or at risk for a RAS related disease, disorder and/or condition is treated by selecting the animal having, or at risk for, the RAS related disease, disorder and/or condition and administering to the animal a therapeutically effective amount of a compound comprising a modified oligonucleotide consisting of 12 to 30 linked nucleosides. The modified oligonucleotide can be complementary to an AGT nucleic acid as shown in any of SEQ IDNOs: 1-16.

In certain embodiments, an animal having or at risk for a RAS related disease, disorder and/or condition is treated by selecting the animal having or at risk for the RAS related disease, disorder and/or condition and administering to the animal a therapeutically effective amount of a compound comprising a modified oligonucleotide consisting of 12 to 30 linked nucleosides and having a nucleobase sequence comprising at least 8 contiguous nucleobases complementary to a target segment or target region of SEQ ID NOs: 1-16 as described herein.

In certain embodiments, the AGT modulation occurs in a cell, tissue, organ or organism. In certain embodiments, the cell, tissue or organ is in an animal. In certain embodiments, the animal is a human. In certain embodiments, AGT mRNA levels are reduced. In certain embodiments, AGT protein levels are reduced. Such reduction can occur in a time-dependent manner or in a dose-dependent manner.

Also disclosed are methods, compounds, and compositions useful for preventing, treating, and ameliorating RAS related diseases, disorders, and conditions.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-O(CH₂)₂-OCH₃) refers to an O-methoxy-ethyl modification of the 2' position of a furosyl ring. A 2'-O-methoxyethyl modified sugar is a modified sugar.
"2'-O-methoxyethyl nucleotide" means a nucleotide comprising a 2'-O-methoxyethyl modified sugar moiety.
"5-methylcytosine" means a cytosine modified with a methyl group attached to the 5' position. A 5-methylcytosine is a modified nucleobase.
"About" means within ±10% of a value. For example, if it is stated "the compounds inhibited AGT by at least about 70%", it is implied that the AGT levels are inhibited within a range of 63% to 77%.
"ACE escape", also known as angiotensin II reactivation, refers to the inability of currently available ACE inhibitor treatment to reliably suppress plasma angiotensin II levels. The increase in plasma angiotensin II levels during ACE inhibition occurs via other enzymes converting angiotensin I to angiotensin. This incomplete blockage of angiotensin II levels prevents the ACE inhibitors from effectively treating some hypertensive subjects. Angiotensin Receptor Blockers (ARBs) may also be susceptible to ACE escape as other receptors besides the AT1 receptor engage angiotensin metabolites.
"Active pharmaceutical agent" or "pharmaceutical agent" means the substance or substances in a pharmaceutical composition that provide a therapeutic benefit when administered to an individual. For example, in certain embodiments an antisense oligonucleotide targeted to AGT is an active pharmaceutical agent.
"Active target region" or "target region" means a region to which one or more active antisense compounds is targeted.
"Active antisense compounds" means antisense compounds that reduce target nucleic acid levels or protein levels.
"Administered concomitantly" refers to the co-administration of two agents in any manner in which the pharmacological effects of both are manifest in the patient at the same time. Concomitant administration does not require that both agents be administered in a single pharmaceutical composition, in the same dosage form, or by the same route of administration. The effects of both agents need not manifest themselves at the same time. The effects need only be overlapping for a period of time and need not be co-extensive.
"Administering" means providing a pharmaceutical agent to an individual, and includes, but is not limited to administering by a medical professional and self-administering.
"Aldosterone escape" or "aldosterone breakthrough" refers to the inability of currently available ACE inhibitor Angiotensin Receptor Blocker (ARB) and/or Direct Renin Inhibitor (DRI) treatment to reliably suppress aldosterone release in some treated subjects. This incomplete blockage of aldosterone prevents the ACE inhibitors, DRIs and ARBs from effectively treating some hypertensive subjects.
"Amelioration" refers to a lessening of at least one indicator, sign, or symptom of an associated disease, disorder, or condition. In certain embodiments, amelioration includes a delay or slowing in the progression of one or more indicators of a condition or disease. The severity of indicators may be determined by subjective or objective measures, which are known to those skilled in the art. For example, amelioration of hypertension can be assessed by measuring systolic and diastolic blood pressure.
"Angiotensinogen" and "AGT" is used interchangeably herein. Angiotensinogen is also known as SERPINA8 and ANHU.
"Angiotensinogen nucleic acid" or "AGT nucleic acid" means any nucleic acid encoding AGT. For example, in certain embodiments, an AGT nucleic acid includes a DNA sequence encoding AGT, an RNA sequence transcribed from DNA encoding AGT (including genomic DNA comprising introns and exons), and an mRNA sequence encoding AGT. "AGT mRNA" means an mRNA encoding an AGT protein.
"AGT specific inhibitor" refers to any agent capable of specifically inhibiting the expression of AGT mRNA and/or AGT protein at the molecular level. For example, AGT specific inhibitors include nucleic acids (including antisense compounds such as RNasH, siRNA and blockmer antisense compounds), peptides, antibodies, small molecules, and other agents capable of specifically inhibiting the expression of AGT mRNA and/or AGT protein. In certain embodiments, by specifically modulating AGT mRNA level and/or AGT protein expression, AGT specific inhibitors can affect components of the renin-angiotensin system (RAS) pathway. In certain embodiments, by specifically modulating AGT mRNA level and/or AGT protein expression, AGT specific inhibitors can affect RAS pathway related diseases, disorders and/or conditions such as blood pressure. Similarly, in certain embodiments, AGT specific inhibitors can affect other molecular processes in an animal.
"Animal" refers to a human or non-human animal, including, but not limited to, mice, rats, rabbits, dogs, cats, pigs, and non-human primates, including, but not limited to, monkeys and chimpanzees.
"Anti-hypertensive drug" refers to a drug capable of lowering blood pressure. Examples of such drugs include, but are not limited to, RAS inhibitors, diuretics, calcium channel blockers, adrenergic receptor antagonists, adrenergic agonists and vasodilators. In one example, the anti-hypertensive drug captopril can be used in combination with the AGT compound described herein to treat an animal having or at risk of having a RAS pathway related disease, disorder and/or condition.
"Anti-hypertensive procedure" refers to a medical procedure performed on a subject to reduce hypertension. Examples of such procedures include renal denervation and baroreceptor activation therapy,
"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.
"Antisense compound" means an oligomeric compound that is capable of undergoing hybridization to a target nucleic acid through hydrogen bonding.
"Antisense inhibition" means reduction of target nucleic acid levels or target protein levels in the presence of an antisense compound complementary to a target nucleic acid compared to target nucleic acid levels or target protein levels in the absence of the antisense compound.
"Antisense oligonucleotide" means a single-stranded oligonucleotide having a nucleobase sequence that permits hybridization to a corresponding region or segment of a target nucleic acid.
"Bicyclic sugar" means a furosyl ring modified by the bridging of two non-geminal ring atoms. A bicyclic sugar is a modified sugar.
"Bicyclic nucleic acid" or "BNA" refers to a nucleoside or nucleotide wherein the furanose portion of the nucleoside or nucleotide includes a bridge connecting two carbon atoms on the furanose ring, thereby forming a bicyclic ring system.
"Blood pressure" refers to the pressure of the blood in the circulatory system against the walls of the blood vessel. The blood pressure is due mainly to the beating of the heart in an animal. During each heartbeat, the blood pressure varies between a maximum (systolic) blood pressure (SBP) and minimum (diastolic) blood pressure (DBP). The mean arterial pressure (MAP) is the average arterial pressure during a heartbeat cycle. Blood pressure can be measure by a blood pressure meter (i.e., a sphygmomanometer). Normal blood pressure at rest is within the range of 100-140mmHg systolic and 60-90mmHg diastolic and is commonly expressed as the systolic pressure (top reading) / diastolic pressure (bottom reading) mmHg.
"Chemically distinct region" refers to a region of an antisense compound that is in some way chemically different than another region of the same antisense compound. For example, a region having 2'-O-methoxyethyl nucleotides is chemically distinct from a region having nucleotides without 2'-O-methoxyethyl modifications.
"Chimeric antisense compound" means an antisense compound that has at least two chemically distinct regions.
"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses concomitant, parallel or sequential administration.
"Complementarity" means the capacity for pairing between nucleobases of a first nucleic acid and a second nucleic acid.
"Constrained ethyl" or "cEt" refers to a bicyclic nucleoside having a furanosyl sugar that comprises a methyl(methyleneoxy) (4'-CH(CH₃)-O-2') bridge between the 4' and the 2' carbon atoms.
"Contiguous nucleobases" means nucleobases immediately adjacent to each other.
"Diluent" means an ingredient in a composition that lacks pharmacological activity, but is pharmaceutically necessary or desirable. For example, the diluent in an injected composition may be a liquid, e.g. saline solution.
"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.
"Effective amount" means the amount of active pharmaceutical agent sufficient to effectuate a desired physiological outcome in an individual in need of the agent. The effective amount may vary among individuals depending on the health and physical condition of the individual to be treated, the taxonomic group of the individuals to be treated, the formulation of the composition, assessment of the individual's medical condition, and other relevant factors. In an example, an effective amount of an AGT antisense oligonucleotide decreases blood pressure and/or ameliorates organ damage due to hypertension.
"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.
"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap segment" and the external regions may be referred to as "wing segments."
"Hybridization" means the annealing of complementary nucleic acid molecules. In certain embodiments, complementary nucleic acid molecules include an antisense compound and a target nucleic acid.
"Hypertension" or "HTN" refers to a chronic medical condition where the blood pressure in an animal is elevated. The elevated blood pressure requires the heart to work harder to circulate blood through the blood vessels. High blood pressure is said to be present if it is persistently at or above 140/90 mmHg. Hypertension is classified as primary (essential) or secondary. Primary hypertension has no clear cause and is thought to be linked to genetics, diet, lack of exercise and obesity. Secondary hypertension is caused by another medical condition. Hypertension is a major risk factor for shortened life expectancy, chronic kidney disease, stroke, myocardial infarction, heart failure, aneurysms of the blood vessels (e.g. aortic aneurysm), peripheral artery disease, organ damage (e.g., heart enlargement or hypertrophy) and other cardiovascular diseases, disorders and/or conditions or symptoms thereof. Anti-hypertensive drugs, diet changes and lifestyle changes may reduce hypertension and reduce the diseases, disorders and/or conditions associated with hypertension. Hypertension can be nonresistant to drug intervention (i.e., controllable by commercially available drug therapies) or resistant to drug intervention.
"Identifying an animal having, or at risk for, a RAS related disease, disorder and/or condition" means identifying an animal having been diagnosed with a RAS related disease, disorder and/or condition or identifying an animal predisposed to develop a RAS related disease, disorder and/or condition. Individuals predisposed to develop a RAS related disease, disorder and/or condition include, for example, individuals with a familial history a RAS related disease such as hypertension. Such identification may be accomplished by any method including evaluating an individual's medical history and standard clinical tests or assessments.
"Immediately adjacent" means there are no intervening elements between the immediately adjacent elements.
"Individual" means a human or non-human animal selected for treatment or therapy.
"Internucleoside linkage" refers to the chemical bond between nucleosides.
"Linked nucleosides" means adjacent nucleosides which are bonded together.
"Modified internucleoside linkage" refers to a substitution or any change from a naturally occurring internucleoside bond (i.e. a phosphodiester internucleoside bond).
"Modified nucleobase" refers to any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).
"Modified nucleotide" means a nucleotide having, independently, a modified sugar moiety, modified internucleoside linkage and/or modified nucleobase. A "modified nucleoside" means a nucleoside having a modified sugar moiety and/or modified nucleobase.
"Modified oligonucleotide" means an oligonucleotide comprising a modified internucleoside linkage, a modified sugar and/or a modified nucleobase.
"Modified sugar" refers to a substitution or change from a natural sugar.
"Modulating" refers to changing or adjusting a feature in a cell, tissue, organ or organism. For example, modulating AGT mRNA can mean to increase or decrease the level of AGT mRNA and/or AGT protein in a cell, tissue, organ or organism. Modulating AGT mRNA and/or protein can lead to an increase or decrease in a RAS related disease, disorder and/or condition in a cell, tissue, organ or organism. A "modulator" effects the change in the cell, tissue, organ or organism. For example, an AGT antisense compound can be a modulator that increases or decreases the amount of AGT mRNA and/or AGT protein in a cell, tissue, organ or organism.
"Motif' means the pattern of chemically distinct regions in an antisense compound.
"Naturally occurring internucleoside linkage" means a 3' to 5' phosphodiester linkage.
"Natural sugar moiety" means a sugar found in DNA (2'-H) or RNA (2'-OH).
"Nonresistant hypertension", "nonrefractory hypertension" or "controlled hypertension" is defined as hypertension that responds to treatment resulting in, for example, blood pressure <140 mmHg SBP or <90 mmHg DBP with concurrent use of up to 3 anti-hypertensive agents.
"Nucleic acid" refers to molecules composed of monomeric nucleotides. A nucleic acid includes ribonucleic acids (RNA), deoxyribonucleic acids (DNA), single-stranded nucleic acids, double-stranded nucleic acids, small interfering ribonucleic acids (siRNA), and microRNAs (miRNA).
"Nucleobase" means a heterocyclic moiety capable of pairing with a base of another nucleic acid.
"Nucleobase sequence" means the order of contiguous nucleobases independent of any sugar, linkage, or nucleobase modification.
"Nucleoside" means a nucleobase linked to a sugar.
"Nucleotide" means a nucleoside having a phosphate group covalently linked to the sugar portion of the nucleoside.
"Organ damage" or "end organ damage" refers to damage occurring in major organs fed by the circulatory system such as the heart (e.g., heart muscle hypertrophy, reduced heart function and/or heart failure), kidney (e.g., albuminurea, proteinurea, reduced renal function and/or renal failure), eyes (e.g., hypertensive retinopathy), brain (e.g., stroke) and the like. The organs can be damaged by hypertension in an animal. In certain embodiments, the heart damage is fibrosis, heart cell and/or muscle hypertrophy leading to heart enlargement.
"Oligomeric compound" or "oligomer" means a polymer of linked monomeric subunits which is capable of hybridizing to at least a region of a nucleic acid molecule.
"Oligonucleotide" means a polymer of linked nucleosides each of which can be modified or unmodified, independent one from another.
"Parenteral administration" means administration through injection or infusion. Parenteral administration includes subcutaneous administration, intravenous administration, intramuscular administration, intraarterial administration, intraperitoneal administration, or intracranial administration, e.g. intrathecal or intracerebroventricular administration.
"Pharmaceutical composition" means a mixture of substances suitable for administering to an individual. For example, a pharmaceutical composition may comprise one or more active pharmaceutical agents and a sterile aqueous solution.
"Pharmaceutically acceptable salts" means physiologically and pharmaceutically acceptable salts of antisense compounds, i.e., salts that retain the desired biological activity of the parent oligonucleotide and do not impart undesired toxicological effects thereto.
"Phosphorothioate linkage" means a linkage between nucleosides where the phosphodiester bond is modified by replacing one of the non-bridging oxygen atoms with a sulfur atom. A phosphorothioate linkage is a modified internucleoside linkage.
"Portion" means a defined number of contiguous (i.e. linked) nucleobases of a nucleic acid. In certain embodiments, a portion is a defined number of contiguous nucleobases of a target nucleic acid. In certain embodiments, a portion is a defined number of contiguous nucleobases of an antisense compound.
"Prevent" refers to delaying or forestalling the onset, development or progression of a disease, disorder, or condition for a period of time from minutes to indefinitely. Prevent also means reducing the risk of developing a disease, disorder, or condition.
"Renin-angiotensin system", "Renin-angiotensin system pathway", "RAS pathway" or "RAS" refer to a multi-component enzymatic pathway where a precursor component (angiotensinogen) is converted by various enzymes such as renin and enzyme angiotensin-converting-enzyme (ACE) into downstream components such as angiotensin I and angiotensin II. Angiotensin I stimulates secretion of the steroid aldosterone in the pathway. Various components of this pathway have been targeted by agonists or antagonists to block the production of the components. For example renin inhibitors, ACE inhibitors, angiotensin-receptor blockers (ARBs) and the like have been developed to inhibit or block the RAS pathway. However, commercially available therapies targeting various RAS pathway components have been ineffective in completely inhibiting or blocking the RAS pathway due to various mechanisms (Nobakht et al., Nat Rev Nephrol, 2011, 7:356-359).
"RAS related disease, disorder and/or condition" or "RAS pathway related disease, disorder and/or condition" refers to any disease, disorder or condition related to RAS or RAAS (Renin-Angiotensin-Aldosterone System) in an animal. Examples of RAS related diseases, disorders and/or conditions include shortened life expectancy, hypertension (e.g. nonresistant hypertension, resistant hypertension), kidney disease (e.g.,chronic kidney disease, polycystic kidney disease), stroke, heart disease (e.g., myocardial infarction, heart failure, valvular heart disease), aneurysms of the blood vessels (e.g. aortic aneurysm), peripheral artery disease, organ damage (e.g., heart damage or hypertrophy), tissue fibrosis and other cardiovascular diseases, disorders and/or conditions or symptoms thereof. In certain embodiments, RAS related disease, disorder and/or condition does not include hypertension.
"Resistant hypertension" or "RHTN" is defined as (1) blood pressure >140 mmHg SBP or ≥90 mmHg DBP despite concurrent use of 3 anti-hypertensive agents from different drug classes or (2) use of ≥4 anti-hypertensive drugs regardless of blood pressure.
"Side effects" means physiological disease and/or conditions attributable to a treatment other than the desired effects. In certain embodiments, side effects include injection site reactions, liver function test abnormalities, renal function abnormalities, liver toxicity, renal toxicity, central nervous system abnormalities, myopathies, and malaise. For example, increased aminotransferase levels in serum may indicate liver toxicity or liver function abnormality. For example, increased bilirubin may indicate liver toxicity or liver function abnormality.
"Single-stranded oligonucleotide" means an oligonucleotide which is not hybridized to a complementary strand.
"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, i.e. under physiological conditions in the case of *in vivo* assays and therapeutic treatments. In an example, an antisense compound is specifically hybridizable to a target when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired, *i.e.,* under physiological conditions in the case of *in vivo* assays or therapeutic treatment, and under conditions in which assays are performed in the case of *in vitro* assays.
"Targeting" or "targeted" means the process of design and selection of an antisense compound that will specifically hybridize to a target nucleic acid and induce a desired effect.
"Target nucleic acid," "target RNA," and "target RNA transcript" all refer to a nucleic acid capable of being targeted by antisense compounds.
"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.
"Therapeutically effective amount" means an amount of a pharmaceutical agent that provides a therapeutic benefit to an individual.
"Treat" refers to administering a pharmaceutical composition to an animal in order to effect an alteration or improvement of a disease, disorder, or condition in the animal. In certain embodiments, one or more pharmaceutical compositions can be administered to the animal.
"Unmodified nucleotide" means a nucleotide composed of naturally occuring nucleobases, sugar moieties, and internucleoside linkages. In certain embodiments, an unmodified nucleotide is an RNA nucleotide (i.e. β-D-ribonucleotide) or a DNA nucleotide (i.e. β-D-deoxyribonucleotide).

### Certain Embodiments

In certain embodiments, disclosed herein are compounds specifically modulating AGT. In certain embodiments, the AGT specific modulators are AGT specific inhibitors, for use in treating, preventing, or ameliorating a RAS related disease, disorder and/or condition. In certain embodiments, AGT specific inhibitors are nucleic acids (including antisense compounds), peptides, antibodies, small molecules, and other agents capable of inhibiting the expression of AGT mRNA and/or AGT protein. In certain embodiments, the AGT specific inhibitors are antisense oligonucleotides. In certain embodiments, the antisense oligonucleotides are modified antisense oligonucleotides.

In certain embodiments, the compounds target an AGT nucleic acid. In certain embodiments, the AGT nucleic acid is any of the human sequences set forth in GENBANK Accession No. NM_000029.3 (incorporated herein as SEQ ID NO: 1), GENBANK Accession No. CR606672.1 (incorporated herein as SEQ ID NO: 2), GENBANK Accession No. AK307978.1 (incorporated herein as SEQ ID NO: 3), GENBANK Accession No. AK303755.1 (incorporated herein as SEQ ID NO: 4), GENBANK Accession No. AK293507.1 (incorporated herein as SEQ ID NO: 5), and the complement of the nucleotides 24354000 to 24370100 of GENBANK Accession No. NT_167186.1 (incorporated herein as SEQ ID NO: 6). In certain embodiments, the AGT nucleic acid is any of the mouse sequences set forth in GENBANK Accession NM_007428.3 (incorporated herein as SEQ ID NO: 7), GENBANK Accession W13136.1 (incorporated herein as SEQ ID NO: 8), GENBANK Accession AI785758.1 (incorporated herein as SEQ ID NO: 9), GENBANK Accession BG863202.1 (incorporated herein as SEQ ID NO: 10), the complement of GENBANK Accession BF020372.1 (incorporated herein as SEQ ID NO: 11), and the complement of the nucleotides 51911800 to 51928000 of GENBANK Accession No. NT_078575.6 (incorporated herein as SEQ ID NO: 12). In certain embodiments, the AGT nucleic acid is any of the rat sequences set forth in GENBANK Accession NM_134432.2 (incorporated herein as SEQ ID NO: 13), GENBANK Accession CV104009.1 (incorporated herein as SEQ ID NO: 14), GENBANK Accession BF549490.1 (incorporated herein as SEQ ID NO: 15), and the complement of the nucleotides 17172500 to17188250 of GENBANK Accession NW_047536.2 (incorporated herein as SEQ ID NO: 16).

In certain embodiments, the compounds targeting AGT described herein are for use in modulating AGT expression. In certain embodiments, the AGT modulators are AGT specific inhibitors. In certain embodiments, the AGT specific inhibitors decrease AGT expression and/or the RAS pathway. In certain embodiments, the compounds targeting AGT inhibit the RAS pathway by at least 70%, 75%, 80%, 85%, 90%, 95% or 100%.

In certain embodiments, the compounds described herein are for use in inhibiting AGT expression in an animal having, or at risk of having, a RAS pathway related disease, disorder and/or condition comprising selecting the animal suffering from the RAS pathway related disease, disorder or condition and, administering a compound targeting AGT to the animal, wherein the compound administered to the animal inhibits AGT expression in the animal having or at risk for having the RAS pathway related disease, disorder and/or condition.

In certain embodiments, the compounds described herein are for use in treating an animal having, or at risk of having, a RAS pathway related disease, disorder and/or condition comprising selecting the animal having, or at risk of having, the RAS pathway related disease, disorder or condition, and administering a therapeutically effective amount of a compound targeting AGT to the animal, wherein the compound administered to the animal treats the animal having, or at risk of having, the RAS pathway related disease, disorder and/or condition, in the animal.

In certain embodiments, the compounds described herein are for use in treating an animal having, or at risk of having, RAS dependent organ damage comprising selecting an animal suffering from a RAS pathway related disease, disorder and/or condition, and administering a therapeutically effective amount of a compound targeting AGT to the animal, wherein the compound administered to the animal treats and/or reverses the RAS dependent end organ damage in the animal with the RAS pathway related disease, disorder or condition. In certain embodiments the RAS pathway related disease, disorder or condition is hypertension. In certain embodiments the end organ damage is heart muscle hypertrophy.

In certain embodiments, the compounds described herein are for use in treating an animal having or at risk for a RAS pathway related disease comprising selecting the animal having or at risk for a RAS pathway related disease, and administering to the animal a therapeutically effective amount of a compound comprising a modified oligonucleotide consisting of 12 to 30 linked nucleosides, wherein the modified antisense oligonucleotide is complementary to an AGT nucleic acid as shown in any of SEQ ID NOs: 1-16, and wherein the compound administered to the animal treats the animal having or at risk for having the RAS pathway related disease.

In certain embodiments, the RAS pathway related disease, disorder or condition is shortened life expectancy, hypertension, kidney disease (e.g., chronic kidney disease), stroke, cardiac disease (e.g., myocardial infarction, heart failure, valvular heart disease), aneurysms of the blood vessels, peripheral artery disease, organ damage and other cardiovascular diseases, disorders and/or conditions or symptoms thereof. In certain embodiments, the hypertension is nonresistant hypertension or resistant hypertension. In certain embodiments, the aneurysm of the blood vessels is aortic aneurysm. In certain embodiments, the organ damage is heart muscle hypertrophy or fibrosis in an organ or tissue. In certain embodiments, the organ is heart, liver or kidney and the tissue is derived from the heart, liver or kidney. In certain embodiments, the RAS pathway related disease, disorder or condition is not hypertension.

In certain embodiments, the compounds described herein are for use in treating nonresistant hypertension comprising selecting the animal having or at risk for nonresistant hypertension, and administering to the animal a therapeutically effective amount of a compound comprising a modified oligonucleotide consisting of 12 to 30 linked nucleosides, wherein the modified antisense oligonucleotide is complementary to an AGT nucleic acid as shown in any of SEQ ID NOs: 1-16, and wherein the compound administered to the animal treats the animal having or at risk of having nonresistant hypertension. In certain embodiments the modified oligonucleotide is administered in combination with one or more anti-hypertensive drugs.

In certain embodiments, the compounds described herein are for use in treating resistant hypertension comprising selecting the animal having or at risk for resistant hypertension, and administering to the animal a therapeutically effective amount of a compound comprising a modified oligonucleotide consisting of 12 to 30 linked nucleosides, wherein the modified antisense oligonucleotide is complementary to an AGT nucleic acid as shown in any of SEQ ID NOs: 1-16, and wherein the compound administered to the animal treats the animal having or at risk of having resistant hypertension. In certain embodiments the modified oligonucleotide is administered in combination with one or more anti-hypertensive drugs.

In certain embodiments, disclosed are methods, compounds, and compositions for modulating a symptom or marker of a RAS pathway related disease, disorder and/or condition. In certain embodiments, the marker can be selected from one or more of shortened life expectancy, hypertension, chronic kidney disease, stroke, myocardial infarction, heart failure, valvular heart disease, aneurysms of the blood vessels, peripheral artery disease, organ damage and other cardiovascular diseases, disorders and/or conditions or symptoms thereof.

In certain embodiments, the compounds for use in the methods comprise an antisense oligonucleotide comprising a nucleobase sequence at least 80%, at least 85%, at least 90%, at least 95%, at least 96%, at least 97%, at least 98%, or at least 99% complementary to an equal length portion of SEQ ID NOs: 1-16. In certain embodiments, the compound may comprise a modified oligonucleotide comprising a nucleobase sequence 100% complementary to an equal length portion of SEQ ID NOs: 1-16.

In certain embodiments, the compounds for use in the methods comprise an antisense oligonucleotide consisting of 12 to 30 linked nucleosides and having a nucleobase sequence comprising at least 8, at least 9, at least 10, at least 11, at least 12, at least 13, at least 14, at least 15, at least 16, at least 17, at least 18, at least 19 or at least 20 contiguous nucleobases of a nucleobase sequence complementary to any of the sequences recited in SEQ ID NOs: 1-16.

In certain embodiments, the compounds for use in the methods comprise an antisense oligonucleotide consisting of 12 to 30 linked nucleosides. In certain embodiments, the modified oligonucleotide consists of 12, 13, 14, 15, 16, 17, 18, 19, 20, 21, 22, 23, 24, 25, 26, 27, 28, 29 or 30 linked nucleosides.

In certain embodiments, the compounds for use in the methods consist of a single-stranded modified oligonucleotide.

In certain embodiments, the compounds for use in the methods comprise at least one modified internucleoside linkage. In certain embodiments, the modified internucleoside linkage is a phosphorothioate internucleoside linkage. In certain embodiments, each modified internucleoside linkage is a phosphorothioate internucleoside linkage.

In certain embodiments, the compounds for use in the methods comprise at least one nucleoside comprising a modified sugar. In certain embodiments, the modified sugar is a bicyclic sugar. In certain embodiments, the modified sugar comprises a 2'-O-methoxyethyl (2'MOE).

In certain embodiments, the compounds for use in the methods comprise at least one nucleoside comprising a modified nucleobase. In certain embodiments, the modified nucleobase is a 5-methylcytosine.

In certain embodiments, the compounds for use in the methods comprise a modified antisense oligonucleotide comprising: (i) a gap segment consisting of linked deoxynucleosides; (ii) a 5' wing segment consisting of linked nucleosides; (iii) a 3' wing segment consisting of linked nucleosides, wherein the gap segment is positioned immediately adjacent to and between the 5' wing segment and the 3' wing segment and wherein each nucleoside of each wing segment comprises a modified sugar.

In certain embodiments, the compounds for use in the methods comprise a modified antisense oligonucleotide comprising: (i) a gap segment consisting of eight to sixteen linked deoxynucleosides; (ii) a 5' wing segment consisting of two to six linked nucleosides; (iii) a 3' wing segment consisting of two to six linked nucleosides, wherein the gap segment is positioned immediately adjacent to and between the 5' wing segment and the 3' wing segment, wherein each nucleoside of each wing segment comprises a 2'-O-methoxyethyl sugar; and wherein each internucleoside linkage is a phosphorothioate linkage.

In certain embodiments, the compounds for use in the methods comprise a modified antisense oligonucleotide comprising: (i) a gap segment consisting of ten linked deoxynucleosides; (ii) a 5' wing segment consisting of five linked nucleosides; (iii) a 3' wing segment consisting of five linked nucleosides, wherein the gap segment is positioned immediately adjacent to and between the 5' wing segment and the 3' wing segment, wherein each nucleoside of each wing segment comprises a 2'-O-methoxyethyl sugar; and wherein each internucleoside linkage is a phosphorothioate linkage.

In certain embodiments, the animal is a human.

In certain embodiments, the compounds described herein are administered to an animal to treat, prevent or ameliorate a RAS pathway related disease, disorder or condition. In certain embodiments, administration to an animal is by a parenteral route. In certain embodiments, the parenteral administration is any of subcutaneous or intravenous administration. In certain embodiments, the compound is administered to the animal at most once a day, at most once a week, at most once every two weeks, at most once every month, at most once a quarter, at most once every half year, at most once every year, at most once every five years or at most once every ten years.

In certain embodiments, the compound is co-administered with one or more second agent(s). The compound disclosed herein and one or more second agent can be administered concomitantly or sequentially.

In certain embodiments the second agent is an anti-hypertensive drug, a procedure to decrease hypertension, a diet change and/or a lifestyle change.

Examples of the anti-hypertensive drug include, but are not limited to, any of a RAS inhibitor, diuretic, calcium channel blocker, adrenergic receptor antagonist, adrenergic agonist and vasodilator.

In certain embodiments, the compound or oligonucleotide is in salt form.

In certain embodiments, the compounds or compositions are formulated with a pharmaceutically acceptable carrier or diluent.

In certain embodiments, disclosed is the use of a compound targeting AGT as described herein in the manufacture of a medicament. In certain embodiments, disclosed is the use of a compound targeting AGT as described herein for treating, preventing, or ameliorating a RAS pathway related disease, disorder and/or condition as described herein. In certain embodiments, the compound is an AGT specific inhibitor, for use in treating, preventing, or ameliorating a RAS related disease, disorder and/or condition. In certain embodiments, the AGT specific inhibitor is a nucleic acid (including antisense compound), peptide, antibody, small molecule, or other agent capable of inhibiting the expression of AGT mRNA and/or AGT protein. In certain embodiments, the AGT specific inhibitor is an antisense oligonucleotide. In certain embodiments, the antisense oligonucleotide is a modified antisense oligonucleotide. In certain embodiments, the AGT has a sequence as shown in any of SEQ ID NOs: 1-16.

In certain embodiments, the AGT specific inhibitor is used to reduce AGT expression. The AGT compound can be used in combination therapy with one or more additional agent or therapy as described herein. Agents or therapies can be administered concomitantly or sequentially to an animal.

In certain embodiments, disclosed is a kit for treating, preventing, or ameliorating a RAS pathway related disease and/or condition, disease, disorder or condition, wherein the kit comprises: (i) an AGT specific inhibitor as described herein; and optionally (ii) an additional agent or therapy as described herein.

A kit of the present disclosure may further include instructions for using the kit to treat, prevent, or ameliorate a RAS pathway related disease, disorder or condition as described herein.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound can be "antisense" to a target nucleic acid, meaning that is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to AGT nucleic acid is 10 to 30 nucleotides in length. In other words, antisense compounds are from 10 to 30 linked nucleobases. In other embodiments, the antisense compound comprises a modified oligonucleotide consisting of 8 to 80, 10 to 80, 12 to 50, 15 to 30, 18 to 24, 19 to 22, or 20 linked nucleobases. In certain such embodiments, the antisense compound comprises a modified oligonucleotide consisting of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked nucleobases in length, or a range defined by any two of the above values. In some embodiments, the antisense compound is an antisense oligonucleotide.

In certain embodiments, the antisense compound comprises a shortened or truncated modified oligonucleotide. The shortened or truncated modified oligonucleotide can have a single nucleoside deleted from the 5' end (5' truncation), the central portion or alternatively from the 3' end (3' truncation). A shortened or truncated oligonucleotide can have two or more nucleosides deleted from the 5' end, two or more nucleosides deleted from the central portion or alternatively can have two or more nucleosides deleted from the 3' end. Alternatively, the deleted nucleosides can be dispersed throughout the modified oligonucleotide, for example, in an antisense compound having one or more nucleoside deleted from the 5' end, one or more nucleoside deleted from the central portion and/or one or more nucleoside deleted from the 3' end.

In certain embodiments, the antisense compound comprises a lengthened or long modified oligonucleotide. When a single additional nucleoside is present in a lengthened oligonucleotide, the additional nucleoside can be located at the 5' end, 3' end or central portion of the oligonucleotide. When two or more additional nucleosides are present, the added nucleosides can be adjacent to each other, for example, in an oligonucleotide having two nucleosides added to the 5' end (5' addition), to the 3' end (3' addition) or the central portion, of the oligonucleotide. Alternatively, the added nucleoside can be dispersed throughout the antisense compound, for example, in an oligonucleotide having one or more nucleoside added to the 5' end, one or more nucleoside added to the 3' end, and/or one or more nucleoside added to the central portion.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL in vitro and in vivo. Furthermore, this oligonucleotide demonstrated potent anti-tumor activity in vivo.
Maher and Dolnick (Nuc. Acid. Res. 16:3341-3358, 1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Antisense Compound Motifs

In certain embodiments, antisense compounds targeted to an AGT nucleic acid have chemically modified subunits arranged in patterns, or motifs, to confer to the antisense compounds properties such as enhanced the inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound can optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer can in some embodiments include β-D-ribonucleosides, β-D-deoxyribonucleosides, 2'-modified nucleosides (such 2'-modified nucleosides can include 2'-MOE, and 2'-O-CH₃, among others), and bicyclic sugar modified nucleosides (such bicyclic sugar modified nucleosides can include those having a 4'-(CH2)n-O-2' bridge, where n=1 or n=2). Each distinct region comprises uniform sugar moieties or comprises different types of sugar moieties. The wing-gap-wing motif is frequently described as "X-Y-Z", where "X" represents the length of the 5' wing region, "Y" represents the length of the gap region, and "Z" represents the length of the 3' wing region. As used herein, a gapmer described as "X-Y-Z" has a configuration such that the gap segment is positioned immediately adjacent each of the 5' wing segment and the 3' wing segment. Thus, no intervening nucleotides exist between the 5' wing segment and gap segment, or the gap segment and the 3' wing segment. Any of the antisense compounds described herein can have a gapmer motif. In some embodiments, X and Z are the same, in other embodiments they are different. In some embodiments, the sugar moieties in the X wing segment are the same. In some embodiments, the types of sugar moieties in the X wing segment are the different. In some embodiments, the sugar moieties in the Z wing segment are the same. In some embodiments, the types of sugar moieties in the Z wing segment are the different.

In a preferred embodiment, Y is between 8 and 15 nucleotides. X, Y or Z can be any of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more nucleotides. Thus, gapmers include, but are not limited to, for example 5-10-5, 4-8-4, 4-12-3, 4-12-4, 3-14-3, 2-13-5, 2-16-2, 1-18-1, 3-10-3, 2-10-2, 1-10-1, 2-8-2, 6-8-6, 5-8-5, 1-8-1, 2-6-2, 6-8-6, 5-8-5, 1-8-1, 2-6-2, 2-13-2, 1-8-2, 2-8-3, 3-10-2, 1-18-2, or 2-18-2.

In certain embodiments, the antisense compound as a "wingmer" motif, having a wing-gap or gap-wing configuration, i.e. an X-Y or Y-Z configuration as described above for the gapmer configuration. Thus, wingmer configurations include, but are not limited to, for example 5-10, 8-4, 4-12, 12-4, 3-14, 16-2, 18-1, 10-3, 2-10, 1-10, 8-2, 2-13, or 5-13.

In certain embodiments, antisense compounds targeted to an AGT nucleic acid possess a 5-10-5 gapmer motif. In certain embodiments, antisense compounds targeted to an AGT nucleic acid possess a 3-14-3 gapmer motif. In certain embodiments, antisense compounds targeted to an AGT nucleic acid possess a 2-13-5 gapmer motif.

In certain embodiments, an antisense compound targeted to an AGT nucleic acid has a gap-widened motif. In certain embodiments, a gap-widened antisense oligonucleotide targeted to an AGT nucleic acid has a gap segment of fourteen 2'-deoxyribonucleosides positioned immediately adjacent to and between wing segments of three chemically modified nucleosides. In certain embodiments, the chemical modification comprises a 2'-sugar modification. In another embodiment, the chemical modification comprises a 2'-MOE sugar modification.

In certain embodiments, a gap-widened antisense oligonucleotide targeted to an AGT nucleic acid has a gap segment of thirteen 2'-deoxyribonucleosides positioned immediately adjacent to and between a 5' wing segment of two chemically modified nucleosides and a 3' wing segment of five chemically modified nucleosides. In certain embodiments, the chemical modification comprises a 2'-sugar modification. In another embodiment, the chemical modification comprises a 2'-MOE sugar modification.

### Target Nucleic Acids, Target Regions and Nucleotide Sequences

Nucleotide sequences that encode human AGT include, without limitation, the following: GENBANK Accession No. NM_000029.3 (incorporated herein as SEQ ID NO: 1), GENBANK Accession No. CR606672.1 (incorporated herein as SEQ ID NO: 2), GENBANK Accession No. AK307978.1 (incorporated herein as SEQ ID NO: 3), GENBANK Accession No. AK303755.1 (incorporated herein as SEQ ID NO: 4), GENBANK Accession No. AK293507.1 (incorporated herein as SEQ ID NO: 5), and the complement of the nucleotides 24354000 to 24370100 of GENBANK Accession No. NT_167186.1 (incorporated herein as SEQ ID NO: 6).

Nucleotide sequences that encode mouse AGT include, without limitation, the following: GENBANK Accession NM_007428.3 (incorporated herein as SEQ ID NO: 7), GENBANK Accession W13136.1 (incorporated herein as SEQ ID NO: 8), GENBANK Accession AI785758.1 (incorporated herein as SEQ ID NO: 9), GENBANK Accession BG863202.1 (incorporated herein as SEQ ID NO: 10), the complement of GENBANK Accession BF020372.1 (incorporated herein as SEQ ID NO: 11), and the complement of the nucleotides 51911800 to 51928000 of GENBANK Accession No. NT_078575.6 (incorporated herein as SEQ ID NO: 12). In certain embodiments, the AGT nucleic acid is any of the rat sequences set forth in GENBANK Accession NM_134432.2 (incorporated herein as SEQ ID NO: 13), GENBANK Accession CV104009.1 (incorporated herein as SEQ ID NO: 14), GENBANK Accession BF549490.1 (incorporated herein as SEQ ID NO: 15), and the complement of the nucleotides 17172500 to 17188250 of GENBANK Accession NW_047536.2 (incorporated herein as SEQ ID NO: 16).

It is understood that the sequence set forth in each SEQ ID NO in the examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for AGT can be obtained by accession number from sequence databases such as NCBI. In certain embodiments, a target region may encompass the sequence from a 5' target site of one target segment within the target region to a 3' target site of another target segment within the target region.

In certain embodiments, a "target segment" is a smaller, sub-portion of a target region within a nucleic acid. For example, a target segment can be the sequence of nucleotides of a target nucleic acid to which one or more antisense compound is targeted. "5' target site" refers to the 5'-most nucleotide of a target segment. "3' target site" refers to the 3'-most nucleotide of a target segment.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain embodiments, reductions in AGT mRNA levels are indicative of inhibition of AGT expression. Reductions in levels of an AGT protein are also indicative of inhibition of target mRNA levels. Further, phenotypic changes are indicative of inhibition of AGT expression. For example, a decrease in hypertension can be indicative of inhibition of AGT expression. In another example, a decrease in heart size can be indicative of inhibition of AGT expression. In another example, a decrease in angiotensin level can be indicative of inhibition of AGT expression.

### Hybridization

In some embodiments, hybridization occurs between an antisense compound disclosed herein and an AGT nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art (Sambrook and Russell, Molecular Cloning: A Laboratory Manual, 3rd Ed., 2001). In certain embodiments, the antisense compounds disclosed herein are specifically hybridizable with an AGT nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as an AGT nucleic acid).

Non-complementary nucleobases between an antisense compound and an AGT nucleic acid may be tolerated provided that the antisense compound remains able to specifically hybridize to a target nucleic acid. Moreover, an antisense compound may hybridize over one or more segments of an AGT nucleic acid such that intervening or adjacent segments are not involved in the hybridization event (e.g., a loop structure, mismatch or hairpin structure).

In certain embodiments, the antisense compounds disclosed herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to an AGT nucleic acid, a target region, target segment, or specified portion thereof. Percent complementarity of an antisense compound with a target nucleic acid can be determined using routine methods. For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present disclosure. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds disclosed herein, or specified portions thereof, are fully complementary (i.e. 100% complementary) to a target nucleic acid, or specified portion thereof. For example, antisense compound may be fully complementary to an AGT nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e. linked) or non-contiguous. In one embodiment, a non-complementary nucleobase is located in the wing segment of a gapmer antisense oligonucleotide.

In certain embodiments, antisense compounds that are, or are up to 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as an AGT nucleic acid, or specified portion thereof.

In certain embodiments, antisense compounds that are, or are up to 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, such as an AGT nucleic acid, or specified portion thereof.

The antisense compounds disclosed herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 12 nucleobase portion of a target segment. In certain embodiments, the antisense compounds are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds disclosed herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds disclosed herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain embodiments, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to an AGT nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (*R* or *S*), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds disclosed herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' (also referred to as constrained ethyl or cEt) and 4'-CH(CH₂OCH₃)-O-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from -[C(Rₐ)(R_{b})]ₙ-, -C(Rₐ₎=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each R is, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNAs have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-CH₂-O-2') BNA, (B) β-D-methyleneoxy (4'-CH₂-O-2') BNA, (C) ethyleneoxy (4'-(CH₂)₂-O-2') BNA, (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, (J) propylene carbocyclic (4'-(CH₂)₃-2') BNA and (K) vinyl BNA as depicted below. 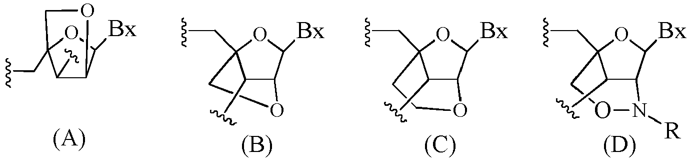 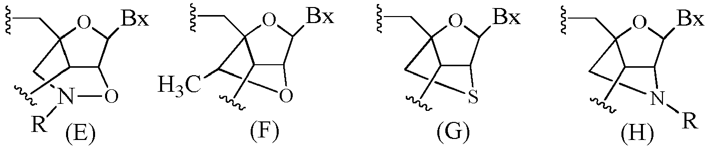 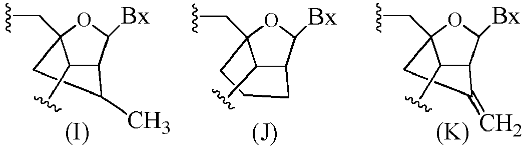 wherein Bx is the base moiety and R is independently H, a protecting group, C₁-C₁₂ alkyl or C₁-C₁₂ alkoxy.

In certain embodiments, bicyclic nucleosides are disclosed having Formula I: 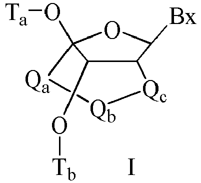 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are disclosed having Formula II: 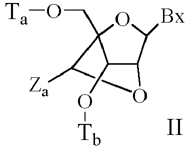 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are disclosed having Formula III: 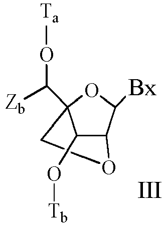 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are disclosed having Formula IV: 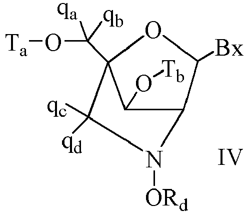 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;
In certain embodiments, bicyclic nucleosides are disclosed having Formula V: 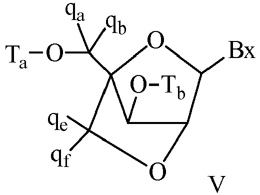 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are disclosed having Formula VI: 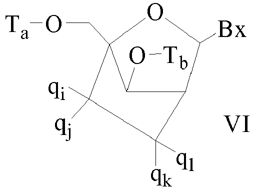 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007, 129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)_{b}CH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O-*methyl, *O*-propyl, and *O*-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854 or fluoro HNA (F-HNA) having a tetrahydropyran ring system as illustrated below:

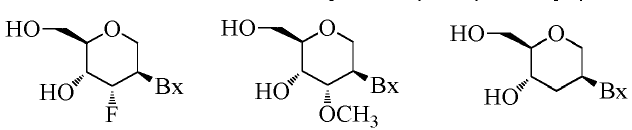

In certain embodiments, sugar surrogates are selected having Formula VII: 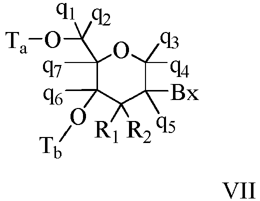 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are disclosed wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are disclosed wherein one of R₁ and R₂ is fluoro. In certain embodiments, R₁ is fluoro and R₂ is H; R₁ is methoxy and R₂ is H, and R₁ is methoxyethoxy and R₂ is H.

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following formula: 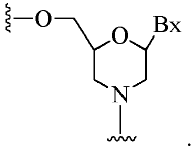 In certain embodiments, morpholinos may be modified, for example by adding or altering various substituent groups from the above morpholino structure. Such sugar surrogates are referred to herein as "modifed morpholinos."

Combinations of modifications are also disclosed without limitation, such as 2'-F-5'-methyl substituted nucleosides (see PCT International Application WO 2008/101157 published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

In certain embodiments, antisense compounds comprise one or more modified cyclohexenyl nucleosides, which is a nucleoside having a six-membered cyclohexenyl in place of the pentofuranosyl residue in naturally occurring nucleosides. Modified cyclohexenyl nucleosides include, but are not limited to those described in the art (see for example commonly owned, published PCT Application WO 2010/036696, published on April 10, 2010, Robeyns et al., J. Am. Chem. Soc., 2008,130(6), 1979-1984; Horváth et al., Tetrahedron Letters, 2007, 48, 3621-3623; Nauwelaerts et al., J. Am. Chem. Soc., 2007, 129(30), 9340-9348; Gu et al.,, Nucleosides, Nucleotides & Nucleic Acids, 2005, 24(5-7), 993-998; Nauwelaerts et al., Nucleic Acids Research, 2005, 33(8), 2452-2463; Robeyns et al., Acta Crystallographica, Section F: Structural Biology and Crystallization Communications, 2005, F61(6), 585-586; Gu et al., Tetrahedron, 2004, 60(9), 2111-2123; Gu et al., Oligonucleotides, 2003, 13(6), 479-489; Wang et al., J. Org. Chem., 2003, 68, 4499-4505; Verbeure et al., Nucleic Acids Research, 2001, 29(24), 4941-4947; Wang et al., J. Org. Chem., 2001, 66, 8478-82; Wang et al., Nucleosides, Nucleotides & Nucleic Acids, 2001, 20(4-7), 785-788; Wang et al., J. Am. Chem., 2000, 122, 8595-8602; Published PCT application, WO 06/047842; and Published PCT Application WO 01/049687). Certain modified cyclohexenyl nucleosides have Formula X. 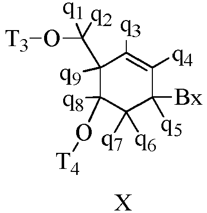 wherein independently for each of said at least one cyclohexenyl nucleoside analog of Formula X:
Bx is a heterocyclic base moiety;
T₃ and T₄ are each, independently, an internucleoside linking group linking the cyclohexenyl nucleoside analog to an antisense compound or one of T₃ and T₄ is an internucleoside linking group linking the tetrahydropyran nucleoside analog to an antisense compound and the other of T₃ and T₄ is H, a hydroxyl protecting group, a linked conjugate group, or a 5'-or 3'-terminal group; and
q₁, q₂, q₃, q₄, q₅, q₆, q₇, q₈ and q₉ are each, independently, H, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or other sugar substituent group.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position of the sugar ring.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854). Such ring systems can undergo various additional substitutions to enhance activity.

Methods for the preparations of modified sugars are well known to those skilled in the art. Some representative U.S. patents that teach the preparation of such modified sugars include without limitation, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032 and International Application PCT/US2005/019219, filed June 2, 2005 and published as WO 2005/121371 on December 22, 2005.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Modified Nucleobases

Nucleobase (or base) modifications or substitutions are structurally distinguishable from, yet functionally interchangeable with, naturally occurring or synthetic unmodified nucleobases. Both natural and modified nucleobases are capable of participating in hydrogen bonding. Such nucleobase modifications may impart nuclease stability, binding affinity or some other beneficial biological property to antisense compounds. Modified nucleobases include synthetic and natural nucleobases such as, for example, 5-methylcytosine (5-me-C). Certain nucleobase substitutions, including 5-methylcytosine substitutions, are particularly useful for increasing the binding affinity of an antisense compound for a target nucleic acid. For example, 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278).

Additional modified nucleobases include 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine.

Heterocyclic base moieties may also include those in which the purine or pyrimidine base is replaced with other heterocycles, for example 7-deaza-adenine, 7-deazaguanosine, 2-aminopyridine and 2-pyridone. Nucleobases that are particularly useful for increasing the binding affinity of antisense compounds include 5-substituted pyrimidines, 6-azapyrimidines and N-2, N-6 and O-6 substituted purines, including 2 aminopropyladenine, 5-propynyluracil and 5-propynylcytosine.

In certain embodiments, antisense compounds targeted to an AGT nucleic acid comprise one or more modified nucleobases. In certain embodiments, gap-widened antisense oligonucleotides targeted to an AGT nucleic acid comprise one or more modified nucleobases. In certain embodiments, the modified nucleobase is 5-methylcytosine. In certain embodiments, each cytosine is a 5-methylcytosine.

### Compositions and Methods for Formulating Pharmaceutical Compositions

Antisense oligonucleotides can be admixed with pharmaceutically acceptable active or inert substance for the preparation of pharmaceutical compositions or formulations. Compositions and methods for the formulation of pharmaceutical compositions are dependent upon a number of criteria, including, but not limited to, route of administration, extent of disease, or dose to be administered.

An antisense compound targeted to an AGT nucleic acid can be utilized in pharmaceutical compositions by combining the antisense compound with a suitable pharmaceutically acceptable diluent or carrier.

In certain embodiments, the "pharmaceutical carrier" or "excipient" is a pharmaceutically acceptable solvent, suspending agent or any other pharmacologically inert vehicle for delivering one or more nucleic acids to an animal. The excipient can be liquid or solid and can be selected, with the planned manner of administration in mind, so as to provide for the desired bulk, consistency, etc., when combined with a nucleic acid and the other components of a given pharmaceutical composition. Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, sodium starch glycolate, etc.); and wetting agents (e.g., sodium lauryl sulphate, etc.).

Pharmaceutically acceptable organic or inorganic excipients, which do not deleteriously react with nucleic acids, suitable for parenteral or non-parenteral administration can also be used to formulate the compositions. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to an AGT nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer (e.g., PBS). In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers.

### Dosing

In certain embodiments, pharmaceutical compositions are administered according to a dosing regimen (e.g., dose, dose frequency, and duration) wherein the dosing regimen can be selected to achieve a desired effect. The desired effect can be, for example, reduction of AGT or the prevention, reduction, amelioration or slowing the progression of a disease, disorder or condition associated with AGT or the RAS pathway.

In certain embodiments, the variables of the dosing regimen are adjusted to result in a desired concentration of pharmaceutical composition in a subject. "Concentration of pharmaceutical composition" as used with regard to dose regimen can refer to the compound, oligonucleotide, or active ingredient of the pharmaceutical composition. For example, in certain embodiments, dose and dose frequency are adjusted to provide a tissue concentration or plasma concentration of a pharmaceutical composition at an amount sufficient to achieve a desired effect.

Dosing is dependent on severity and responsiveness of the disease state to be treated, with the course of treatment lasting from several days to several months, or until a cure is effected or a diminution of the disease state is achieved. Dosing is also dependent on drug potency and metabolism. In certain embodiments, dosage is from 0.01 µg to 100mg per kg of body weight, or within a range of 0.001mg to 1000mg dosing, and may be given once or more daily, weekly, monthly, quarterly or yearly, or even once every 2 to 20 years. Following successful treatment, it may be desirable to have the patient undergo maintenance therapy to prevent the recurrence of the disease state, wherein the oligonucleotide is administered in maintenance doses, ranging from 0.01 µg to 100mg per kg of body weight, once or more daily, once or more weekly, once or more monthly, once or more quarterly, once or more yearly, to once every 20 years or ranging from 0.001mg to 1000mg dosing. In certain embodiments, it may be desirable to administer the oligonucleotide from at most once daily, once weekly, once monthly, once quarterly, once yearly, once every two years, once every three years, once every four years, once every five years, once every ten year, to once every 20 years.

### Administration

The compounds or pharmaceutical compositions can be administered in a number of ways depending upon whether local or systemic treatment is desired and upon the area to be treated. Administration can be oral, inhaled or parenteral.

In certain embodiments, the compounds and compositions as described herein are administered parenterally. Parenteral administration includes intravenous, intra-arterial, subcutaneous, intraperitoneal or intramuscular injection or infusion; or intracranial, e.g., intrathecal or intraventricular, administration.

In certain embodiments, parenteral administration is by infusion. Infusion can be chronic or continuous or short or intermittent. In certain embodiments, infused pharmaceutical agents are delivered with a pump.

In certain embodiments, parenteral administration is by injection. The injection can be delivered with a syringe or a pump. In certain embodiments, the injection is a bolus injection. In certain embodiments, the injection is administered directly to a tissue or organ.

In certain embodiments, formulations for parenteral, intrathecal or intraventricular administration can include sterile aqueous solutions which can also contain buffers, diluents and other suitable additives such as, but not limited to, penetration enhancers, carrier compounds and other pharmaceutically acceptable carriers or excipients.

In certain embodiments, formulations for oral administration of the compounds or compositions can include, but is not limited to, pharmaceutical carriers, excipients, powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders can be desirable. In certain embodiments, oral formulations are those in which compounds disclosed herein are administered in conjunction with one or more penetration enhancers, surfactants and chelators.

### Conjugated Antisense Compounds

In certain embodiments, the compounds disclosed herein can be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

In certain embodiments, antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of AGT nucleic acids can be tested *in vitro* in a variety of cell types. Cell types used for such analyses are available from commerical vendors (e.g. American Type Culture Collection, Manassus, VA; Zen-Bio, Inc., Research Triangle Park, NC; Clonetics Corporation, Walkersville, MD) and cells are cultured according to the vendor's instructions using commercially available reagents (e.g. Invitrogen Life Technologies, Carlsbad, CA). Illustrative cell types include, but are not limited to, HepG2 cells, Hep3B cells, and primary hepatocytes.

### In vitro testing of antisense oligonucleotides

Described herein are methods for treatment of cells with antisense oligonucleotides, which can be modified appropriately for treatment with other antisense compounds.

In general, cells are treated with antisense oligonucleotides when the cells reach approximately 60-80% confluency in culture.

One reagent commonly used to introduce antisense oligonucleotides into cultured cells includes the cationic lipid transfection reagent LIPOFECTIN® (Invitrogen, Carlsbad, CA). Antisense oligonucleotides are mixed with LIPOFECTIN® in OPTI-MEM® 1 (Invitrogen, Carlsbad, CA) to achieve the desired final concentration of antisense oligonucleotide and a LIPOFECTIN® concentration that typically ranges 2 to 12 ug/mL per 100 nM antisense oligonucleotide.

Another reagent used to introduce antisense oligonucleotides into cultured cells includes LIPOFECTAMINE® (Invitrogen, Carlsbad, CA). Antisense oligonucleotide is mixed with LIPOFECTAMINE® in OPTI-MEM® 1 reduced serum medium (Invitrogen, Carlsbad, CA) to achieve the desired concentration of antisense oligonucleotide and a LIPOFECTAMINE® concentration that typically ranges 2 to 12 ug/mL per 100 nM antisense oligonucleotide.

Another reagent used to introduce antisense oligonucleotides into cultured cells includes Cytofectin® (Invitrogen, Carlsbad, CA). Antisense oligonucleotide is mixed with Cytofectin® in OPTI-MEM® 1 reduced serum medium (Invitrogen, Carlsbad, CA) to achieve the desired concentration of antisense oligonucleotide and a Cytofectin® concentration that typically ranges 2 to 12 ug/mL per 100 nM antisense oligonucleotide.

Another reagent used to introduce antisense oligonucleotides into cultured cells includes Oligofectamine™ (Invitrogen Life Technologies, Carlsbad, CA). Antisense oligonucleotide is mixed with Oligofectamine™ in Opti-MEM™-1 reduced serum medium (Invitrogen Life Technologies, Carlsbad, CA) to achieve the desired concentration of oligonucleotide with an Oligofectamine™ to oligonucleotide ratio of approximately 0.2 to 0.8 µL per 100 nM.

Another reagent used to introduce antisense oligonucleotides into cultured cells includes FuGENE 6 (Roche Diagnostics Corp., Indianapolis, IN). Antisense oligomeric compound was mixed with FuGENE 6 in 1 mL of serum-free RPMI to achieve the desired concentration of oligonucleotide with a FuGENE 6 to oligomeric compound ratio of 1 to 4 µL of FuGENE 6 per 100 nM.

Another technique used to introduce antisense oligonucleotides into cultured cells includes electroporation (Sambrook and Russell in Molecular Cloning. A Laboratory Manual. Third Edition. Cold Spring Harbor laboratory Press, Cold Spring Harbor, New York. 2001).

Cells are treated with antisense oligonucleotides by routine methods. Cells are typically harvested 16-24 hours after antisense oligonucleotide treatment, at which time RNA or protein levels of target nucleic acids are measured by methods known in the art and described herein (Sambrook and Russell in Molecular Cloning. A Laboratory Manual. Third Edition. Cold Spring Harbor laboratory Press, Cold Spring Harbor, New York. 2001). In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art (Sambrook and Russell in Molecular Cloning. A Laboratory Manual. Third Edition. Cold Spring Harbor laboratory Press, Cold Spring Harbor, New York. 2001). Antisense oligonucleotides are typically used at concentrations ranging from 1 nM to 300 nM when transfected with LIPOFECTAMINE2000®, Lipofectin or Cytofectin. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art (Sambrook and Russell, Molecular Cloning: A Laboratory Manual, 3rd Ed., 2001). RNA is prepared using methods well known in the art, for example, using the TRIZOL® Reagent (Invitrogen, Carlsbad, CA) according to the manufacturer's recommended protocols.

### Analysis of inhibition of target levels or expression

Inhibition of levels or expression of an AGT nucleic acid can be assayed in a variety of ways known in the art (Sambrook and Russell, Molecular Cloning: A Laboratory Manual, 3rd Ed., 2001). For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM® 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM® 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents are obtained from Invitrogen (Carlsbad, CA). RT, real-time-PCR reactions are carried out by methods well known to those skilled in the art.

Gene (or RNA) target quantities obtained by real time PCR are normalized using either the expression level of a gene whose expression is constant, such as cyclophilin A or GAPDH, or by quantifying total RNA using RIBOGREEN® (Invitrogen, Inc. Carlsbad, CA). Cyclophilin A or GAPDH expression is quantified by real time PCR, by being run simultaneously with the target, multiplexing, or separately. Total RNA is quantified using RIBOGREEN® RNA quantification reagent (Invitrogen, Carlsbad, CA). Methods of RNA quantification by RIBOGREEN® are taught in Jones, L.J., et al, (Analytical Biochemistry, 1998, 265, 368-374). A CYTOFLUOR® 4000 instrument (PE Applied Biosystems) is used to measure RIBOGREEN® fluorescence.

Probes and primers are designed to hybridize to an AGT nucleic acid. Methods for designing real-time PCR probes and primers are well known in the art, and may include the use of software such as PRIMER EXPRESS® Software (Applied Biosystems, Foster City, CA).

The PCR probes can have JOE or FAM covalently linked to the 5' end and TAMRA or MGB covalently linked to the 3' end, where JOE or FAM is the fluorescent reporter dye and TAMRA or MGB is the quencher dye. In some cell types, primers and probe designed to a sequence from a different species are used to measure expression. For example, a human GAPDH primer and probe set can be used to measure GAPDH expression in monkey-derived cells and cell lines.

Gene target quantities obtained by RT, real-time PCR can be normalized using either the expression level of GAPDH, a gene whose expression is constant, or by quantifying total RNA using RiboGreen™ (Molecular Probes, Inc. Eugene, OR). GAPDH expression can be quantified by RT, real-time PCR, by being run simultaneously with the target, multiplexing, or separately. Total RNA can be quantified using RiboGreen™ RNA quantification reagent (Molecular Probes, Inc. Eugene, OR).

### Analysis of Protein Levels

Antisense inhibition of AGT nucleic acids can be assessed by measuring AGT protein levels. Protein levels of AGT can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS) (Sambrook and Russell, Molecular Cloning: A Laboratory Manual, 3rd Ed., 2001). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. Antibodies useful for the detection of human and rat AGT are commercially available.

### In Vivo Testing of Antisense Compounds

Antisense compounds, for example, antisense oligonucleotides, are tested in animals to assess their ability to inhibit expression of AGT and/or the RAS pathway and produce phenotypic changes such as a decrease in one or more RAS pathway related diseases. Testing may be performed in normal animals, or in experimental disease models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency depends upon factors such as route of administration and animal body weight. In one embodiment, following a period of treatment with antisense oligonucleotides, RNA is isolated from liver tissue and changes in AGT nucleic acid expression are measured. Changes in AGT protein levels can also be measured. Changes in AGT expression can also be measured by determing the level of inhibiton of the RAS pathway. RAS pathway related diseases, disorders and/or conditions may be used as markers for determining the level of AGT inhibition.

### Certain Indications

In certain embodiments, disclosed herein are methods of treating an individual comprising administering one or more pharmaceutical compositions as disclosed herein. In certain embodiments, the individual has, or is at risk for, a RAS pathway related disease, disorder or condition. In certain embodiments, disclosed herein are methods for prophylactically reducing AGT expression in an individual. Certain embodiments include treating an individual in need thereof by administering to an individual a therapeutically effective amount of an antisense compound targeted to an AGT nucleic acid.

In certain embodiments, administration of a therapeutically effective amount of an antisense compound targeted to an AGT nucleic acid is accompanied by monitoring of AGT levels in the serum or tissue of an individual, to determine an individual's response to administration of the antisense compound. An individual's response to administration of the antisense compound is used by a physician to determine the amount and duration of therapeutic intervention.

In certain embodiments, administration of an antisense compound targeted to an AGT nucleic acid results in reduction of AGT expression by at least 15, 20, 25, 30, 35, 40, 45, 50, 55, 60, 65, 70, 75, 80, 85, 90, 95, 99% or 100% or a range defined by any two of these values. In certain embodiments, administration of an antisense compound targeted to an AGT nucleic acid results in inhibiton of the RAS pathway by at least 15, 20, 25, 30, 35, 40, 45, 50, 55, 60, 65, 70, 75, 80, 85, 90, 95, 99% or 100% or a range defined by any two of these values. In certain embodiments, administration of an antisense compound targeted to an AGT nucleic acid results in a change the RAS pathway related disease, disorder, condition, symptom or marker (e.g., hypertension or organ damage). In certain embodiments, administration of an AGT antisense compound increases or decreases the RAS related disease, disorder, condition, symptom or marker by at least 15, 20, 25, 30, 35, 40, 45, 50, 55, 60, 65, 70, 75, 80, 85, 90, 95, 99% or 100% or a range defined by any two of these values.

In certain embodiments, pharmaceutical compositions comprising an antisense compound targeted to AGT are used for the preparation of a medicament for treating a patient suffering or susceptible to a RAS related disease, disorder or condition.

In certain embodiments, the methods described herein include administering a compound comprising a modified oligonucleotide having an 8, 9, 10, 11, 12, 13, 14, 15, 16, 17, 18, 19 or 20 contiguous nucleobase portion complementary to AGT.

### Certain Combination Therapies

In certain embodiments, a first agent comprising an antisense compound disclosed herein is co-administered with one or more secondary agents. In certain embodiments, the antisense compound is an antisense oligonucleotide. In certain embodiments, the antisense oligonucleotide is a modified oligonucleotide.

In certain embodiments, such second agents are designed to treat the same RAS pathway related disease, disorder or condition as the first agent described herein. In certain embodiments, such second agents are designed to treat a different disease, disorder, or condition as the first agent described herein. In certain embodiments, such second agents are designed to treat an undesired side effect of one or more pharmaceutical compositions as described herein. In certain embodiments, such first agents are designed to treat an undesired side effect of a second agent. In certain embodiments, second agents are co-administered with the first agent to treat an undesired effect of the first agent. In certain embodiments, second agents are co-administered with the first agent to produce a combinational or additive effect. In certain embodiments, second agents are co-administered with the first agent to produce a synergistic effect.

In certain embodiments, the co-administration of the first and second agents permits use of lower dosages than would be required to achieve a therapeutic or prophylactic effect if the agents were administered as independent therapy. In certain embodiments the dose of a co-administered second agent is the same as the dose that would be administered if the second agent was administered alone. In certain embodiments the dose of a co-administered second agent is greater than the dose that would be administered if the second agent was administered alone.

In certain embodiments, a first agent and one or more second agents are administered at the same time. In certain embodiments, the first agent and one or more second agents are administered at different times. In certain embodiments, the first agent and one or more second agents are prepared together in a single pharmaceutical formulation. In certain embodiments, the first agent and one or more second agents are prepared separately.

In certain embodiments, second agents include, but are not limited to, certain procedures to reduce hypertension, diet changes, lifestyle changes, anti-fibrotic drugs and anti-hypertensive drugs such as RAS inhibitors, diuretics, calcium channel blockers, adrenergic receptor antagonists, adrenergic agonists and vasodilators.

Examples of procedures that can reduce hypertension include, but are not limited to, renal denervation and baroreceptor activation therapy.

Examples of RAS inhibitors include, but are not limited to ACE inhibitors (e.g., captopril, enalapril, fosinopril, lisinopril, perindopril, quinapril, ramipril, trandolapril and benazepril), angiotensin II receptor antagonists (e.g., candesartan, eprosartan, irbesartan, losartan, olmesartan, telmisartan and valsartan), renin inhibitors (e.g., aliskiren), aldosterone receptor antagonists (e.g., eplerenone and spironolactone).

Examples of diuretics include loop diuretics (e.g., bumetanide, ethacrynic acid, furosemide, torsemide), thiazide diuretics (e.g., epitizide, hydrochlorothiazide, chlorothiazide and bendroflumethiazide), thiazide-like diuretics (e.g., indapamide, chlorthalidone and metolazone) and potassium-sparing diuretics (e.g., amiloride, triamterene and spironolactone).

Examples of calcium channel blockers include dihydropyridines (e.g., amlodipine, felodipine, isradipine, lercanidipine, nicardipine, nifedipine, nimodipine and nitrendipine) and non-dihydropyridines (e.g., diltiazem and verapamil).

Examples of adrenergic receptor antagonists include Beta blockers (e.g., atenolol, metoprolol, nadolol, oxprenolol, pindolol, propranolol and timolol), Alpha blockers (e.g., doxazosin, phentolamine, indoramin, phenoxybenzamine, prazosin, terazosin and tolazoline) and mixed Alpha + Beta blockers (e.g., bucindolol, carvedilol and labetalol).

Examples of vasodilators include sodium nitroprusside and hydralazine and its derivatives.

Examples of adrenergic agonists include alpha-2 agonists (e.g., clonidine, guanabenz, methyldopa and moxonidine).

Additional examples of anti-hypertensive drugs include guanethidine, reserpine and the like.

The second agents can be used in combination with the therapeutic compounds described herein to decrease a RAS pathway related disease, disorder and/or condition such as hypertension, organ damage and the like.

### ADVANTAGES OF THE INVENTION

Disclosed herein are methods and compositions for the modulation of AGT that can treat, prevent and/or ameliorate a RAS pathway related disease, disorder and/or condition such as hypertension or organ damage. In a particular embodiment, provided are AGT antisense oligonucleotides (antisense oligonucleotides targeting a nucleic acid encoding AGT protein) to treat, prevent and/or ameliorate a RAS pathway related disease, disorder and/or condition or symptoms thereof as described herein.

Currently, commercially available therapies targeting various RAS pathway components have been ineffective in completely inhibiting or blocking the RAS pathway. The mechanism(s) for this ineffective inhibition has not been fully elucidated, but may occur by ACE escape and/or aldosterone escape pathways. The antisense oligonucleotide targeting AGT described herein has been shown to completely block the RAS pathway. Accordingly, AGT inhibition is not susceptible to the ACE escape or aldosterone escape mechanisms confounding the commercially approved therapies for hypertension. Therefore, an AGT antisense oligonucleotide may provide superior therapeutic efficacy over the current commercial therapeutics used to treat, prevent and/or ameliorate hypertension in a subject, especially those subject suffering from resistant hypertension. An extended duration of action and more complete inhibition of tissue RAS activity also contribute to the superority of an AGT antisense oligonucleotide relative to current commercial therapeutics. Also, the AGT antisense oligonucleotide can be used in combination with commercially available anti-hypertensive medications (e.g., ACE inhibitors and/or ARBs) to provide an additive therapeutic effect.

Additionally, the antisense oligonucleotide targeting AGT has been shown to localize to and decrease AGT in the liver, heart and kidney. An ability to decrease AGT in liver tissue indicates that the oligonucleotide can target and inhibit the main source of AGT production. An ability to decrease AGT in cardiac tissue indicates that the oligonucleotide can be useful in treating cardiac diseases such as myocardial infarction, heart failure, valvular heart disease, heart tissue hypertrophy and the like. An ability to decrease AGT in kidney tissue indicates that the oligonucleotide can be useful in treating kidney related diseases such as hypertension, chronic kidney disease and the like. Reducing AGT, and therefore all angiotensin fragments, in extrahepatic tissues is predicted to be the most efficacious means of limiting pathogenic tissue RAS activity.

Another advantage of the invention is that the antisense oligonucleotide targeting AGT has been shown to reverse cardiac hypertrophy within two weeks of antisense oligonucleotide administration. Hence, an AGT antisense oligonucleotide may be able to reverse organ damage and/or fibrosis caused by hypertension in a subject.

### EXAMPLES

### Non-limiting disclosure

While certain compounds, compositions and methods described herein have been described with specificity in accordance with certain embodiments, the following examples serve only to illustrate the compounds described herein and are not intended to limit the same.

### Example 1: In vivo antisense inhibition of murine angiotensinogen

Sprague-Dawley rats are a multipurpose model used for safety and efficacy evaluations. The effect of antisense inhibition of angiotensinogen with ISIS 552668 was studied in this model.

ISIS 552668 (CACTGATTTTTGCCCAGGAT; SEQ ID NO: 17), which was one of the antisense oligonucleotides tested in the assay, was designed as a 5-10-5 MOE gapmer, and is 20 nucleosides in length, wherein the central gap segment is comprised often 2'-deoxynucleosides and is flanked on both sides (in the 5' and 3' directions) by wings comprising 5 nucleosides each. Each nucleoside in the 5' wing segment and each nucleoside in the 3' wing segment has a 2'-MOE modification. The internucleoside linkages throughout the gapmer are phosphorothioate (P=S) linkages. All cytosine residues throughout the gapmer are 5-methylcytosines. ISIS 552668 is targeted to nucleobases 1679 to 1698 of rat angiotensinogen (GENBANK Accession No. NM_134432.2, incorporated herein as SEQ ID NO: 13).

### Treatment

Groups of Sprague-Dawley rats were injected with 10 mg/kg/week, 20 mg/kg/week, 40 mg/kg/week, or 80 mg/kg/week of ISIS 552668 administered for 4 weeks. A control group of rats was injected with phosphate buffered saline (PBS) administered weekly for 4 weeks. The rats were sacrificed at the end of the study at week 4. Whole liver and kidney were harvested for RNA analysis and plasma was collected for protein analysis.

### Angiotensinogen RNA Analysis

RNA was extracted from liver tissue for real-time PCR analysis of angiotensinogen, using primer probe set RTS3550 (forward sequence AGCACGACTTCCTGACTTGGA, designated herein as SEQ ID NO: 18; reverse sequence TTGTAGGATCCCCGAATTTCC, designated herein as SEQ ID NO: 19; probe sequence AACCCGCCTCCTCGGGCCAT, designated herein as SEQ ID NO: 20). The mRNA levels were normalized using the housekeeping gene, GAPDH. As shown in Table 1, the antisense oligonucleotides achieved significant reduction of angiotensinogen over the PBS control. Results are presented as percent inhibition of angiotensinogen, relative to control. The ED₅₀ for liver and kidney was calculated to be 17.0 and 9.5 respectively.

**Table 1**

| Percent inhibition of angiotensinogen mRNA in Sprague-Dawley rats | | |
|---|---|---|
| Dose (mg/kg/wk) | Liver | Kidney |
| 10 | 19 | 41 |
| 20 | 59 | 68 |
| 40 | 86 | 75 |
| 80 | 96 | 77 |

### Protein Analysis

Angiotensinogen plasma levels were measured by ELISA (IBL International, Toronto, Canada). As shown in Table 2, antisense inhibition of angiotensinogen by ISIS 552668 resulted in a significant dose-dependent reduction of angiotensinogen protein. Results are presented as percent inhibition of angiotensinogen, relative to PBS control. The ED₅₀ for plasma protein reductions was 23.6 mg/kg.

**Table 2**

| Percent inhibition of angiotensinogen protein in Sprague-Dawley rats | |
|---|---|
| Dose (mg/kg/wk) | % inhibition |
| 10 | 25 |
| 20 | 38 |
| 40 | 83 |
| 80 | 93 |

### Effect on blood pressure

Systolic blood pressure (SBP), mean arterial pressure (MAP), and diastolic blood pressure (DBP) in the rats was measured by the tail-cuff volume-pressure method (Kent Scientific, Torrington, CT). The results are presented in Table 3 and indicate that antisense inhibition of angiotensinogen resulted in dose-dependent reductions in all three parameters in the rats

**Table 3**

| Blood pressure parameters (mm Hg) in Sprague-Dawley rats after 4 wks of treatments | | | |
|---|---|---|---|
| Dose (mg/kg/wk) | SBP | MAP | DBP |
| PBS | 149 | 125 | 113 |
| 10 | 138 | 117 | 107 |
| 40 | 118 | 92 | 79 |
| 80 | 109 | 82 | 68 |

### Example 2: In vivo antisense inhibition of angiotensinogen in female SHR rats

Spontaneously hypertensive (SHR) rats are a model used for genetic hypertension and hypertensive drug research (Okamoto, A.K. and Aoki K. Jpn. Circ. J. 1963. 27: 282-293). The effect of antisense inhibition of angiotensinogen with ISIS 552668 was studied in this model.

### Study 1

Groups of female SHR rats were injected with 50 mg/kg/week, 75 mg/kg/week, or 100 mg/kg/week of ISIS 552668 administered for 2 weeks. A control group of female SHR rats was injected with phosphate buffered saline (PBS) administered weekly for 2 weeks. Another control group constituted a group of wild-type rats injected with phosphate buffered saline (PBS) administered weekly for 2 weeks.

### Effect on blood pressure

Systolic blood pressure (SBP), mean arterial pressure (MAP), and diastolic blood pressure (DBP) in the rats was measured by the tail-cuff volume-pressure method (Kent Scientific, Torrington, CT). The results are presented in Table 4. The results indicate that antisense inhibition of angiotensinogen resulted in dose-dependent reductions in all three parameters in the rats compared to the control group.

**Table 4**

| Blood pressure parameters in 2 weeks (mm Hg) | | | | |
|---|---|---|---|---|
| Group | Dose of ASO (mg/kg/wk) | SBP | MAP | DBP |
| Wild-type control | - | 136 | 100 | 83 |
| SHR control | - | 179 | 146 | 131 |
| SHR treated with ISIS 552668 | 50 | 144 | 114 | 100 |
| | 75 | 140 | 111 | 98 |
| | 100 | 122 | 95 | 81 |

### Study 2

Groups of female SHR rats were injected with 50 mg/kg/week, 75 mg/kg/week, or 100 mg/kg/week of ISIS 552668 administered for 2 weeks. A control group of female SHR rats was injected with phosphate buffered saline (PBS) administered weekly for 2 weeks. Another control group constituted a group of wild-type rats injected with phosphate buffered saline (PBS) administered weekly for 2 weeks. After 2 weeks, the rats were treated with Captopril at 50 mg/kg/day and blood pressure was measured after 4 days of this treatment.

### Effect on blood pressure

Systolic blood pressure (SBP), mean arterial pressure (MAP), and diastolic blood pressure (DBP) in the rats was measured by the tail-cuff volume-pressure method (Kent Scientific, Torrington, CT). The results are presented in Table 5.

**Table 5**

| Blood pressure parameters (mm Hg) | | | | | |
|---|---|---|---|---|---|
| Group | Captopril | Dose of ASO (mg/kg/wk) | SBP | MAP | DBP |
| Wild-type control | No | - | 131 | 96 | 79 |
| SHR control | No | - | 179 | 146 | 131 |
| SHR rats | Yes | - | 145 | 113 | 97 |
| | Yes | 50 | 127 | 97 | 82 |
| | Yes | 75 | 113 | 87 | 74 |
| | Yes | 100 | 120 | 94 | 81 |

The results indicate that antisense inhibition of angiotensinogen generally resulted in dose-dependent reductions in all three parameters in the rats. The results also indicate that treatment of the rats with ISIS 552668 at doses of 50 mg/kg/week and 75 mg/kg/week before Captopril treatment further decreased blood pressure parameters compared to that with Captopril treatment alone. Hence, additive blood pressure lowering was achieved with the combination of angiotensinogen antisense oligonucleotide and Captopril treatment, suggesting that improvements in efficacy are possible when antisense oligonucleotides are added to existing RAS inhibitor therapeutics. Such improvement in treatments could be desirable in resistant hypertensive subjects or subjects not achieving their blood pressure goal with existing RAS inhibitors and/or patients that experience ACE escape and/or aldosterone breakthrough.

Additionally, Captopril treatment did not result in further blood pressure reductions in rats receiving 100 mg/kg/week of ISIS 552668, demonstrating that maximal inhibition of RAS signaling was already achieved by treatment with ISIS 552668 in this group. The data indicates that complete inhibition of RAS-dependent blood pressure control could be achieved within 2 weeks of ASO treatment.

### Effect on heart size

The size of the heart was measured immediately after harvest. The results from each group are presented in Table 6, expressed as a percent of body weight. The results indicate that treatment with ISIS 552668 after 3 weeks reversed the increase in heart size of the SHR rats to that observed in the wild-type controls.

**Table 6**

| Heart size (% body weight) | | | |
|---|---|---|---|
| Group | Captopril | Dose of ASO (mg/kg/wk) | Heart size (%) |
| Wild-type control | No | - | 44 |
| SHR rats | Yes | - | 47 |
| | Yes | 50 | 46 |
| | Yes | 75 | 45 |
| | Yes | 100 | 42 |

### Study 3

A group of SHR was injected with 100 mg/kg/week of ISIS 552668 administered for 3 weeks. A control group of SHR was injected with phosphate buffered saline (PBS) administered weekly for 3 weeks. Another control group of SHR was injected with 100 mg/kg/week of a control oligonucleotide (a nucleotide not targeted to angiotensinogen) administered for 3 weeks. Another control group of SHR was treated with Captopril at 150 mg/kg/day administered for 3 weeks.

### Effect on blood pressure

Mean arterial pressure (MAP) in the rats was measured by the tail-cuff volume-pressure method (Kent Scientific, Torrington, CT). The results are presented in Table 7 and indicate that treatment with ISIS 552668 and Captopril reduced MAP levels to comparable levels.

**Table 7**

| MAP (mm Hg) | | | |
|---|---|---|---|
| Group | Week 1 | Week 2 | Week 3 |
| Control oligonucleotide | 136 | 135 | 146 |
| PBS control | 150 | 136 | 150 |
| Captopril control | 107 | 109 | 112 |
| ISIS 552668 | 117 | 104 | 109 |

### RNA Analysis

RNA was extracted from liver tissue for real-time PCR analysis of angiotensinogen, using primer probe set RTS3550. The mRNA levels were normalized using the housekeeping gene, GAPDH. As shown in Table 8, ISIS 552668 achieved significant reduction of angiotensinogen over the PBS control. Results are presented as percent inhibition of angiotensinogen, relative to control.

**Table 8**

| Percent inhibition of angiotensinogen mRNA in SHR compared to the PBS control | | | |
|---|---|---|---|
| Group | Liver | Kidney | Heart |
| Control oligonucleotide | 5 | 0 | 0 |
| Captopril control | 0 | 41 | 0 |
| ISIS 552668 | 87 | 76 | 89 |

RNA was extracted from kidney tissue for real-time PCR analysis of renin, using primer probe set rRenin2 (forward sequence GCTTTGGACGAATCTTGCTCA, designated herein as SEQ ID NO: 21; reverse sequence TCCCCGCTCCTCCAGG, designated herein as SEQ ID NO: 22; probe sequence AAAATGCCCTCGGTCCGGGAAA, designated herein as SEQ ID NO: 23). The mRNA levels were normalized using the housekeeping gene, GAPDH. As shown in Table 9, Captopril treatment resulted in about three-fold induction of kidney renin expression compared to that with ISIS 552668. Increase in renin in the body is deleterious to the overall health of the animal (Nguyen and Muller, The Biology of the (Pro)Renin Receptor, J Am Soc Nephrol, 2010 Jan, 21(1):18-23). Hence, the relative lower increase in renin after treatment with ISIS 552668 indicates that it is a safer treatment for hypertensive subjects compared to treatment with Captopril. Results are presented as percent expression of renin, relative to control.

**Table 9**

| Percent increase of kidney renin mRNA in SHR | |
|---|---|
| Group | % |
| PBS | 100 |
| Control oligonucleotide | 162 |
| Captopril control | 1110 |
| ISIS 552668 | 379 |

### Protein Analysis

Angiotensinogen plasma levels were measured by ELISA (IBL International, Toronto, Canada). As shown in Table 10, antisense inhibition of angiotensinogen by ISIS 552668 resulted in a significant reduction of angiotensinogen protein. Results are presented as percent inhibition of angiotensinogen, relative to PBS control.

Immunohistochemical analysis of liver, cardiac and kidney tissues also demonstrated significant reduction of angiotensinogen from these tissues after treatment with ISIS 552668 compared to the PBS control.

**Table 10**

| Percent inhibition of angiotensinogen protein in the plasma of SHR compared to the PBS control | |
|---|---|
| Group | % |
| Control oligonucleotide | 15 |
| Captopril control | 24 |
| ISIS 552668 | 91 |

### Study 4

Groups of SHR were injected with 25 mg/kg/day, 50 mg/kg/day, 100 mg/kg/day, or 200 mg/kg/day of Captopril administered for 10 weeks. After 10 weeks, ISIS 552668 was administered as a single dose of 50 mg/kg to each of these groups. Two more groups of rats were administered ISIS 552668 at a single dose of 50 mg/kg and 100 mg/kg but were not given Captopril treatment. A control group of SHR was injected with PBS and was given no other treatment. Another control group of SHR was administered a control oligonucleotide (a nucleotide not targeted to angiotensinogen) as a single dose of 50 mg/kg and was given no other treatment.

### Effect on blood pressure

Systolic blood pressure (SBP) in the rats was measured before and after dosing with ISIS 552668 by the tail-cuff volume-pressure method (Kent Scientific, Torrington, CT). The results are presented in Table 11 and indicate that treatment with the ISIS oligonucleotide was comparable to that with Captopril. Both ISIS 552668 and Captopril reduced blood pressure (BP) levels compared to the PBS control.

**Table 11**

| Blood pressure parameters SHRs before and after the addition of 50 mg/kg ISIS 552668 treatment to SHRs given Captopril for 10 weeks | | | |
|---|---|---|---|
| Group | Dose of agent | Before ISIS 552668 dosing | After ISIS 552668 dosing |
| PBS | - | 174 | - |
| Control ASO | 50 mg/kg/week | 166 | - |
| Captopril | 25 mg/kg/day | 159 | 153 |
| Captopril | 50 mg/kg/day | 147 | 133 |
| Captopril | 100 mg/kg/day | 137 | 127 |
| Captopril | 200 mg/kg/day | 135 | - |
| ISIS 552668 | 50 mg/kg/week | 146 | - |
| ISIS 552668 | 100 mg/kg/week | 131 | - |

The results indicate that treatment of the rats with ISIS 552668 at a dose of 50 mg/kg/week with Captopril treatment further decreased blood pressure parameters compared to that with Captopril treatment alone. Hence, additive blood pressure lowering was achieved with the combination of angiotensinogen antisense oligonucleotide in animals chronically treated with Captopril, suggesting that improvements in efficacy are possible when antisense oligonucleotides are added to existing RAS inhibitor therapeutics. Such improvement in treatments could be desirable in resistant hypertensive subjects or subjects not achieving their blood pressure goal with existing RAS inhibitors and/or patients that experience ACE escape and/or aldosterone breakthrough.

### Example 3: In vivo antisense inhibition of angiotensinogen in Dahl/SS rats

Dahl/Salt Sensititive (Dahl/SS) rats are a model used for diseases associated with high blood pressure (Cowley, A.W. Jr. et al., Physiol. Genomics. 2000. 2: 107-115). The effect of antisense inhibition of angiotensinogen with ISIS 552668 was studied in this model.

### Treatment

The rats were fed a diet with 8% NaCl. A group of female Dahl/SS rats was injected with 40 mg/kg/week of ISIS 552668 administered for 3 weeks. A group of female Dahl/SS rats was injected with 40 mg/kg/week of control oligonucleotides ISIS 141923 administered for 3 weeks. A group of female Dahl/SS rats was injected 150 mg/kg of Captopril administered daily for 3 weeks. A control group of female Dahl/SS rats was injected with phosphate buffered saline (PBS) administered weekly for 3 weeks. Another control group constituted a group of Dahl/Salt Resistant (Dahl/SR) rats injected with phosphate buffered saline (PBS) administered weekly for 3 weeks.

### Effect on blood pressure

Systolic blood pressure (SBP), mean arterial pressure (MAP), and diastolic blood pressure (DBP) in the rats was measured by the tail-cuff volume-pressure method (Kent Scientific). The results are presented in Table 12, expressed as the percent decrease in blood pressure compared to the control SHR group. The results indicate that antisense inhibition of angiotensinogen resulted in dose-dependent reductions in all three parameters in the rats. The results also indicate that treatment of the rats with ISIS 552668 decreased blood pressure parameters more than that with Captopril treatment.

**Table 12**

| Blood pressure parameters in 3 weeks (mm Hg) | | | |
|---|---|---|---|
| Group | SBP | MAP | DBP |
| Dahl/SR control | 157 | 126 | 111 |
| Dahl/SS control | 188 | 147 | 127 |
| Dahl/SS+ISIS 141923 | 188 | 147 | 127 |
| Dahl/SS+ISIS 552668 | 146 | 111 | 94 |
| Dahl/SS+Captopril | 162 | 128 | 111 |

## Claims

1. A compound comprising a modified antisense oligonucleotide consisting of 12 to 30 linked nucleosides, wherein the modified antisense oligonucleotide is targeted to an angiotensinogen (AGT) nucleic acid, for use in treating an animal having a RAS pathway related disease, disorder and/or condition, wherein the RAS pathway related disease, disorder or condition is resistant hypertension.

2. The compound for use of claim 1, wherein the compound decreases blood pressure.

3. The compound for use of claim 1 or claim 2, wherein the compound treats and/or reverses organ damage.

4. The compound for use of claim 3, wherein the organ damage is heart muscle hypertophy or fibrosis in an organ, and wherein optionally the organ is heart, liver or kidney.

5. The compound for use of any preceding claim, wherein the animal experiences ACE escape and/or aldosterone escape.

6. The compound for use of any preceding claim, wherein the modified antisense oligonucleotide consists of a single-stranded modified oligonucleotide.

7. The compound for use of any preceding claim, wherein the modified antisense oligonucleotide consists of 20 linked nucleosides.

8. The compound for use of any preceding claim, wherein the modified antisense oligonucleotide comprises:
(i) at least one modified internucleoside linkage, wherein optionally the modified internucleoside linkage is a phosphorothioate internucleoside linkage;
(ii) at least one modified sugar, wherein optionally the modified sugar is a bicyclic sugar, a 2'-F, a 2'-O-methyl or a 2'-O-methoxyethyl, and/or
(iii) at least one modified nucleobase, wherein optionally the modified nucleobase is a 5-methylcytosine.

9. The compound for use of claim 8, wherein the modified antisense oligonucleotide comprises:
(a) a gap segment consisting of linked deoxynucleosides;
(b) a 5' wing segment consisting of linked nucleosides; and
(c) a 3' wing segment consisting of linked nucleosides;
wherein the gap segment is positioned immediately adjacent to and between the 5' wing segment and the 3' wing segment and wherein each nucleoside of each wing segment comprises a modified sugar,
and wherein optionally the modified antisense oligonucleotide comprises:
(a) a gap segment consisting of eight to sixteen linked deoxynucleosides;
(b) a 5' wing segment consisting of two to six linked nucleosides; and
(c) a 3' wing segment consisting of two to six linked nucleosides;
wherein the gap segment is positioned immediately adjacent to and between the 5' wing segment and the 3' wing segment, wherein each nucleoside of each wing segment comprises a 2'-O-methoxyethyl sugar, and wherein each internucleoside linkage is a phosphorothioate linkage, or
( a) a gap segment consisting of ten linked deoxynucleosides;
( b) a 5' wing segment consisting of five linked nucleosides; and
( c) a 3' wing segment consisting of five linked nucleosides;
wherein the gap segment is positioned immediately adjacent to and between the 5' wing segment and the 3' wing segment, wherein each nucleoside of each wing segment comprises a 2'-O-methoxyethyl sugar, and wherein each internucleoside linkage is a phosphorothioate linkage.

10. The compound for use of any preceding claim, wherein the animal is a human.

11. The compound for use of any preceding claim, wherein the modified antisense oligonucleotide is complementary to an AGT nucleic acid having a sequence as shown in any of SEQ ID NOs: 1-16.

12. The compound for use of claim 11, wherein the modified antisense oligonucleotide comprises a nucleobase sequence at least 80%, at least 85%, at least 90%, at least 95% or 100% complementary to any of the nucleobase sequences recited in SEQ ID NOs: 1-16.

13. The compound for use of any preceding claim, wherein the compound comprises a conjugate group.

14. The compound for use of any preceding claim, wherein the compound is a salt form.

15. The compound for use of any preceding claim, wherein the compound is formulated with a pharmaceutically acceptable carrier or diluent.

## Patentansprüche

1. Verbindung, umfassend ein modifiziertes Antisense-Oligonukleotid, das aus 12 bis 30 verbundenen Nukleosiden besteht, wobei das modifizierte Antisense-Oligonukleotid auf eine Angiotensinogen-(AGT)-Nukleinsäure gerichtet ist, zur Verwendung bei der Behandlung eines Tieres, das an einer mit dem RAS-Weg in Zusammenhang stehenden Erkrankung, Störung und/oder Bedingung leidet, wobei die mit dem RAS-Weg in Zusammenhang stehende Erkrankung, Störung oder Bedingung resistente Hypertonie ist.

2. Verbindung zur Verwendung nach Anspruch 1, wobei die Verbindung den Blutdruck senkt.

3. Verbindung zur Verwendung nach Anspruch 1 oder Anspruch 2, wobei die Verbindung Organschädigung behandelt und/oder umkehrt.

4. Verbindung zur Verwendung nach Anspruch 3, wobei die Organschädigung Herzmuskelhypertophie oder Fibrose in einem Organ ist und wobei das Organ gegebenenfalls Herz, Leber oder Niere ist.

5. Verbindung zur Verwendung nach einem vorhergehenden Anspruch, wobei das Tier ACE-Escape und/oder Aldosteron-Escape erfährt.

6. Verbindung zur Verwendung nach einem vorhergehenden Anspruch, wobei das modifizierte Antisense-Oligonukleotid aus einem einzelsträngigen modifizierten Oligonukleotid besteht.

7. Verbindung zur Verwendung nach einem vorhergehenden Anspruch, wobei das modifizierte Antisense-Oligonukleotid aus 20 verbundenen Nukleosiden besteht.

8. Verbindung zur Verwendung nach einem vorhergehenden Anspruch, wobei das modifizierte Antisense-Oligonukleotid umfasst:
(i) mindestens eine modifizierte Internukleosidbindung, wobei die modifizierte Internukleosidbindung gegebenenfalls eine Phosphorthioatinternukleosidbindung ist;
(ii) mindestens einen modifizierten Zucker, wobei der modifizierte Zucker gegebenenfalls ein bicyclischer Zucker, ein 2'-F, ein 2'-O-Methyl oder ein 2'-O-Methoxyethyl ist, und/oder
(iii) mindestens eine modifizierte Nukleobase, wobei die modifizierte Nukleobase gegebenenfalls ein 5-Methylcytosin ist.

9. Verbindung zur Verwendung nach Anspruch 8, wobei das modifizierte Antisense-Oligonukleotid umfasst:
(a) ein Lückensegment, das aus verbundenen Desoxynukleosiden besteht;
(b) ein 5'-Flügelsegment, das aus verbundenen Nukleosiden besteht; und
(c) ein 3'-Flügelsegment, das aus verbundenen Nukleosiden besteht;
wobei das Lückensegment unmittelbar neben und zwischen dem 5'-Flügelsegment und dem 3'-Flügelsegment positioniert ist und wobei jedes Nukleosid jedes Flügelsegments einen modifizierten Zucker umfasst,
und wobei das modifizierte Antisense-Oligonukleotid gegebenenfalls umfasst:
(a) ein Lückensegment, das aus 8 bis 16 verbundenen Desoxynukleosiden besteht;
(b) ein 5'-Flügelsegment, das aus 2 bis 6 verbundenen Nukleosiden besteht; und
(c) ein 3'-Flügelsegment, das aus 2 bis 6 verbundenen Nukleosiden besteht;
wobei das Lückensegment unmittelbar neben und zwischen dem 5'-Flügelsegment und dem 3'-Flügelsegment positioniert ist, wobei jedes Nukleosid jedes Flügelsegments einen 2'-O-Methoxyethyl-Zucker umfasst und wobei jede Internukleosidbindung eine Phosphorthioatbindung ist, oder
(a) ein Lückensegment, das aus 10 verbundenen Desoxynukleosiden besteht;
(b) ein 5'-Flügelsegment, das aus 5 verbundenen Nukleosiden besteht; und
(c) ein 3'-Flügelsegment, das aus 5 verbundenen Nukleosiden besteht;
wobei das Lückensegment unmittelbar neben und zwischen dem 5'-Flügelsegment und dem 3'-Flügelsegment positioniert ist, wobei jedes Nukleosid jedes Flügelsegments einen 2'-O-Methoxyethyl-Zucker umfasst und wobei jede Internukleosidbindung eine Phosphorthioatbindung ist.

10. Verbindung zur Verwendung nach einem vorhergehenden Anspruch, wobei das Tier ein Mensch ist.

11. Verbindung zur Verwendung nach einem vorhergehenden Anspruch, wobei das modifizierte Antisense-Oligonukleotid zu einer AGT-Nukleinsäure mit einer in einer der SEQ ID NO: 1-16 gezeigten Sequenz komplementär ist.

12. Verbindung zur Verwendung nach Anspruch 11, wobei das modifizierte Antisense-Oligonukleotid eine Nukleobasensequenz umfasst, die zu mindestens 80%, mindestens 85%, mindestens 90%, mindestens 95% oder 100% zu einer der in SEQ ID NO: 1-16 aufgeführten Nukleobasensequenzen komplementär ist.

13. Verbindung zur Verwendung nach einem vorhergehenden Anspruch, wobei die Verbindung eine konjugierte Gruppe umfasst.

14. Verbindung zur Verwendung nach einem vorhergehenden Anspruch, wobei die Verbindung eine Salzform ist.

15. Verbindung zur Verwendung nach einem vorhergehenden Anspruch, wobei die Verbindung mit einem pharmazeutisch akzeptablen Träger oder Verdünnungsmittel formuliert ist.

## Revendications

1. Composé comprenant un oligonucléotide antisens modifié constitué par 12 à 30 nucléosides liés, dans lequel l'oligonucléotide antisens modifié est ciblé sur un acide nucléique d'angiotensinogène (AGT), destiné à être utilisé dans le traitement d'un animal ayant une maladie, un trouble et/ou une condition associés à la voie de RAS, dans lequel la maladie, le trouble ou la condition associé (e) à la voie de RAS est une hypertension résistante.

2. Composé destiné à l'utilisation de la revendication 1, le composé diminuant la pression sanguine.

3. Composé destiné à l'utilisation de la revendication 1 ou la revendication 2, le composé traitant et/ou inversant une lésion d'organe.

4. Composé destiné à l'utilisation de la revendication 3, dans lequel la lésion d'organe est une hypertrophie du muscle cardiaque ou une fibrose dans un organe, et dans lequel, en option, l'organe est le cœur, le foie ou un rein.

5. Composé destiné à l'utilisation de l'une quelconque des revendications précédentes, dans lequel l'animal expérimente une fuite d'ACE et/ou une fuite d'aldostérone.

6. Composé destiné à l'utilisation de l'une quelconque des revendications précédentes, dans lequel l'oligonucléotide antisens modifié est constitué par un oligonucléotide modifié en simple brin.

7. Composé destiné à l'utilisation de l'une quelconque des revendications précédentes, dans lequel l'oligonucléotide antisens modifié est constitué par 20 nucléosides liés.

8. Composé destiné à l'utilisation de l'une quelconque des revendications précédentes, dans lequel l'oligonucléotide antisens modifié comprend :
(i) au moins une liaison inter-nucléosidique modifiée, en option dans lequel la liaison inter-nucléosidique modifiée est une liaison inter-nucléosidique de phosphorothioate ;
(ii) au moins un sucre modifié, en option le sucre modifié étant un sucre bicyclique, un 2'-F, un 2'-O-méthyle ou un 2'-O-méthoxyéthyle et/ou
(iii) au moins une nucléobase modifiée, en option la nucléobase modifiée étant une 5-méthylcytosine.

9. Composé destiné à l'utilisation de la revendication 8, dans lequel l'oligonucléotide antisens modifié comprend :
(a) un segment de brèche constitué par des désoxynucléosides liés ;
(b) un segment d'aile en 5' constitué par des nucléosides liés ; et
(c) un segment d'aile en 3' constitué par des nucléosides liés ;
dans lequel le segment de brèche est positionné de manière immédiatement adjacente au segment d'aile en 5' et au segment d'aile 3' et entre ceux-ci, et dans lequel chaque nucléoside de chaque segment d'aile comprend un sucre modifié,
et, en option, dans lequel l'oligonucléotide antisens modifié comprend :
(a) un segment de brèche constitué par huit à seize désoxynucléosides liés ;
(b) un segment d'aile en 5' constitué par deux à six nucléosides liés ; et
(c) un segment d'aile en 3' constitué par deux à six nucléosides liés ;
dans lequel le segment de brèche est positionné de manière immédiatement adjacente au segment d'aile en 5' et au segment d'aile 3' et entre ceux-ci, dans lequel chaque nucléoside de chaque segment d'aile comprend un sucre à 2'-O-méthoxyéthyle, et dans lequel chaque liaison inter-nucléosidique est une liaison de phosphorothioate, ou
(a) un segment de brèche constitué par dix désoxynucléosides liés ;
(b) un segment d'aile en 5' constitué par cinq nucléosides liés ; et
(c) un segment d'aile en 3' constitué par cinq nucléosides liés ;
dans lequel le segment de brèche est positionné de manière immédiatement adjacente au segment d'aile en 5' et au segment d'aile 3' et entre ceux-ci, dans lequel chaque nucléoside de chaque segment d'aile comprend un sucre à 2'-O-méthoxyéthyle, et dans lequel chaque liaison inter-nucléosidique est une liaison de phosphorothioate.

10. Composé destiné à l'utilisation de l'une quelconque des revendications précédentes, dans lequel l'animal est un être humain.

11. Composé destiné à l'utilisation de l'une quelconque des revendications précédentes, dans lequel l'oligonucléotide antisens modifié est complémentaire à un acide nucléique d'AGT ayant une séquence telle que présentée dans l'une quelconque des SEQ ID n° : 1 à 16.

12. Composé destiné à l'utilisation de la revendication 11, dans lequel l'oligonucléotide antisens modifié comprend une séquence de nucléobases étant complémentaire au moins à 80%, au moins à 85%, au moins à 90%, au moins à 95% ou à 100% à l'une quelconque des séquences de nucléobases mentionnées dans les SEQ ID n° : 1 à 16.

13. Composé destiné à l'utilisation de l'une quelconque des revendications précédentes, le composé comprenant un groupe conjugué.

14. Composé destiné à l'utilisation de l'une quelconque des revendications précédentes, le composé étant une forme de sel.

15. Composé destiné à l'utilisation de l'une quelconque des revendications précédentes, le composé étant formulé avec un véhicule ou diluant acceptable d'un point de vue pharmaceutique.
